# EUROPEAN PATENT SPECIFICATION

(11) **EP 3 958 980 B1**
(45) Date of publication and mention of the grant of the patent: **15.11.2023**
(21) Application number: 21806122.4
(22) Date of filing: 22.01.2021
(51) Int. Cl.: A61P 35/00, C07D 513/04, A61K 31/5377, A61K 31/54

(54) **TARGETED NOVEL TRIAZOLOTHIADIAZINE DERIVATIVES FOR THE TREATMENT OF LUNG CANCER**
ZIELGERICHTETE NEUE TRIAZOLOTHIADIAZIN-DERIVATE ZUR BEHANDLUNG VON LUNGENKREBS
NOUVEAUX DÉRIVÉS DE TRIAZOLOTHIADIAZINE CIBLÉS POUR LE TRAITEMENT DU CANCER DU POUMON

(30) Priority: 17.06.2020 TR 202009388
(43) Date of publication of application: 02.03.2022
(73) Proprietor: Anadolu Universitesi, Tepebasi/Eskisehir (TR)
(72) Inventor: SEVER, Belgin, Tepebasi/Eskisehir (TR); ÖZDEMIR, Ahmet, Tepebasi/Eskisehir (TR); ALTINTOP, Mehlika Dilek, Tepebasi/Eskisehir (TR); ÇIFTÇI, Gülsen Akalin, Tepebasi/Eskisehir (TR)
(74) Representative: Sevinç, Cenk
(86) International application number: PCT/TR2021/050053
(87) International publication number: WO 2021/257020

(56) References cited:
- WO-A2-2007/064797
- CN-B- 106 243 130
- Özdemir Ahmet, Sever Belgin, Alt ntop Mehlika, Temel Halide, Atl Özlem, Baysal Merve, Demirci Fatih: "Synthesis and Evaluation of New Oxadiazole, Thiadiazole, and Triazole Derivatives as Potential Anticancer Agents Targeting MMP-9", Molecules, SPRINGER VERLAG, BERLIN, DE, vol. 22, no. 7, 4 July 2017 (2017-07-04), page 1109, XP055888088, DE ISSN: 1433-1373, DOI: 10.3390/molecules22071109
- SEVER, B. et al.: "Indomethacin based new triazolothiadiazine derivatives: Synthesis, evaluation of their anticancer effects on T98 human glioma cell line related to COX-2 inhibition and docking studies", European journal of medicinal chemistry, vol. 113, 2016, pages 179-186, XP029475875, DOI: 10.1016/j.ejmech.2016.02.036
- Lien Hsiu-Man, Kuo Po-Tsun, Huang Chao-Lu, Kao Jung-Yie, Lin Ho, Yang Ding-Yah, Lai Ya-Yun: "Study of the Anti-Proliferative Activity of 5-Substituted 4,7-Dimethoxy-1,3-Benzodioxole Derivatives of SY-1 from Antrodia camphorata on Human COLO 205 Colon Cancer Cells", Evidence-Based Complementary and Alternative Medicine, Oxford University Press, US, vol. 2011, no. 8, 1 January 2011 (2011-01-01), pages 1-8, XP055888092, US ISSN: 1741-427X, DOI: 10.1093/ecam/nep230

## Description

### Technical Field of the Invention

The invention is related to novel 1,2,4-triazolo[3,4-*b*]-1,3,4-thiadiazine derivatives exhibiting selective cytotoxic and apoptotic effects through the inhibition of cyclooxygenase-2 (COX-2), Akt and/or Rho-protein kinase (ROCK) enzymes, which are known as important target enzymes in cancer pathogenesis, for the treatment of lung cancer and the synthesis, *in vitro* anticancer activity studies, molecular docking and *in silico* Absorption, Distribution, Metabolism and Excretion (ADME) studies of these compounds. The compounds of the invention are used to prepare a medicine for the treatment of lung cancer.

### Known State of the Art (Prior Art)

Lung cancer is a very invasive cancer type causing rapid metastasis with a high prevalence. Lung cancer is the most important cause of cancer-related deaths worldwide. Despite the developments in combination treatment including chemotherapy and radiotherapy, the recovery rate of patients has not reached the desired level.

It has been shown that COX-2 enzyme, which is an important target in cancer treatment, is overexpressed in non-small cell lung cancers. Akt enzyme is also an essential regulator in cellular metabolism and an increased level of Akt enzyme has been observed in lung cancers. ROCK is involved in a wide range of cellular functions including cell motility, adhesion, migration, proliferation and apoptosis and for this reason, it is considered as an important target for cancer.

In the recent years, 1,3,4-thiadiazines condensed with 1,2,4-triazole have gained importance in studies related to the design and development of anticancer drugs. However, the limited number of targeted anticancer studies related to this ring system encourages medicinal chemists to design and synthesize novel triazolothiadiazine derivatives with high potential to be anticancer drugs acting through different target enzymes.

Many triazolothiadiazine derivatives known in the art which are effective against various cancer types and the synthesis and anticancer activity studies of these compounds are listed below:
(Shivarama Holla et al., 2001, Il Farmaco, 56/8, 565-570; Shivarama Holla et al., 2006, European Journal of Medicinal Chemistry, 41/5, 657-663) synthesized 7-arylidene-6-(2,4-dichloro-5-fluorophenyl)-3-substituted-1,2,4-triazolo[3,4-*b*]-1,3,4-thiadiazine derivatives and evaluated the anticancer potential of these compounds. They found out that some of these derivatives reduced the growth of lung cancer (NCI-H 460), breast cancer (MCF-7) and central nervous system cancer (SF268) cells at a rate of 32% or less. It was observed that methyl, phenyl and 2-chlorophenoxymethyl groups located at the 3rd position of the triazolothiadiazine ring system and 3,4-methylenedioxy, 3,4-dimethoxy and 4-chloro groups at the 7th position contribute positively to anticancer activity.

(Bhat et al., 2009, European Journal of Medicinal Chemistry, 44/12, 5066-5070) synthesized 3-(2,4-dichloro-5-fluorophenyl)-6-(substituted phenyl)-1,2,4-triazolo[3,4-*b*]-1,3,4-thiadiazine derivatives and evaluated the *in vitro* antitumor effects of these derivatives on 60 cell lines formed of leukemia, NSCLC, melanoma, prostate, ovarian and breast cancers. 3-(2,4-Dichloro-5-fluorophenyl)-6-(4-chlorophenyl)-1,2,4-triazolo[3,4-*b*]-1,3,4-thiadiazine exhibited significant cytotoxic activity against all cancer cells (GI₅₀= 1.06-25.4 µM). The high activity of this compound compared to other derivatives is attributed to the presence of the chlorine substituent located at the 4th position of the phenyl ring.

(Sumangala et al., 2012, European Journal of Medicinal Chemistry, 54, 59-64) synthesized 6-substituted-3-[4-(methylsulfonyl)benzyl]-*7H*-[1,2,4]triazolo[3,4-*b*][1,3,4]thiadiazine derivatives and examined the cytotoxicities of these derivatives against human colon epithelial (HT-29) cells in comparison to doxorubicin, which is a standard drug. In these studies, it was determined that 6-(biphenyl-4-yl)-3-(4-(methylsulfonyl)benzyl)-*7H-*[1,2,4]triazolo[3,4-*b*][1,3,4]thiadiazine (IC₅₀= 12.5 µM) and 3-(4-(methylsulfonyl)benzyl)-6-phenyl-*7H*-[1,2,4]triazolo[3,4-*b*][1,3,4]thiadiazine (IC₅₀= 25 µM) were more cytotoxic than other derivatives.

(Khan et al., 2014, Bioorganic & Medicinal Chemistry, 22/21, 6163-6173) synthesized some triazolothiadiazine derivatives and evaluated the cytotoxicities of these compounds against lung cancer (H157) cells. 6-(3,4-Dichlorophenyl)-3-(pyridine-3-yl)-*7H*-[1,2,4]triazolo[3,4-*b*][1,3,4]thiadiazine (IC₅₀= 0.96±0.43 µM) exhibited the highest antiproliferative activity as compared to the standard drug vincristine (IC₅₀= 1.03±0.04 µM). In another study (Khan et al., 2014, European Journal of Medicinal Chemistry, 78, 167-177), they synthesized 3,6-disubstituted-1,2,4-triazolo-[3,4-*b*]-1,3,4-thiadiazine derivatives and evaluated the cytotoxic effects of these derivatives on kidney fibroblast (BHK-21) cells. 6-(3-Methoxyphenyl)-3-(pyridine-4-yl)-*7H*-[1,2,4]triazolo[3,4-*b*][1,3,4]thiadiazine (73.3% inhibition) and 3-(pyridine-4-yl)-6-(*p*-tolyl)-*7H*-[1,2,4]triazolo[3,4-*b*][1,3,4]thiadiazine (72.6% inhibition) showed notable activity in comparison to the standard drug vincristine (74.5% inhibition) against BHK-21 cells.

(Ayta 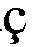 et al., 2016, Bioorganic & Medicinal Chemistry, 24/4, 858-872) synthesized 3-[1-[4-(2-methylpropyl)phenyl]ethyl]-6-(4-substituted phenyl)-*7H*-1,2,4-triazolo[3,4-*b*]-1,3,4-thiadiazine derivatives and investigated the anticancer effects of these compounds on epithelial cancer cell lines. It was shown that compounds having the IC₅₀ value less than ≈5 µM induced apoptotic cell death and SubG1 cell cycle arrest. 3-[1-[4-(2-Methylpropyl)phenyl]ethyl]-6-(4-methylphenyl)-*7H*-1,2,4-triazolo[3,4-*b*]-1,3,4-thiadiazine and 3-[1-[4-(2-methylpropyl)phenyl]ethyl]-6-(4-methoxyphenyl)-*7H*-1,2,4-triazolo[3,4-*b*]-1,3,4-thiadiazine induced apoptosis through ASK-1 protein activation and Akt inhibition. In this study, the effects of compounds carrying the triazolothiadiazine ring on apoptotic pathways were examined. However, Annexin V FITC, caspase-3, mitochondrial membrane depolarization and cell cycle tests, which are important for apoptotic pathway, were not carried out. Moreover, the inhibitory effects of the synthesized compounds on COX and ROCK enzymes were not determined.

(Ibrar et al., 2016, Archiv der Pharmazie (Weinheim), 349/7, 553-565) synthesized novel coumarin-triazolothiadiazine derivatives and evaluated the anticancer effects of these compounds on kidney fibroblast (BHK-21) and lung carcinoma (H-157) cell lines. 3-(6-(Biphenyl-4-yl)-*7H*-[1,2,4]triazolo[3,4-*b*][1,3,4]-thiadiazine-3-yl)-2*H*-chromene-2-one showed the highest cytotoxic activity with the IC₅₀ value of 1.01±0.12 µM.

(Sever et al., 2016, European Journal of Medicinal Chemistry, 113, 179-186) synthesized eight 3-[5-methoxy-2-methyl-1-(4-chlorobenzoyl)-1*H*-indole-3-yl)methyl]-6-(4-substituted phenyl)-7*H*-1,2,4-triazolo[3,4-*b*]-1,3,4-thiadiazine derivatives and examined the antiproliferative effects of these compounds on T98 human glioma cell line. The apoptotic effects of 3-[5-methoxy-2-methyl-1-(4-chlorobenzoyl)-1*H*-indole-3-yl)methyl]-6-(4-methylphenyl)-*7H*-1,2,4-triazolo[3,4-*b*]-1,3,4-thiadiazine, which was determined to be anticancerous with the MTT assay, were measured with flow cytometry; and the COX-2, caspase 3, 8 and 9, cytochrome c mRNA relative amount measurements were carried out with Real Time Polymerase Chain Reaction (RT-PCR). It was determined that this compound significantly reduced the COX-2 mRNA amount as compared to the control group and it did not lead to any significant change in other parameters (caspase-3, 8, 9, cytochrome c). Therefore, a molecular docking study was carried out in the active site of the COX-2 enzyme (PDB: 4COX) for this compound. It was determined that the effective COX-2 inhibitor exhibited favorable interaction in the active site of the enzyme. However, the apoptotic effects of the most effective compound were examined, but no effect was observed. Moreover, the inhibitory effects of the compounds on Akt and ROCK kinase enzymes were not investigated. As the number of compounds is low, this makes it difficult to establish the structure-activity relationship.

(Ahmad et al., 2017, Arabian Journal of Chemistry, 10/2, 3347-3357) synthesized 1,2,4-triazolo[3,4-*b*]-1,3,4-thiadiazine derivatives carrying the long alkenyl/hydroxyalkenyl chain at the 3rd position. They examined the *in vitro* cytotoxic effects of these compounds on hepatocellular carcinoma (Hep3 B), human breast adenocarcinoma (MCF-7), human cervical carcinoma (HeLa) and peripheral blood mononuclear cells (PMBCs), which are normal human cells. The synthesized compounds did not exhibit cytotoxicity against healthy human cells. Among these compounds, 3-[(8*Z*,11*R*)-11-hydroxyheptadec-8-enyl]-6-phenyl-7*H*-1,2,4-triazolo[3,4-*b*]-1,3,4-thiadiazine and 3-[(8*R*,11*Z*)-8-hydroxyheptadec-11-enyl]-6-phenyl-7*H-*1,2,4-triazolo[3,4-*b*]-1,3,4-thiadiazine showed cytotoxic effects with the IC₅₀ values lower than 8.83±1.4 µM, against all tested cancer cells and they were evaluated as the most promising anticancer compounds.

The patent document numbered EP 1 963 329 B1 is related to several compounds having triazolothiadiazole and triazolothiadiazine scaffolds which can inhibit the c-MET receptor tyrosine kinase enzyme found to be effective on several cancer types. Said compounds of the invention were mentioned only to have inhibitory effects on the c-MET enzyme. The apoptotic effects and the inhibitory effects of the compounds on different kinases except c-MET were not examined.

The study carried out by (Altmtop et al., 2011, European Journal of Medicinal Chemistry, 46/11, 5562-5666) is related to triazolothiadizine derivatives which were obtained *via* the ring closure reaction of 4-amino-5-(2-cyclohexyl/(4-hydroxyphenyl)ethyl)-2,4-dihydro-3*H*-1,2,4-triazol-3-thione with phenacyl bromides. In this study, the anticandidal effects of the triazolothiadiazine derivatives on *Candida* species were examined in terms of the treatment of fungal infections.

(Arandkar and Vedula, 2019, Phosphorus, Sulfur, and Silicon and the Related Element, 194/4-6, 533-539) synthesized novel triazolothiadiazine derivatives *via* the reaction of 4-amino-4*H*-1,2,4-triazolo-3,5-dithiol with various phenacyl bromides . The *in vitro* anticancer effects of the obtained triazolothiadiazine derivatives on 60 cell lines were examined. Among the synthesized compounds, 1-(4-fluorophenyl)-2-((6-(4-fluorophenyl)-7*H-*[1,2,4]triazolo[3,4-*b*][1,3,4]thiadiazine-3-yl)thio)ethanone (47.42% growth, 52.58% inhibition) and 1-(4-bromophenyl)-2-((6-(4-bromophenyl)-7*H*-[1,2,4]triazolo[3,4-*b*][1,3,4]thiadiazine-3-yl)thio)ethanone (46.76% growth, 53.24% inhibition) showed significant activity in terms of growth percentages against kidney cancer (OU-31) cell line as 47.42% and 46.76%, respectively. 2-((6-(4-Chlorophenyl)-*7H*-[1,2,4]triazolo[3,4-*b*][1,3,4]thiadiazine-3-yl)thio-1-(4-fluorophenyl)ethanone exhibited significant activity in terms of growth percentages against OU-31 cell line as 48.14% and in terms of leukemia (MOLT-4) cell line as 49.82%. Among the tested compounds, only 1-(4-bromophenyl)-2-((6-(4-bromophenyl)-7*H*-[1,2,4]triazolo[3,4-*b*][1,3,4]thiadiazine-3-yl)thio)ethanone exhibited moderate activity (58.58% growth, 41.42% inhibition) against A549 cells. In this work, any kind of study was not carried out to explain the mechanisms of anticancer action of active compounds.

Triazolothiadiazine derivatives showing selective cytotoxic and apoptotic effects through the inhibition of COX-2, Akt and/or ROCK enzymes, which are three important target enzymes in cancer pathogenesis are not known in the art for the treatment of lung cancer.

### Brief Description of the Invention and its Aims

The invention is related to novel triazolothiadiazine derivatives exhibiting selective cytotoxic and apoptotic effects through the inhibition of COX-2, Akt and/or ROCK enzymes, which are important enzymes in cancer pathogenesis, for the treatment of lung cancer.

With the invention, fifteen 3-[2-(1,3-benzodioxole-5-yl)ethyl)]-6-aryl-*7H*-1,2,4-triazolo[3,4-*b*]-1,3,4-thiadiazine derivatives were synthesized.

Within the scope of the invention, experimental studies related to apoptosis, caspase-3, mitochondrial membrane depolarization, cell cycle arrest were carried out. Moreover, the mechanism of action of compounds having anticancer activity were examined through three target enzymes (COX, Akt and ROCK).

By means of the invention, active, highly orally bioavailable new drug target triazolothiadiazine derivatives, which are predicted to be effective on COX-2, Akt and ROCK enzymes as a result of *in silico* molecular docking and ADME studies were synthesized.

With the invention, novel 3-[2-(1,3-benzodioxole-5-yl)ethyl)]-6-aryl-*7H*-1,2,4-triazolo[3,4-*b*]-1,3,4-thiadiazine derivatives (**1-15**) were synthesized *via* the ring closure reaction of 2-bromo-1-arylethanone derivatives with 4-amino-5-[2-(1,3-benzodioxole-5-yl)ethyl]-2,4-dihydro-3*H*-1,2,4-triazole-3-thione, which was synthesized by the solvent-free reaction of 3-(1,3-benzodioxole-5-yl)propanoic acid with thiocarbohydrazide.

The cytotoxicities of the synthesized compounds against A549 human lung adenocarcinoma cells and CCD-19Lu normal lung fibroblast cells were examined with the MTT assay. Compounds **9, 11, 12** and **14** carrying 4-methylsulfonylphenyl, 4-trifluoromethylphenyl, 3-fluorophenyl and biphenyl groups at the sixth position of the triazolothiadiazine ring, respectively exhibited higher anticancer activity than cisplatin (IC₅₀= 23.00±1.41 µg/mL) against A549 cell line with the IC₅₀ values of 5.75±2.47 µg/mL, 0.73±0.16 µg/mL, 0.80±0.05 µg/mL, 2.63±0.72 µg/mL, respectively. These compounds did not exhibit cytotoxicity against CCDLu19 cells. The apoptotic effects of these compounds on A549 cells were examined with Annexin V FITC, caspase-3, JC-1 and cell cycle assays. According to these findings, compounds **12** (25.90%) and **14** (34.50%) caused the highest early and late apoptotic effects on A549 cell line as compared to cisplatin (44.80%). These compounds did not cause significant caspase-3 activation on A549 cells. As a result of the JC-1 assays, compound **11** (58.80%) caused higher mitochondrial membrane depolarization than cisplatin (48.00%). Compounds **9, 11, 12** and **14** caused an increase (53.29%, 57.29%, 54.20%, 54.92%, respectively) higher than cisplatin (45.92%) at the G1 phase. The *in vitro* inhibitory effects of these compounds on COX, Akt and ROCK enzymes were determined. Both the COX-1 (54.18% and 65.33%, respectively) and COX-2 (38.58% and 53.52%, respectively) inhibitory effects of compounds **11** and **12** were found to be higher than those of cisplatin (24.65% and 43.46%, respectively). Compounds **9, 12** and **14** caused moderate Akt inhibition in comparison to GSK690693. Compound **14** (IC₅₀= 2.55±0.21 µg/mL) exhibited potent ROCK inhibition in comparison to cisplatin (29.84%). These compounds are apoptosis inducing and are targeted potential anticancer agents.

With the invention, novel orally bioavailable anticancer compounds acting on different targets, which can be obtained easily and cheaply, were identified.

The trifluoromethyl substituent at the 4th position of the phenyl ring bound to the 6th position of the triazolothiadiazine ring system of compound **11,** which is one of the active inventive compounds, and the fluorine substituent at the 3rd position of the phenyl ring bound to the 6th position of the triazolothiadiazine ring system of compound **12,** which is another active inventive compound gave rise to increases in COX-1 and COX-2 inhibition. In the study of Arandkar and Vedula (2019), a compound carrying 4-trifluoromethylphenyl or 3-fluorophenyl substituent at the 6th position of the triazolothiadiazine ring is not present. Moreover, as healthy cells are not used in said study, it was not determined whether the anticancer effects of these active compounds are selective or not. Any kind of study directed to explaining the anticancer action mechanisms of active compounds was not carried out.

It was determined that the reason for compounds **9, 12** and **14** to cause moderate Akt inhibition in comparison to cisplatin and GSK690693 was that the trifluoromethyl, phenyl substituents at the 4th position and the fluorine substituent at the 3rd position of the phenyl ring bound to the 6th position of the triazolothiadiazine ring increased Akt inhibitory activity.

It was determined that the reason for compound **14** (IC₅₀= 2.55±0.21 µg/mL) to exhibit potent ROCK inhibition in comparison to cisplatin (29.84%) was that the biphenyl substituent bound to the 6th position of the triazolothiadiazine ring system increased ROCK inhibitory activity.

### Definition of the Figures Describing the Invention

**Figure 1****:** Docking poses and interactions of the synthesized compounds at the active site of COX-2 enzyme
**Figure 2****:** Docking poses and interactions of the synthesized compounds at the active site of Akt enzyme
**Figure 3****:** Docking poses and interactions of the synthesized compounds at the active site of ROCK enzyme
**Figure 4****:** IR spectrum of Compound **1.**
**Figure 5****:** ¹H NMR spectrum of Compound **1.**
**Figure 6****:** ¹³C NMR spectrum of Compound **1.**
**Figure 7****:** Mass spectrum of Compound **1.**
**Figure 8****:** IR spectrum of Compound **2.**
**Figure 9****:** ¹H NMR spectrum of Compound **2.**
**Figure 10****:** ¹³C NMR spectrum of Compound **2.**
**Figure 11****:** Mass spectrum of Compound **2.**
**Figure 12****:** IR spectrum of Compound **3.**
**Figure 13****:** ¹H NMR spectrum of Compound **3.**
**Figure 14****:** ¹³C NMR spectrum of Compound **3.**
**Figure 15****:** Mass spectrum of Compound **3.**
**Figure 16****:** IR spectrum of Compound **4.**
**Figure 17****:** ¹H NMR spectrum of Compound **4.**
**Figure 18****:** ¹³C NMR spectrum of Compound **4.**
**Figure 19****:** Mass spectrum of Compound **4.**
**Figure 20****:** IR spectrum of Compound **5.**
**Figure 21****:** ¹H NMR spectrum of Compound **5.**
**Figure 22****:** ¹³C NMR spectrum of Compound **5.**
**Figure 23****:** Mass spectrum of Compound **5.**
**Figure 24****:** IR spectrum of Compound **6.**
**Figure 25****:** ¹H NMR spectrum of Compound **6.**
**Figure 26****:** ¹³C NMR spectrum of Compound **6.**
**Figure 27****:** Mass spectrum of Compound **6.**
**Figure 28****:** IR spectrum of Compound **7.**
**Figure 29****:** ¹H NMR spectrum of Compound **7.**
**Figure 30****:** ¹³C NMR spectrum of Compound **7.**
**Figure 31****:** Mass spectrum of Compound **7.**
**Figure 32****:** IR spectrum of Compound **8.**
**Figure 33****:** ¹H NMR spectrum of Compound **8.**

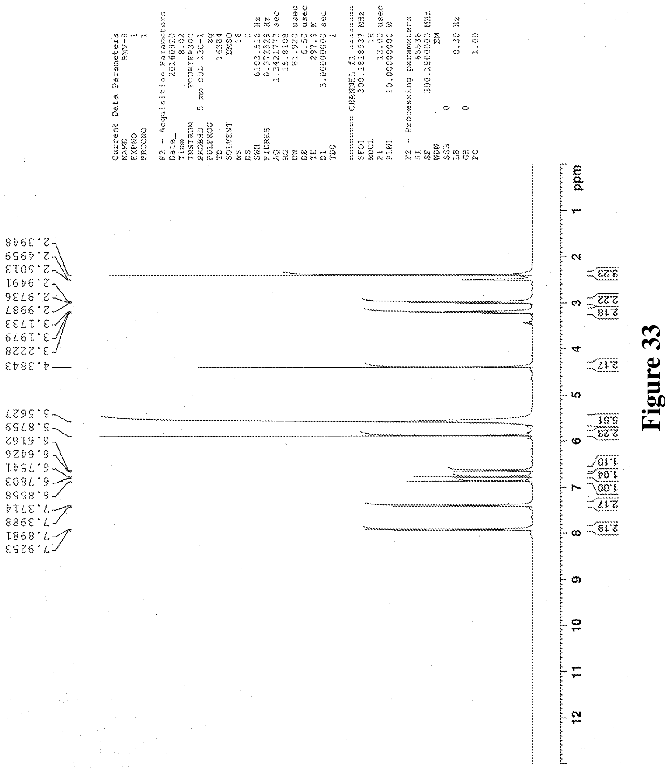

**Figure 33****:** ¹³C NMR spectrum of Compound **8.**

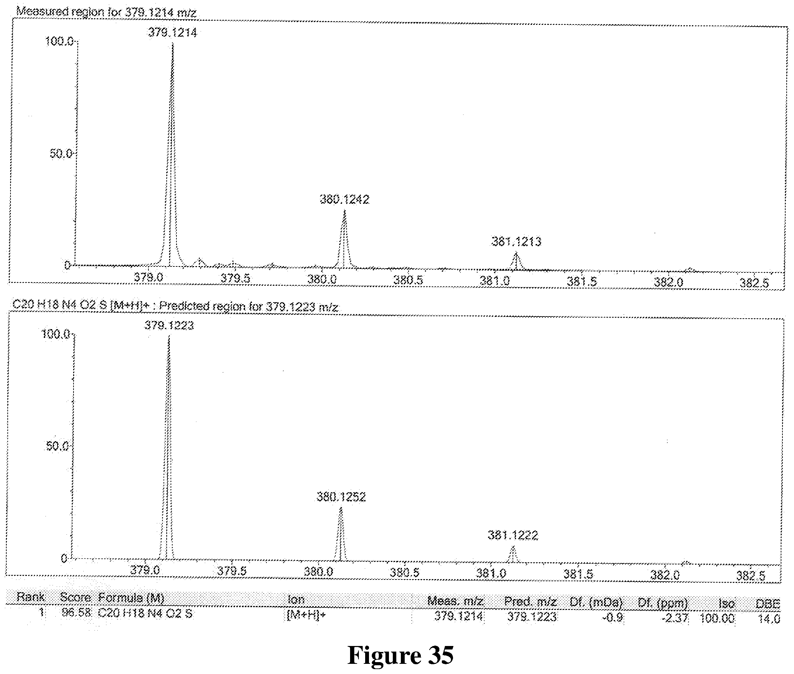

**Figure 35****:** Mass spectrum of Compound **8.**

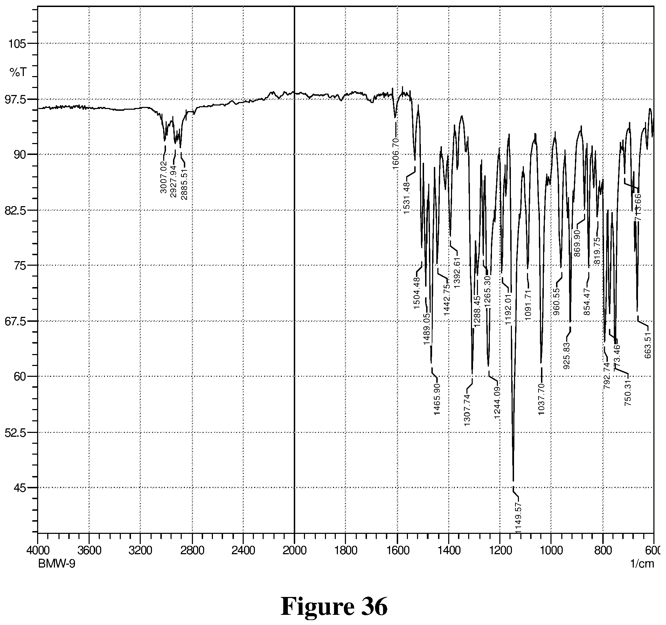

**Figure 36****:** IR spectrum of Compound **9.**

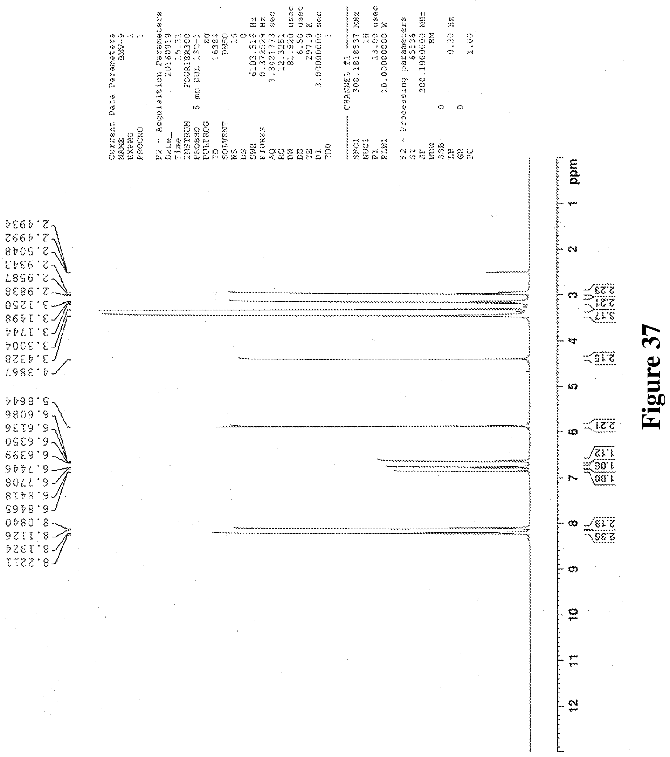

**Figure 37****:** ¹H NMR spectrum of Compound **9.**

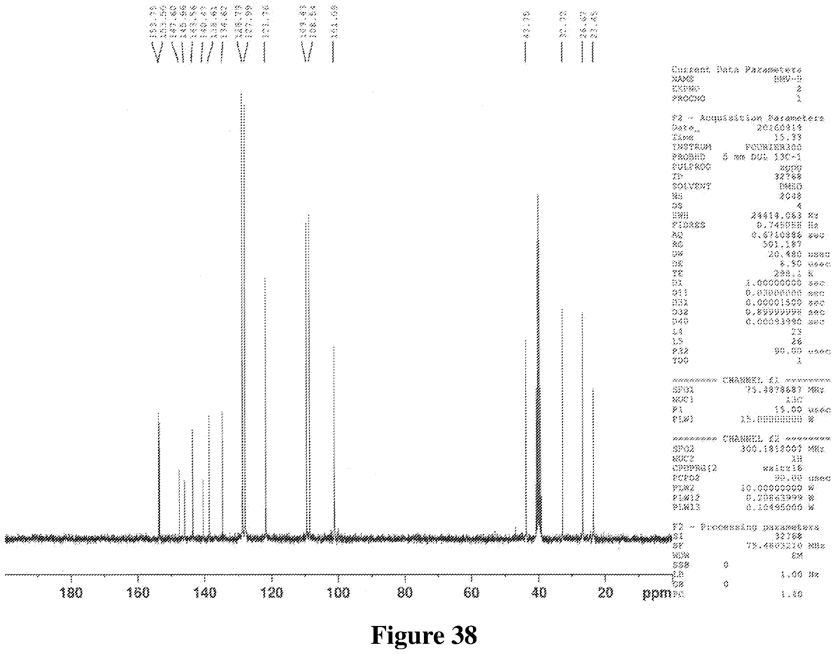

**Figure 38****:** ¹³C NMR spectrum of Compound **9.**

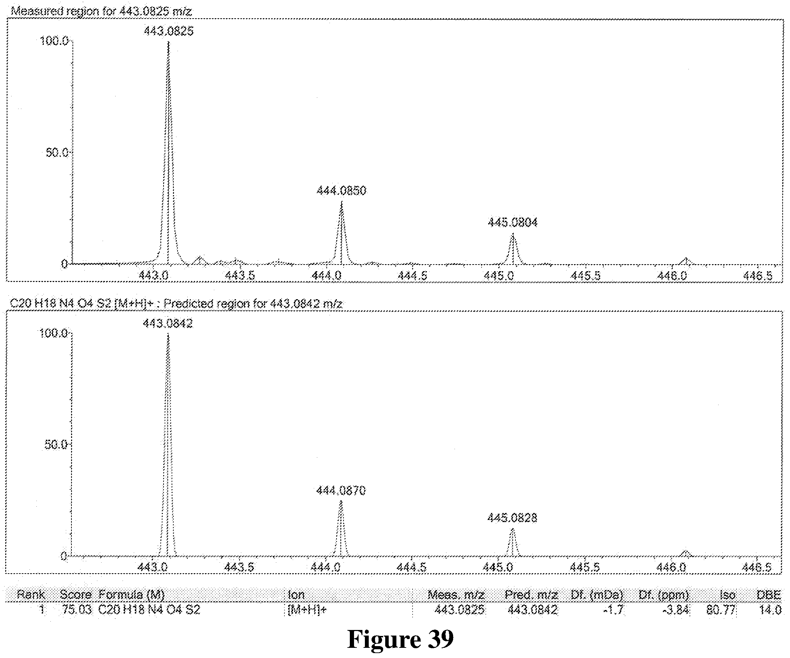

**Figure 39****:** Mass spectrum of Compound **9.**

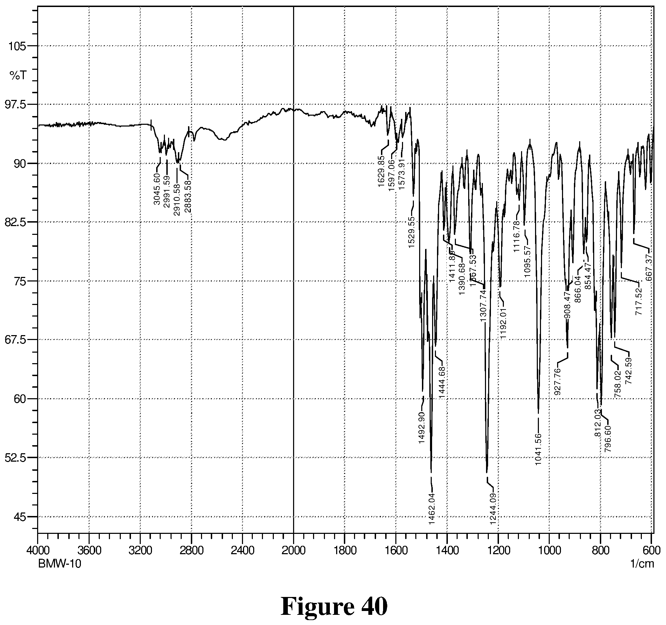

**Figure 40****:** IR spectrum of Compound **10.**

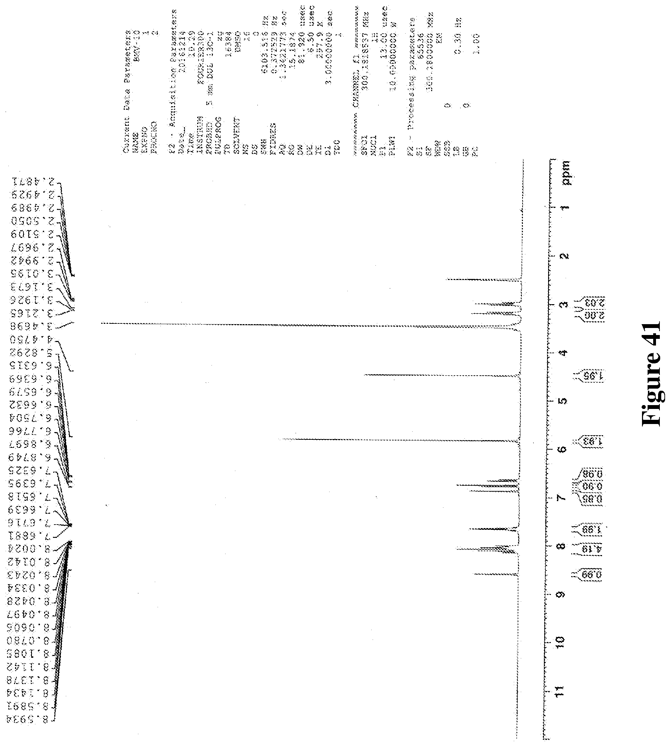

**Figure 41****:** ¹H NMR spectrum of Compound **10.**

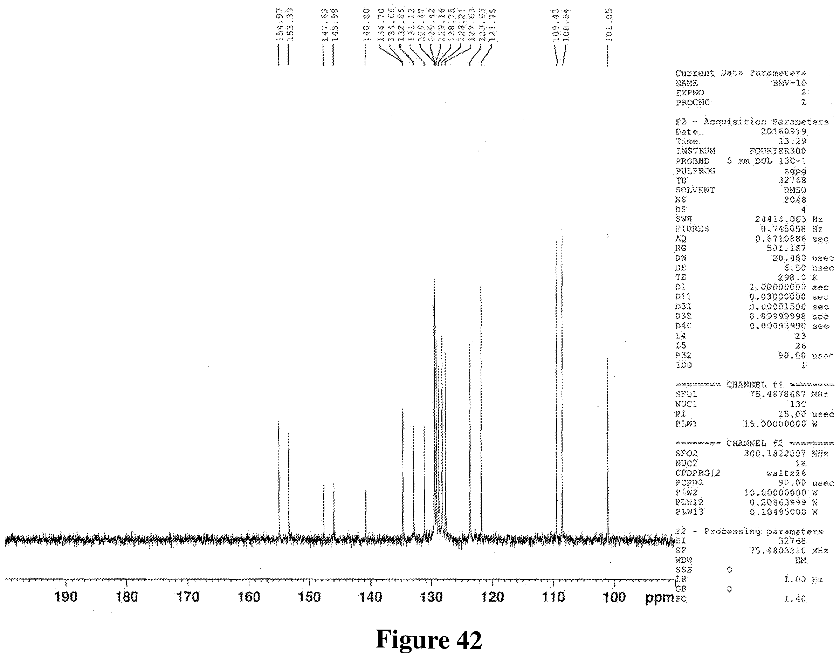

**Figure 42****:** ¹³C NMR spectrum of Compound **10.**

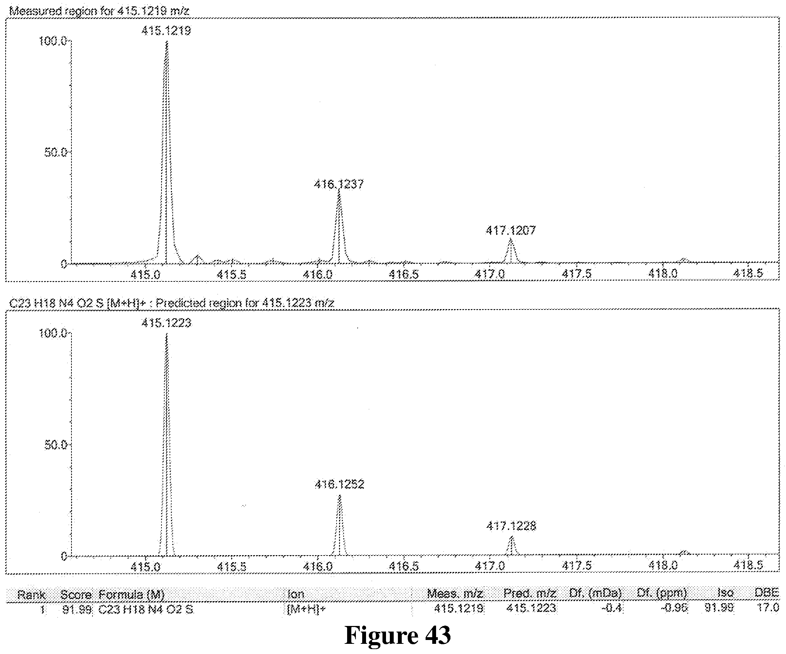

**Figure 43****:** Mass spectrum of Compound **10.**

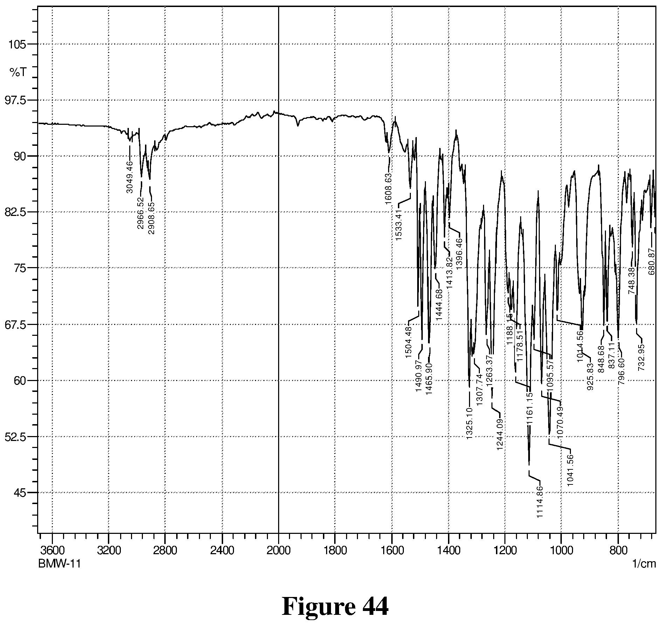

**Figure 44****:** IR spectrum of Compound **11.**

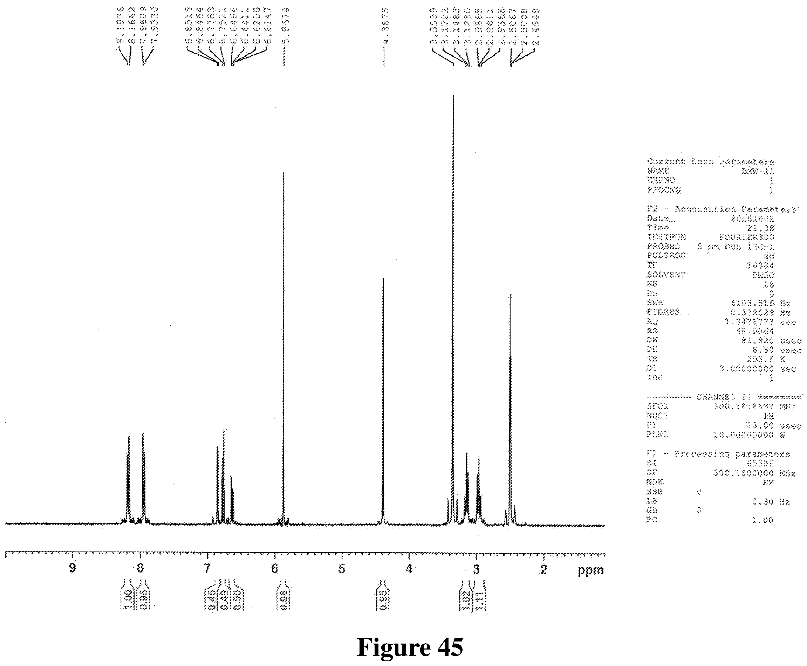

**Figure 45****:** ¹H NMR spectrum of Compound **11.**

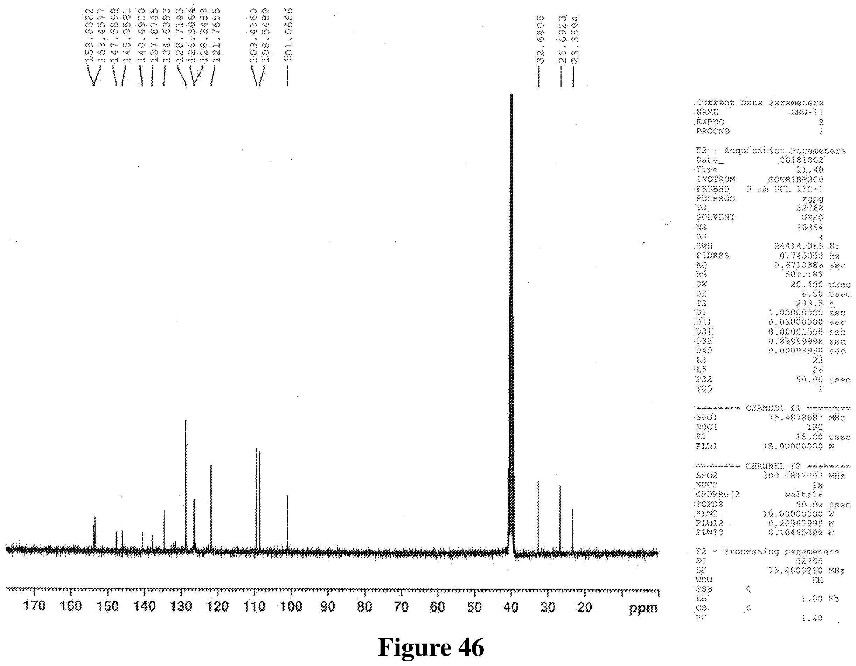

**Figure 46****:** ¹³C NMR spectrum of Compound **11.**

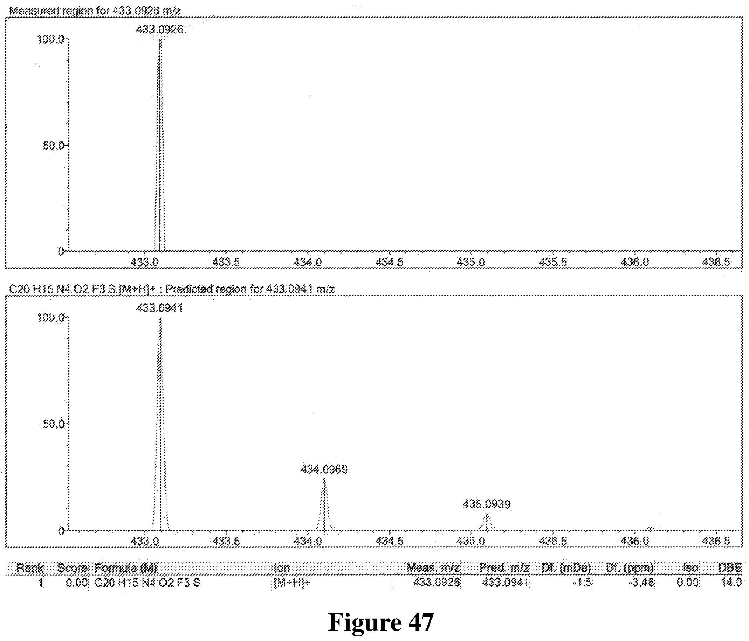

**Figure 47****:** Mass spectrum of Compound **11.**

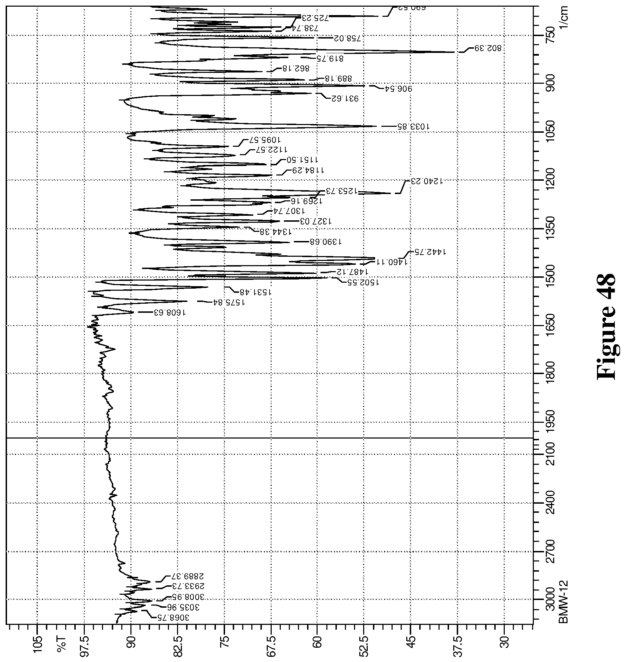

**Figure 48****:** IR spectrum of Compound **12.**

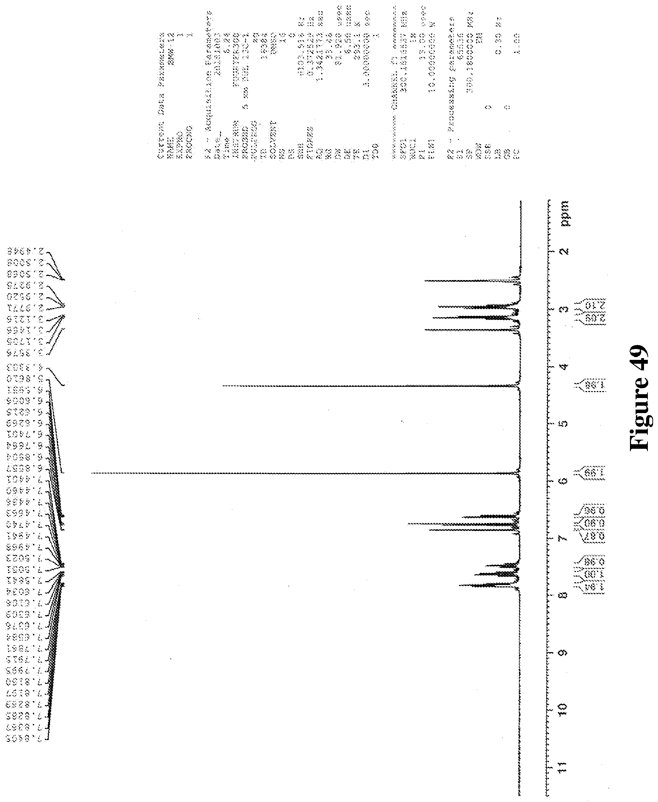

**Figure 49****:** ¹H NMR spectrum of Compound **12.**

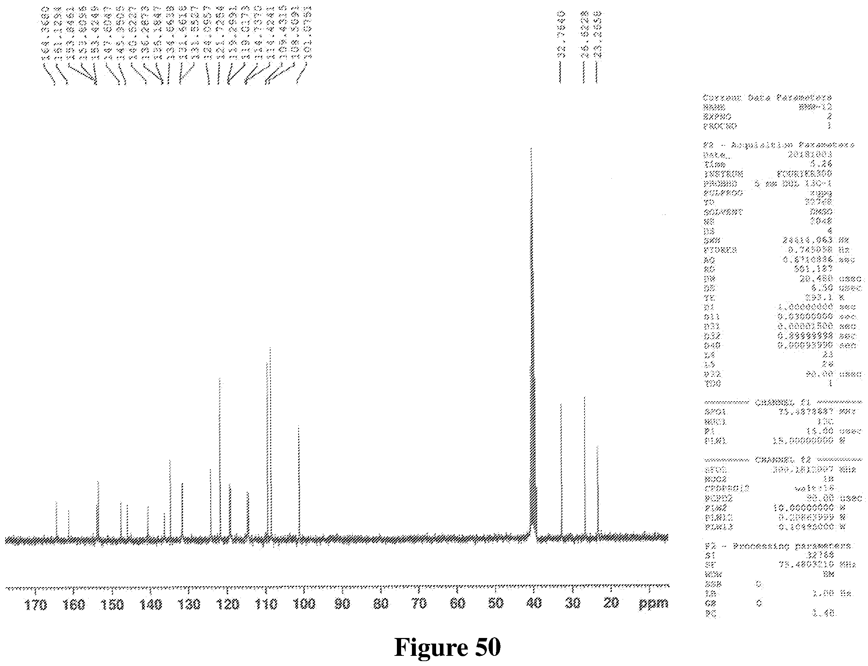

**Figure 50****:** ¹³C NMR spectrum of Compound **12.**

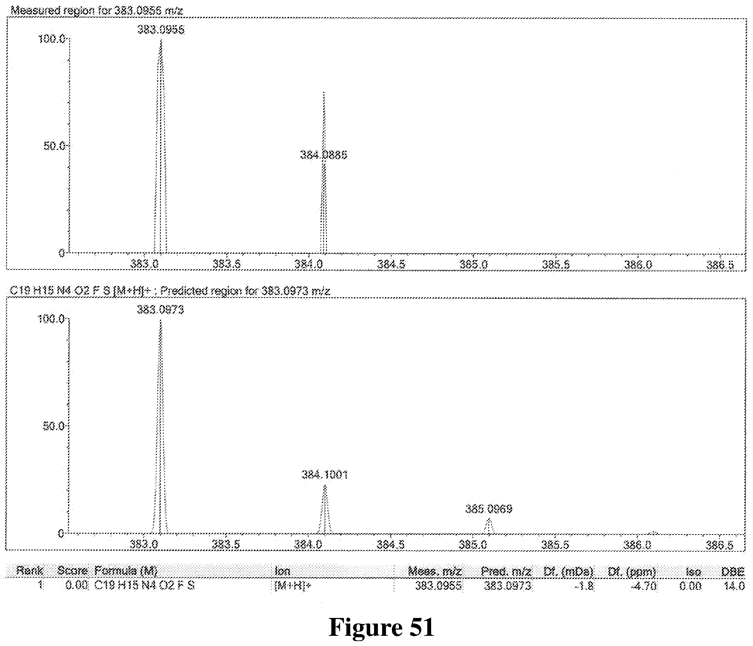

**Figure 51****:** Mass spectrum of Compound **12.**

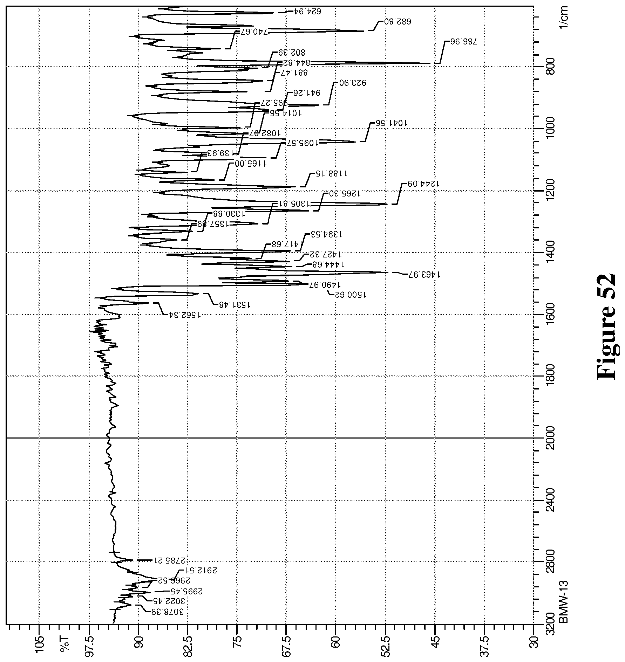

**Figure 52****:** IR spectrum of Compound **13.**

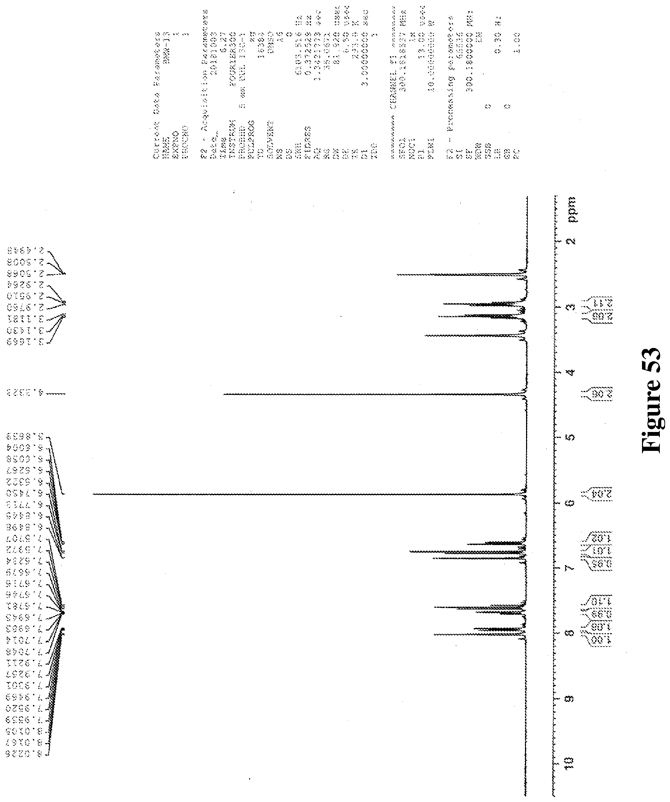

**Figure 53****:** ¹H NMR spectrum of Compound **13.**

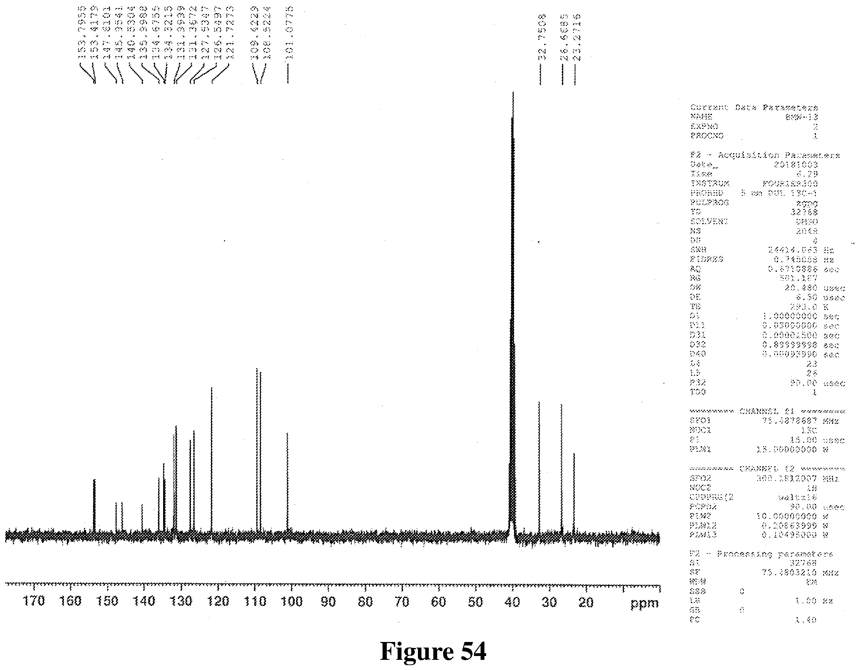

**Figure 54****:** ¹³C NMR spectrum of Compound **13.**

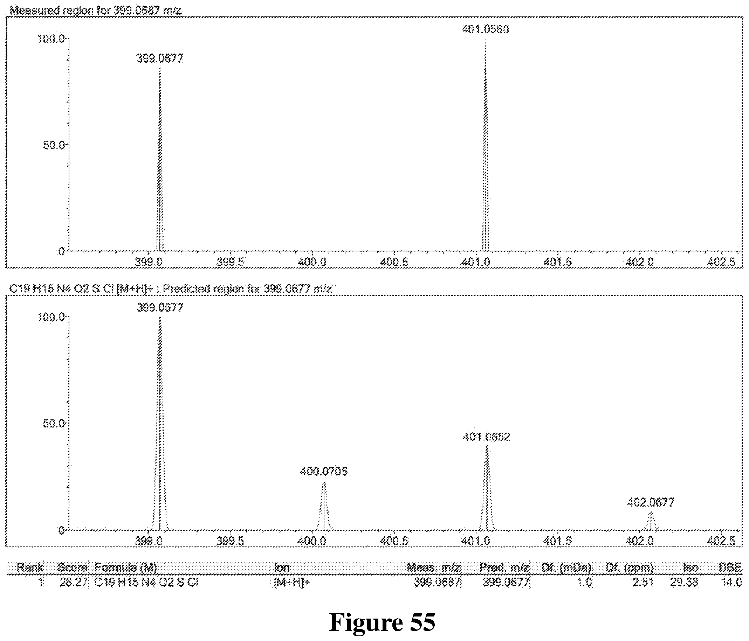

**Figure 55****:** Mass spectrum of Compound **13.**

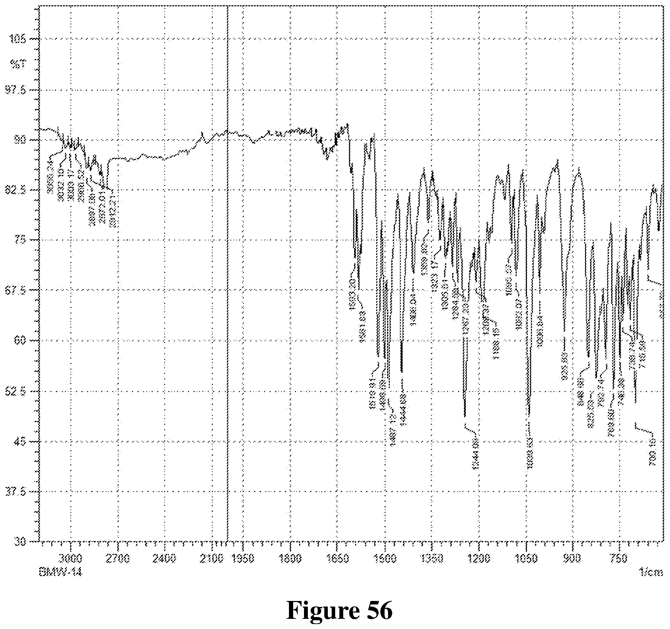

**Figure 56****:** IR spectrum of Compound **14.**

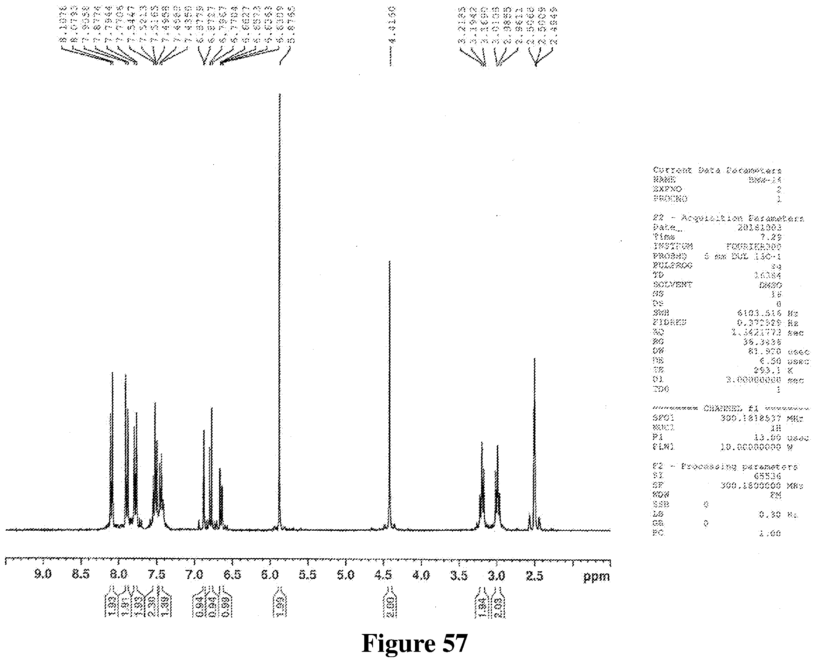

**Figure 57****:** ¹H NMR spectrum of Compound **14.**

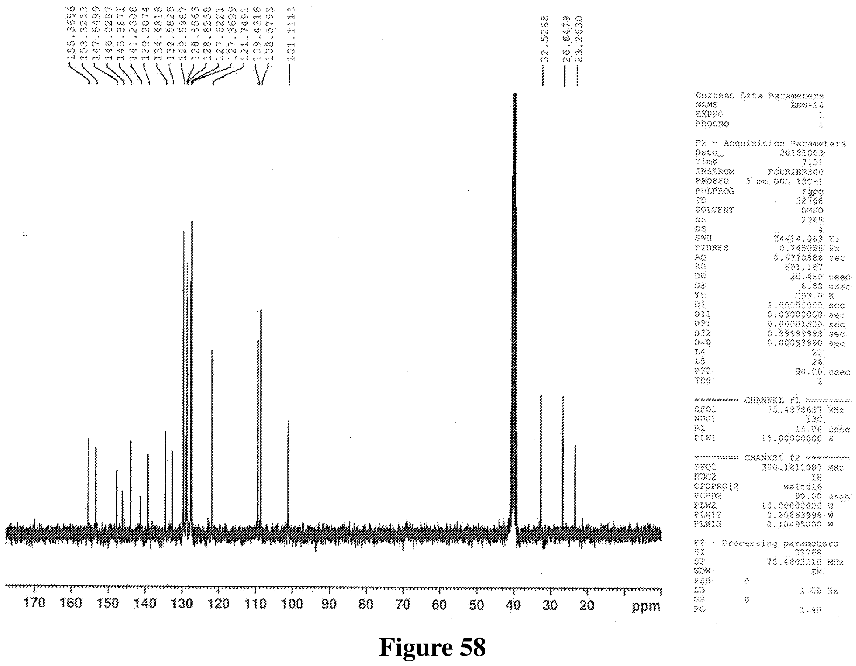

**Figure 58****:** ¹³C NMR spectrum of Compound **14.**

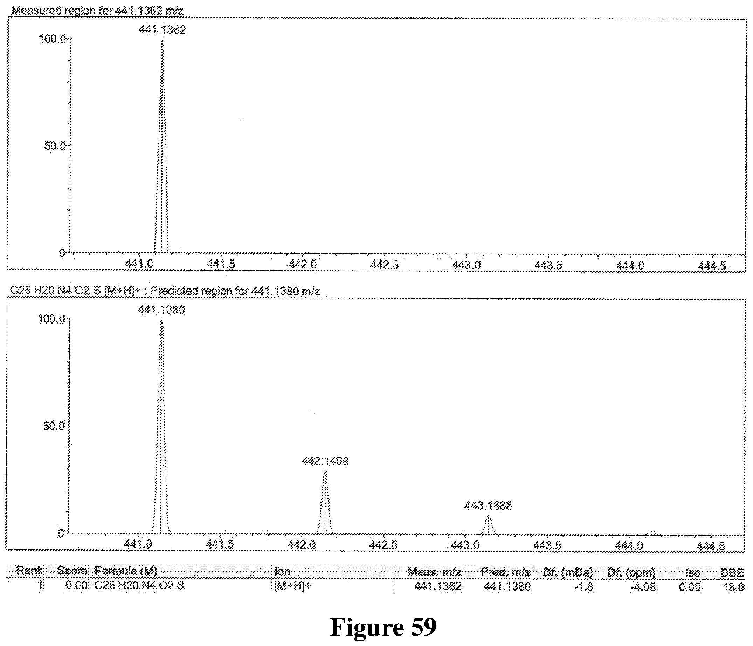

**Figure 59****:** Mass spectrum of Compound **14.**

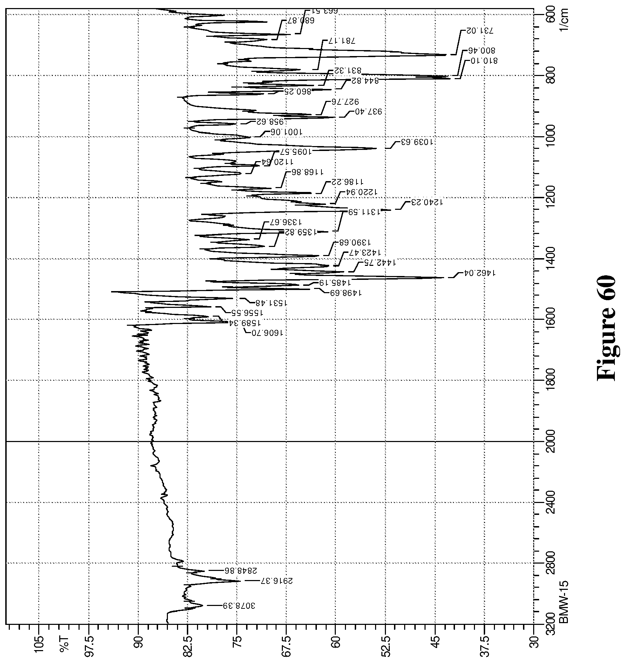

**Figure 60****:** IR spectrum of Compound **15.**

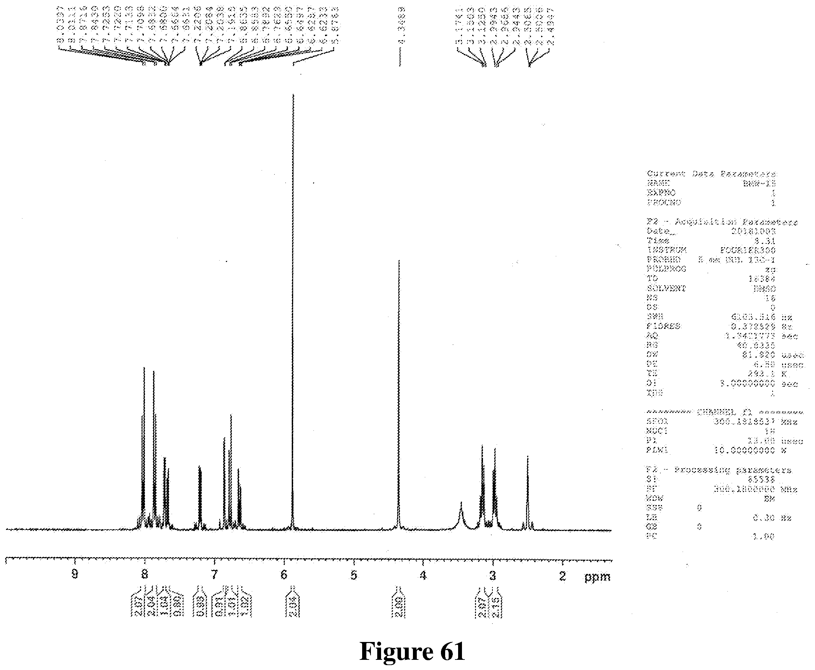

**Figure 61****:** ¹H NMR spectrum of Compound **15.**

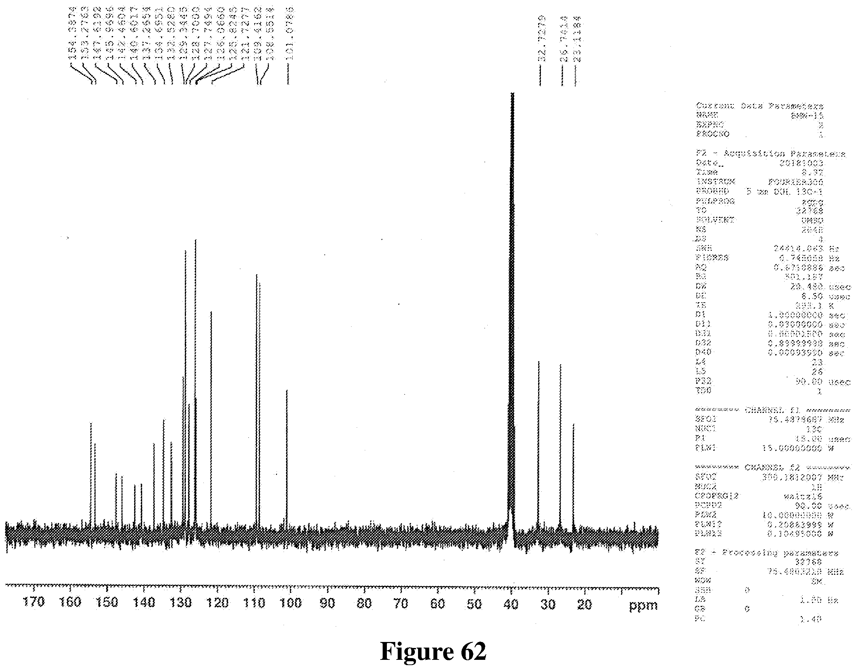

**Figure 62****:** ¹³C NMR spectrum of Compound **15.**

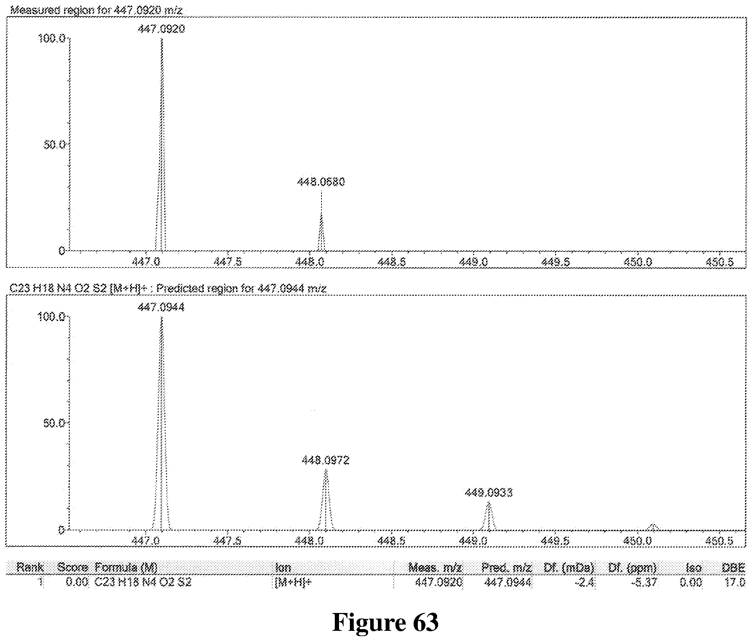

**Figure 63****:** Mass spectrum of Compound **15.**

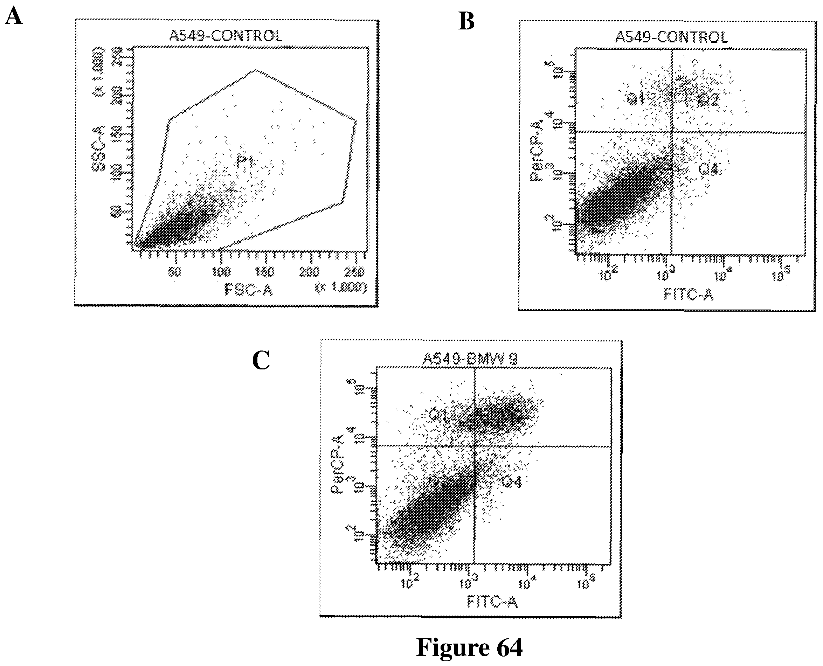

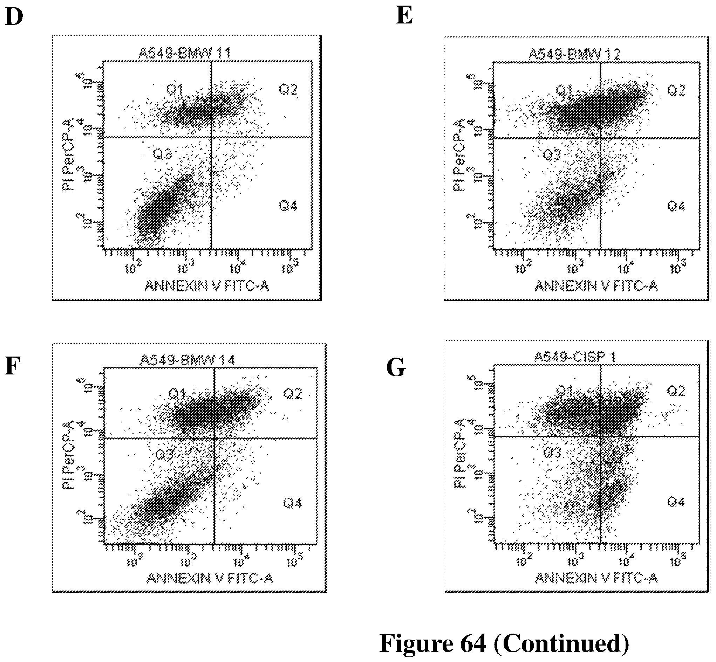

**Figure 64****:** The flow cytometry analysis of compounds **9, 11, 12, 14** and cisplatin at different concentrations on A549 cells (Q1: Necrotic cells, Q2: Late apoptotic cells, Q3: Viable cells, Q4: Early apoptotic cells). **A)** Control (shows all cells in analysis), **B**) Control (shows viable, apoptotic and necrotic cells in untreated cells), **C)** Compound **9, D)** Compound **11, E)** Compound **12, F)** Compound **14, G)** Cisplatin

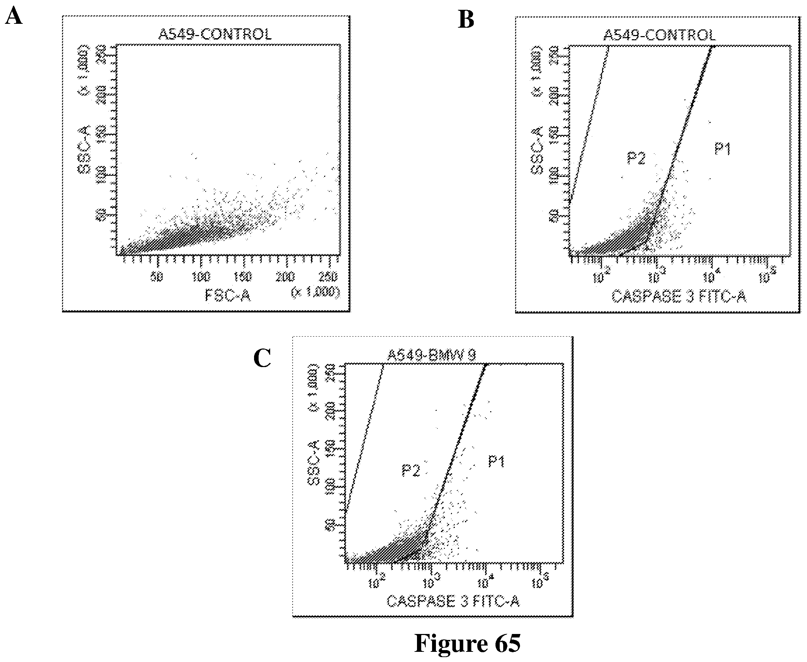

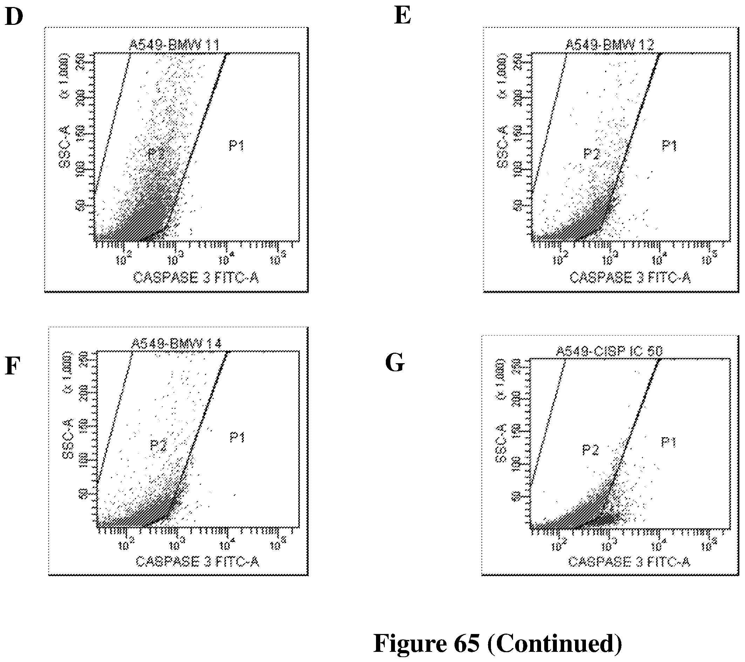

**Figure 65****:** The flow cytometry quadrant analysis of the active caspase-3 activity of compounds **9, 11, 12, 14** and cisplatin (5.75, 0.73, 0.80, 2.63 and 23 µg/mL) on A549 cells (P1: Caspase-3 positive cells, P2: Caspase-3 negative cells). **A)** Control (shows all cells in analysis), **B)** Control (shows caspase-3 positive and negative cells in untreated cells), **C)** Compound **9, D)** Compound **11, E)** Compound **12, F)** Compound **14, G)** Cisplatin

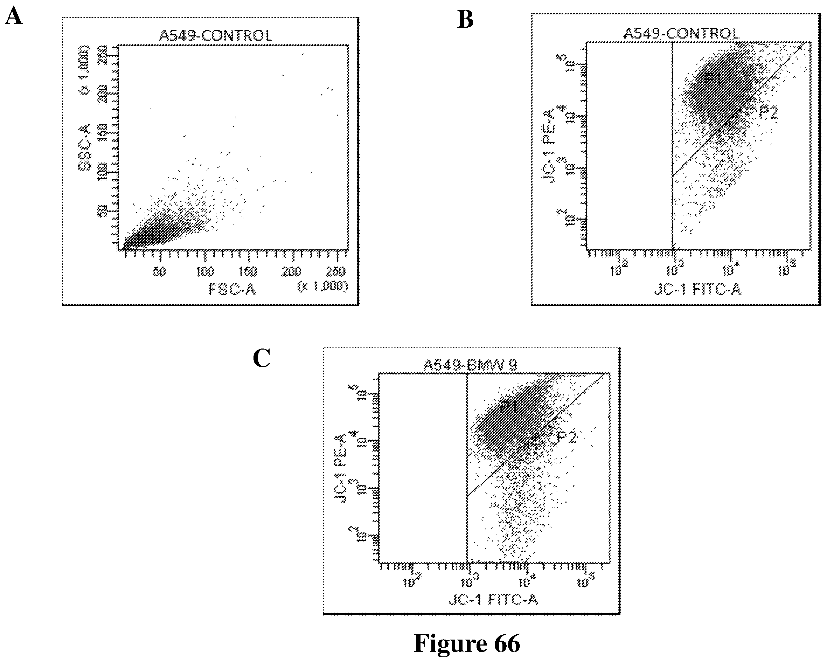

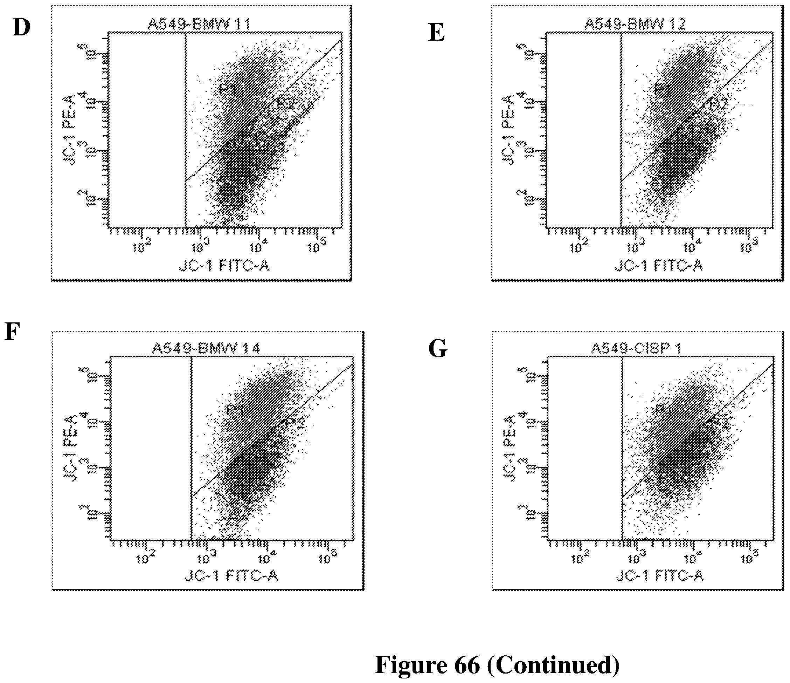

**Figure 66****:** The flow cytometry quadrant analysis of mitochondrial membrane polarized/depolarized effects of compounds **9, 11, 12, 14** and cisplatin (5.75, 0.73, 0.80, 2.63 and 23 µg/mL) on A549 cells (P1: Mitochondrial membrane polarized cells, P2: Mitochondrial membrane depolarized cells). **A)** Control (shows all cells in analysis), **B)** Control (shows mitochondrial membrane polarized and depolarized cells in untreated cells), **C)** Compound **9, D)** Compound **11, E)** Compound **12, F)** Compound **14, G)** Cisplatin

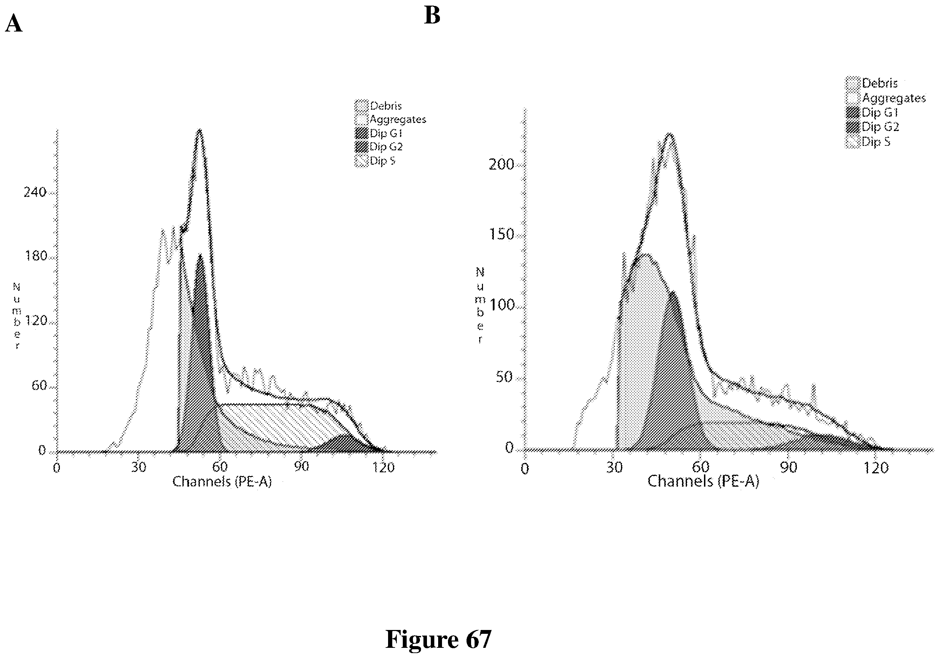

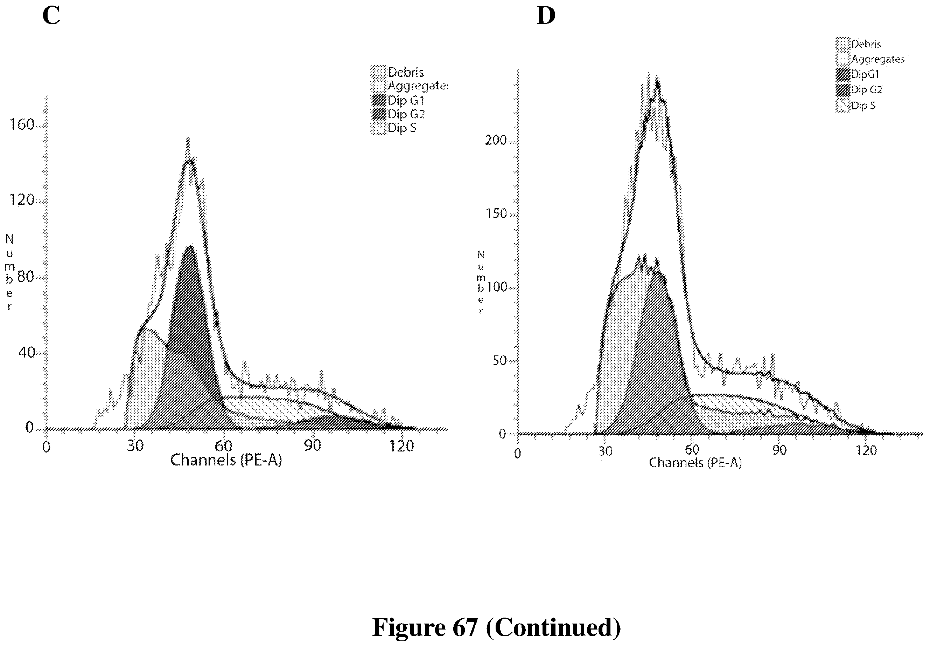

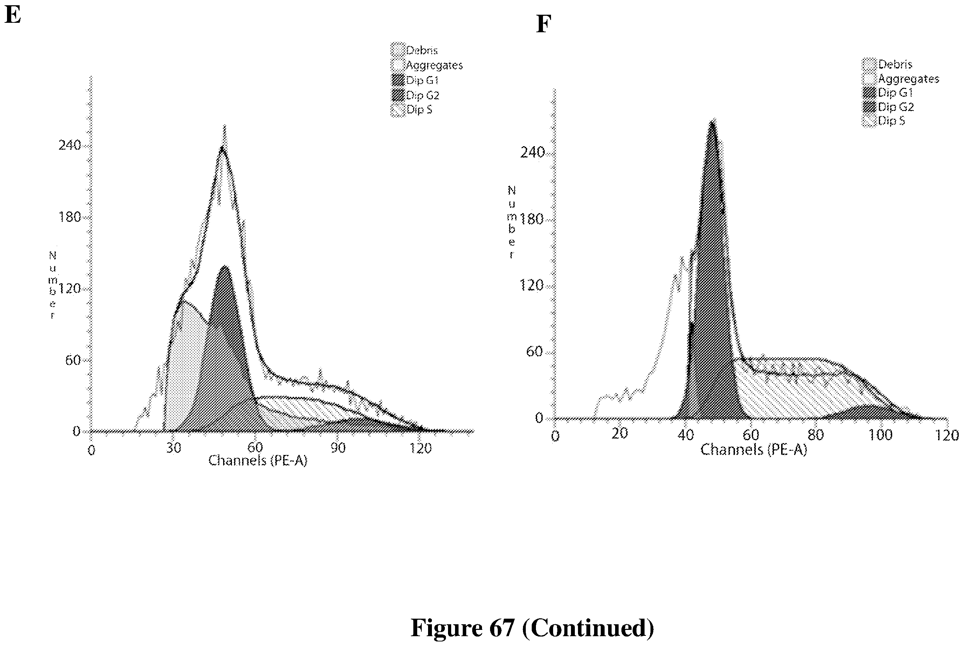

**Figure 67****:** Cell cycle distribution analysis of A549 cells treated with IC₅₀ doses of compounds **9, 11, 12, 14** and cisplatin for 24 hours. 10,000 cells were analyzed for each sample. **A)** Control, **B)** Compound **9, C)** Compound **11, D)** Compound **12, E)** Compound **14, F)** Cisplatin

### Detailed Description of the Invention

The invention is related to 15 novel 3-[2-(1,3-benzodioxole-5-yl)ethyl)]-6-aryl-7*H*-1,2,4-triazolo[3,4-*b*]-1,3,4-thiadiazine derivatives and the selective cytotoxic and apoptotic effects of these compounds through the inhibition of the COX-2, Akt and/or ROCK enzymes, which are known as important target enzymes in cancer pathogenesis, for the treatment of lung cancer.

The present invention is a compound having the formula (I), or pharmaceutically acceptable salts and/or solvates thereof, characterized by; 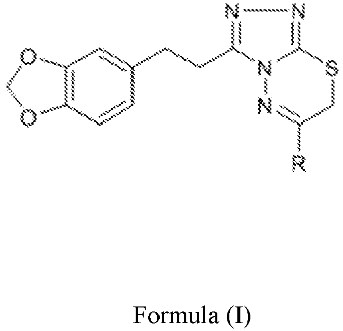 **R:** Phenyl, 4-Nitrophenyl, 4-Cyanophenyl, 4-Fluorophenyl, 4-Chlorophenyl, 4-Bromophenyl, 4-Methoxyphenyl, 4-Methylphenyl, 4-Methylsulfonylphenyl, Naphthalene-2-yl, 4-Trifluoromethylphenyl, 3-Fluorophenyl, 3-Chlorophenyl, Biphenyl-4-yl or 4-(Thiophen-2-yl)phenyl.

The present invention is a compound having the formula (I), or a pharmaceutical composition comprising the pharmaceutically acceptable salts and/or solvates thereof, which can be used in the preparation of pharmaceuticals for the treatment of lung cancer.

### A. Synthesis of 3-[2-(1,3-Benzodioxole-5-yl)ethyl)]-6-aryl-7H-1,2,4-triazolo[3,4-b]-1,3,4-thiadiazine derivatives

### A.1. Preparation of 4-Amino-5-[2-(1,3-benzodioxole-5-yl)ethyl]-2,4-dihydro-3H-1,2,4-triazole-3-thione (Method A)

The equivalent amounts of 3-(1,3-benzodioxole-5-yl)propanoic acid and thiocarbohydrazide were stirred at 170-175 °C in an oil bath for 2 hours. The solid compound obtained was washed with water and was crystallized from ethanol after being dried (Scheme 1 and 2).

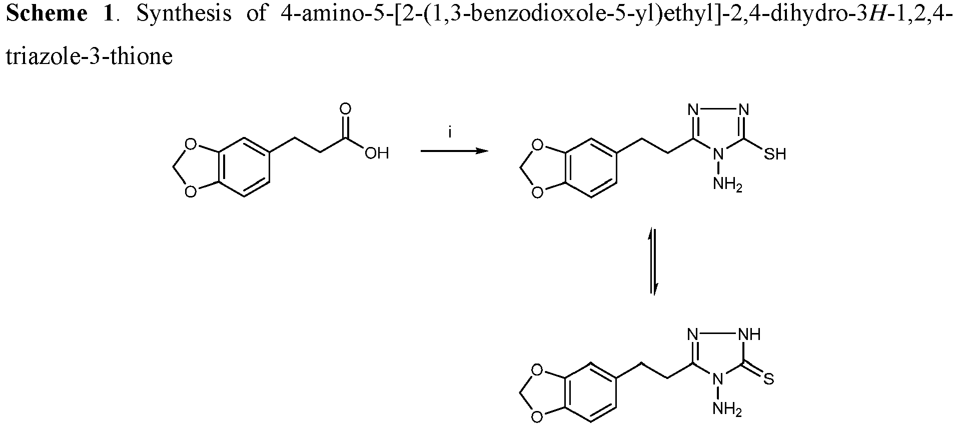

### (i) Thiocarbohydrazide, oil bath, 170-175 °C, 2 hours

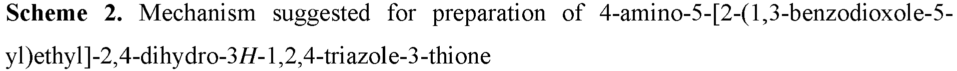

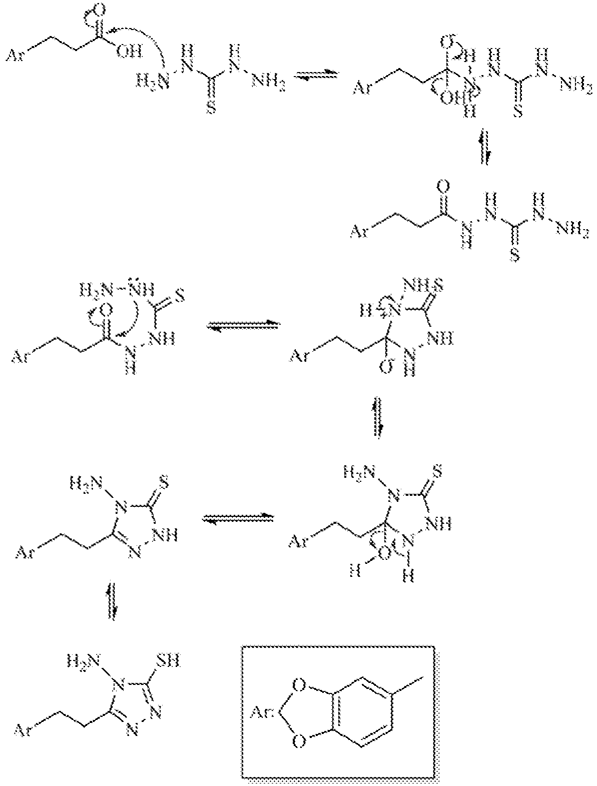

### A.2. Preparation of 3-[2-(1,3-Benzodioxole-5-yl)ethyl)]-6-aryl-7H-1,2,4-triazolo[3,4-b]-1,3,4-thiadiazines (Method B)

The equivalent amounts of 4-amino-5-[2-(1,3-benzodioxole-5-yl)ethyl]-2,4-dihydro-3*H*-1,2,4-triazolo-3-thione and 2-bromo-1-arylethanone derivatives were heated for 6 hours under reflux in ethanol. The precipitate was collected by filtration. After being dried, it was crystallized with ethanol (Scheme 3).

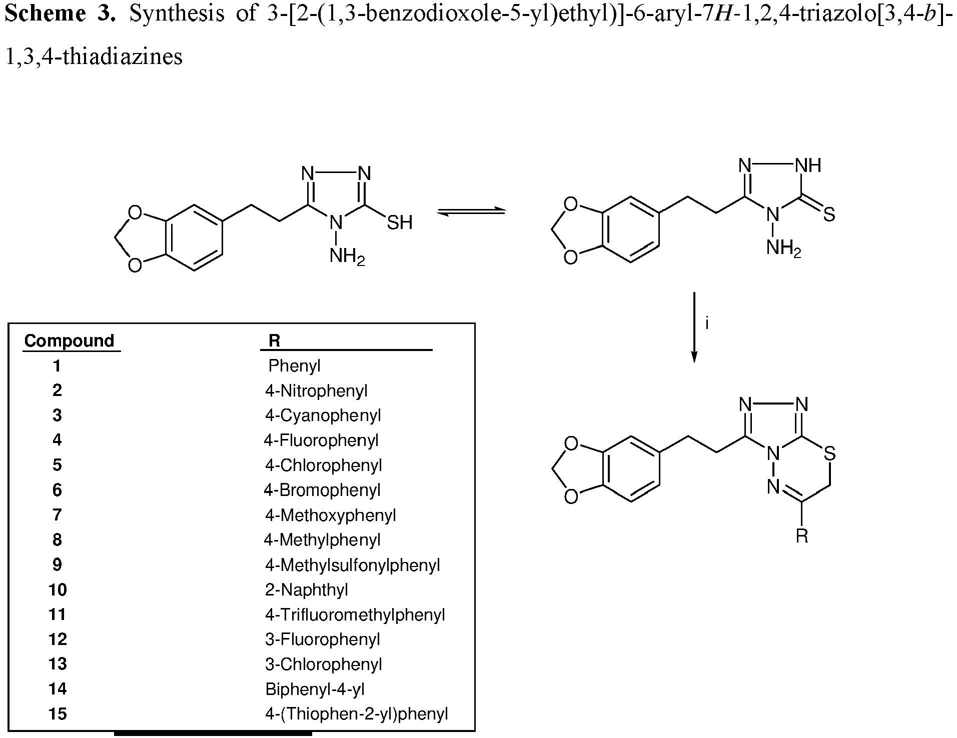

### (i) RCOCH₂Br, ethanol, heating under reflux, 6 hours.

In the mechanism suggested for preparation of 3-[2-(1,3-benzodioxole-5-yl)ethyl)]-6-(aryl)-7H-1,2,4-triazolo[3,4-*b*]-1,3,4-thiadiazines, it is suggested that an S-alkylation product is obtained *via* the electrophilic substitution reaction of the thione group at the 3rd position of aminomercaptotriazole with phenacyl bromides in the initial step, and then there was a ring closure with the removal of one mol water from the molecule through the condensation reaction of the amine group at the 4th position of the triazole ring with phenacyl carbonyl (Scheme 4).

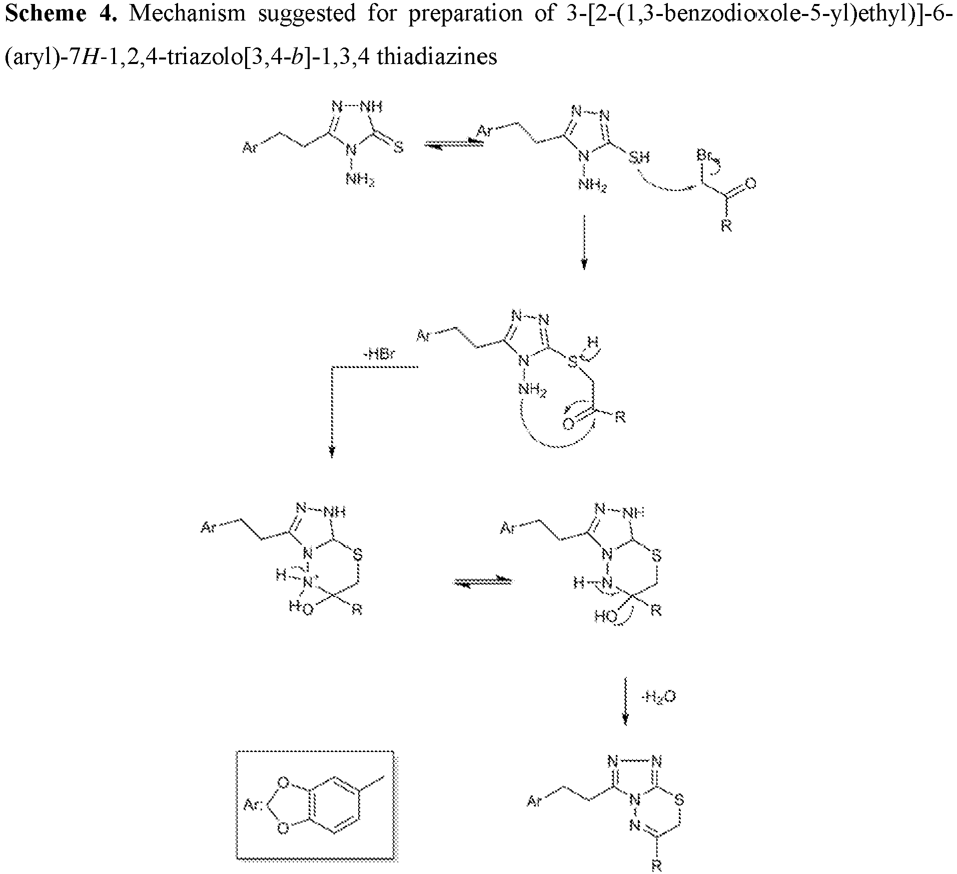

This invention is related to targeted novel 1,2,4-triazolo[3,4-*b*]-1,3,4-thiadiazine derivatives, hydrates, solvates, pharmaceutically acceptable salts, production methods and indications thereof for lung cancer treatment, and the compounds related to this invention are listed below:

### 3-[2-(1,3-Benzodioxole-5-yl)ethyl]-6-phenyl-7H-[1,2,4]triazolo[3,4-b][1,3,4]thiadiazine (Compound 1)

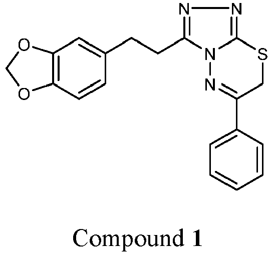

It was synthesized according to Method B using 4-amino-5-[2-(1,3-benzodioxole-5-yl)ethyl]-2,4-dihydro-3*H*-1,2,4-triazole-3-thione (0.001 mol; 0.3000 g) and 2-bromoacetophenone (0.001 mol; 0.2259 g).

### Yield: 77%, M.p.: 111.1 °C

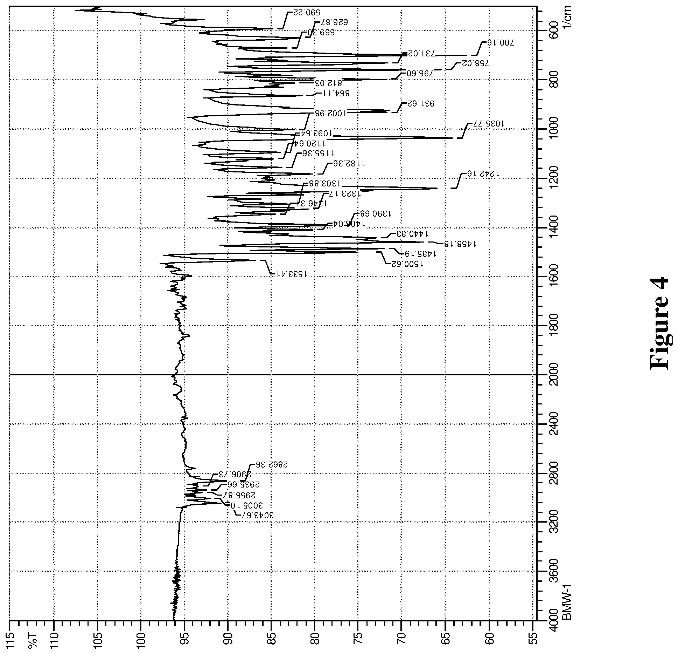

IR νₘₐₓ (cm⁻¹): 3043.67, 3005.10 (Aromatic C-H stretching bands), 2956.87, 2935.66, 2906.73, 2862.36 (Aliphatic C-H stretching bands), 1533.41, 1500.62, 1485.19, 1458.18, 1440.83 (C=C and C=N stretching bands), 1406.04, 1390.68, 1346.31, 1323.17, 1303.88, 1242.16, 1182.36, 1155.36, 1120.64, 1093.64, 1035.77, 1002.98 (C-H bending, C-N, C-O stretching and aromatic C-H in plane bending bands), 931.62, 864.11, 812.03, 796.60, 758.02, 731.02, 700.16, 669.30, 626.87, 590.22 (Aromatic C-H out of plane bending bands and C-S stretching band) (Figure 4).

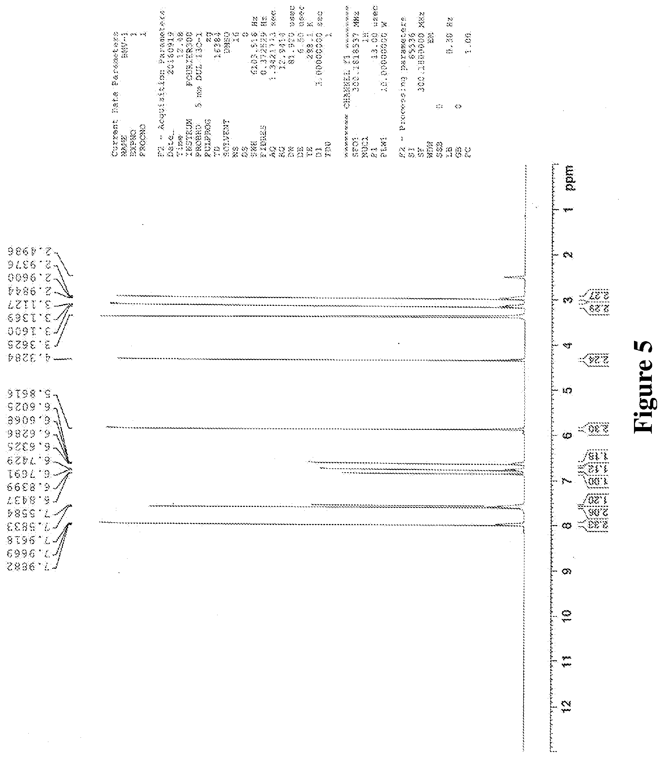

¹H NMR (300 MHz, DMSO-*d₆*): 2.96 (t, *J*= 6.72 Hz, *J*= 7.32 Hz, 2H), 3.14 (t, *J*= 6.93 Hz, *J*= 7.26 Hz, 2H), 4.33 (s, 2H), 5.86 (s, 2H), 6.62 (d, *J*= 7.83 Hz, 1H), 6.76 (d, *J*= 7.86 Hz, 1H), 6.84 (s, 1H), 7.57 (d, *J*= 7.47 Hz, 3H), 7.98 (d, *J*= 7.92 Hz, 2H) (Figure 5).

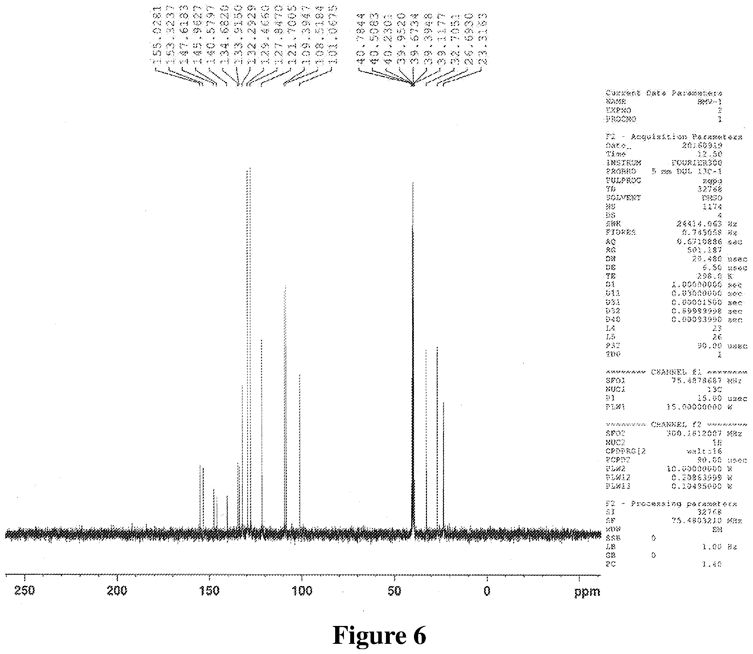

¹³C NMR (75 MHz, DMSO-*d₆*): 23.32 (CH₂), 26.69 (CH₂), 32.71 (CH₂), 101.07 (CH₂), 108.52 (CH), 109.39 (CH), 121.70 (CH), 127.85 (2CH), 129.47 (2CH), 132.29 (CH), 133.92 (C), 134.68 (C), 140.58 (C), 145.96 (C), 147.62 (C), 153.32 (C), 155.03 (C) (Figure 6).

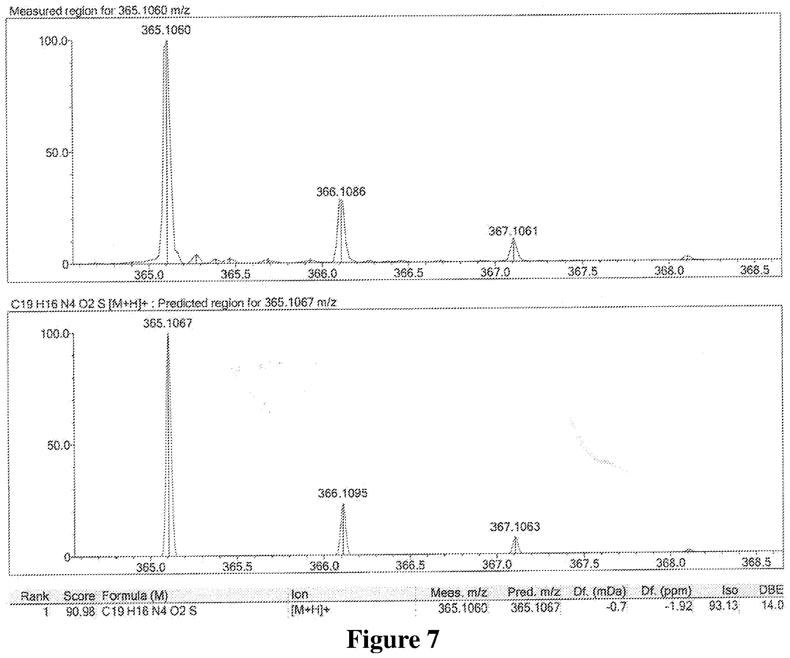

HRMS (ESI) (m/z): [M+H]⁺ calculated for C₁₉H₁₆N₄O₂S: 365.1067, found: 365.1060 (Figure 7).

### 3-[2-(1,3-Benzodioxole-5-yl)ethyl]-6-(4-nitrophenyl)-7H-[1,2,4]triazolo[3,4-b][1,3,4]thiadiazine (Compound 2)

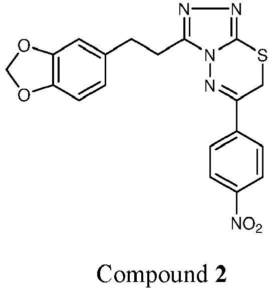

It was synthesized according to Method B using 4-amino-5-[2-(1,3-benzodioxole-5-yl)ethyl]-2,4-dihydro-3*H*-1,2,4-triazole-3-thione (0.001 mol; 0.3000 g) and 2-bromo-4'-nitroacetophenone (0.001 mol; 0.2770 g).

### Yield: 92%, M.p.: 241.7 °C

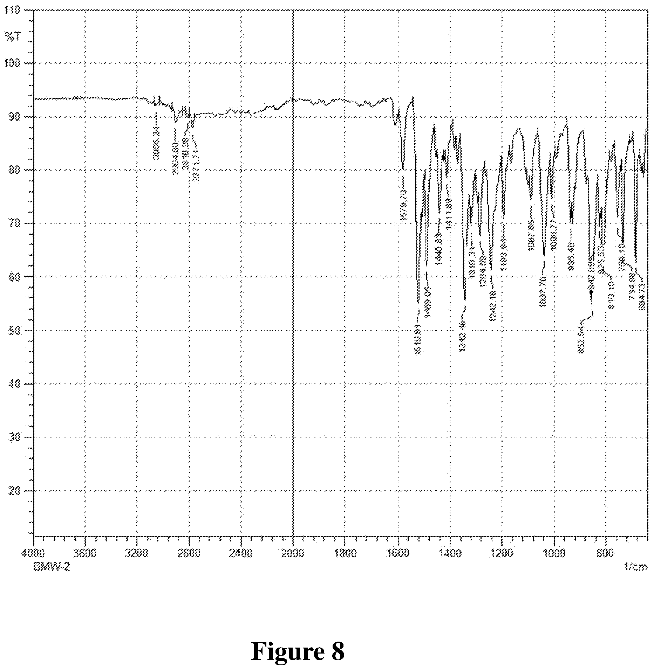

IR νₘₐₓ (cm⁻¹): 3055.24, 3005.10 (Aromatic C-H stretching bands), 2904.80, 2810.28, 2771.71 (Aliphatic C-H stretching bands), 1579.70, 1519.91, 1489.05, 1440.83 (C=C and C=N stretching bands), 1411.89, 1342.46, 1319.31, 1284.59, 1242.16, 1193.94, 1087.85, 1037.70, 1037.70, 1008.77 (C-H bending, C-N, C-O stretching and aromatic C-H in-plane bending bands), 935.48, 852.54, 842.89, 825.53, 810.10, 756.10, 734.88, 684.73 (Aromatic C-H out of plane bending bands and C-S stretching band) (Figure 8).

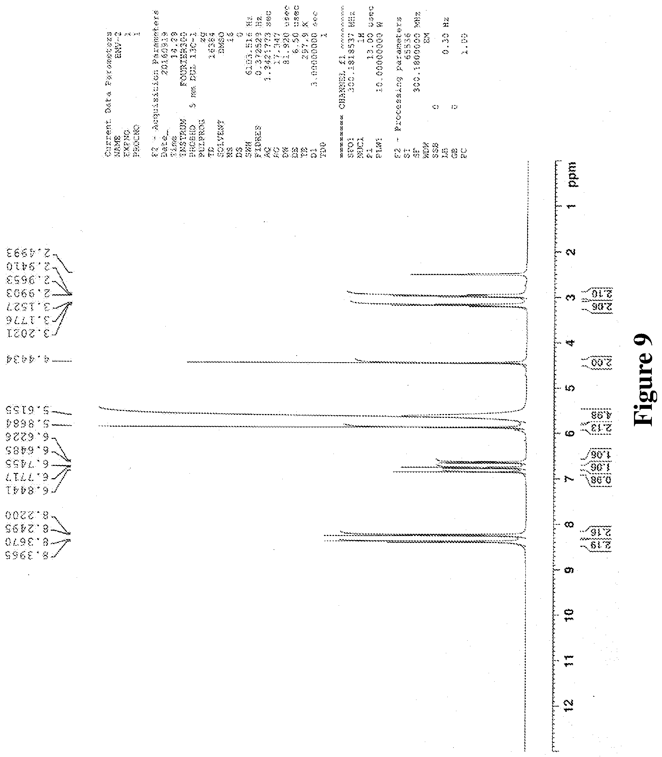

¹H NMR (300 MHz, DMSO-*d₆*): 2.97 (t, *J*= 7.29 Hz, *J*= 7.50 Hz, 2H), 3.18 (t, *J*= 7.35 Hz, *J*= 7.47 Hz, 2H), 4.44 (s, 2H), 5.87 (s, 2H), 6.64 (d, *J*= 7.77 Hz, 1H), 6.76 (d, *J*= 7.86 Hz, 1H), 6.84 (s, 1H), 8.23 (d, *J*= 8.85 Hz, 2H), 8.38 (d, *J*= 8.85 Hz, 2H) (Figure 9).

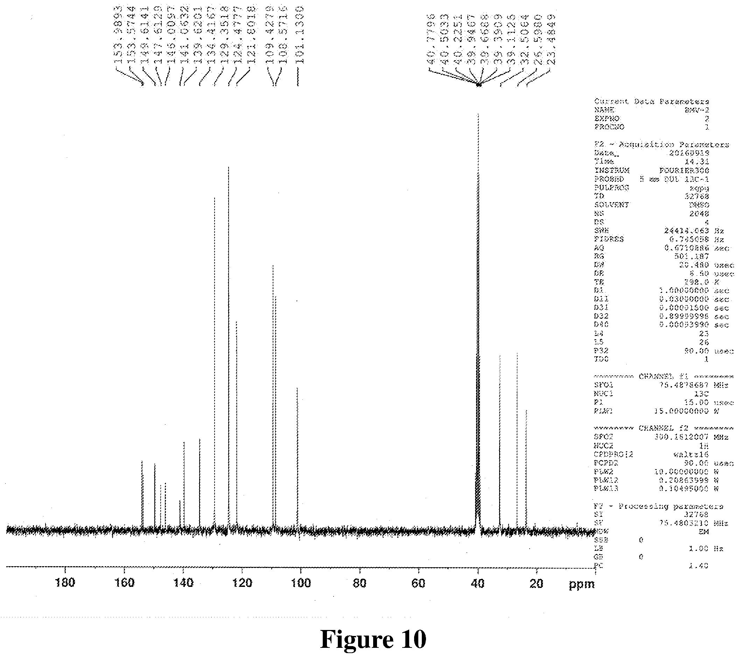

¹³C NMR (75 MHz, DMSO-*d₆*): 23.48 (CH₂), 26.60 (CH₂), 32.51 (CH₂), 101.13 (CH₂), 108.57 (CH), 109.43 (CH), 121.80 (CH), 124.48 (2CH), 129.35 (2CH), 134.42 (C), 139.62 (C), 141.06 (C), 146.01 (C), 147.61 (C), 149.61 (C), 153.57 (C), 153.99 (C) (Figure 10).

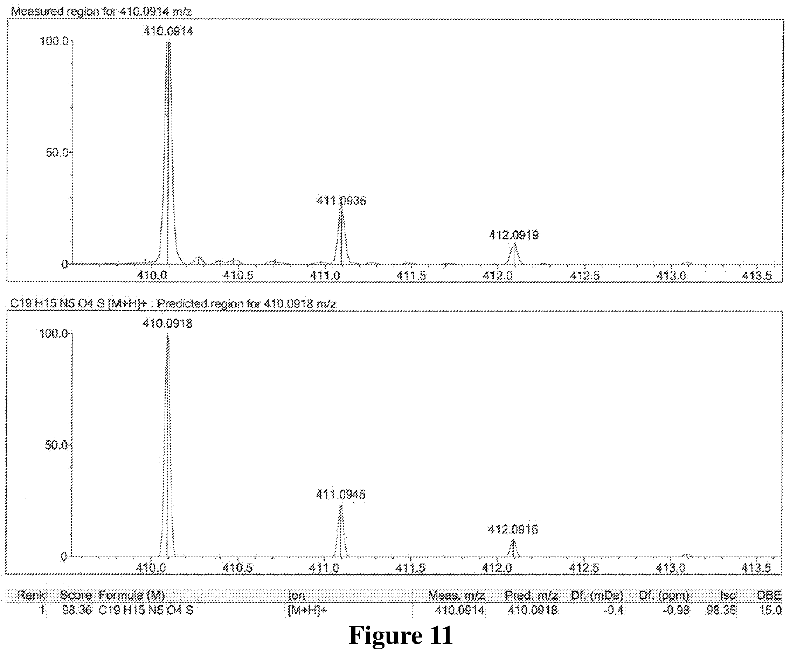

HRMS (ESI) (*m*/*z*): [M+H]⁺ calculated for C₁₉H₁₅N₅O₄S: 410.0918, found: 410.0914 (Figure 11).

### 3-[2-(1,3-Benzodioxole-5-yl)ethyl]-6-(4-cyanophenyl)-7H-[1,2,4]triazolo[3,4-b] [1,3,4]thiadiazine (Compound 3)

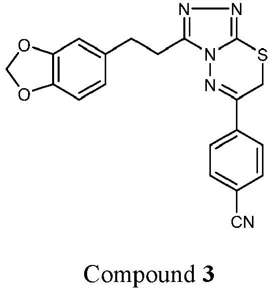

It was synthesized according to Method B using 4-amino-5-[2-(1,3-benzodioxole-5-yl)ethyl]-2,4-dihydro-3H-1,2,4-triazole-3-thione (0.001 mol; 0.3000 g) and 2-bromo-4'-cyanoacetophenone (0.001 mol; 0.2543 g).

### Yield: 87%, M.p.: 204.7 °C

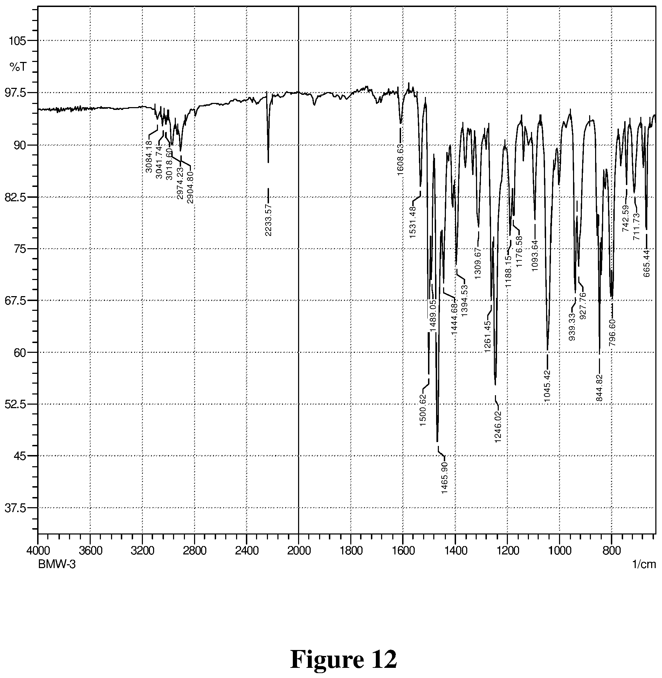

IR νₘₐₓ (cm⁻¹): 3084.18, 3041.74, 3018.60 (Aromatic C-H stretching bands), 2974.23, 2904.80, (Aliphatic C-H stretching bands), 2233.57 (C=N stretching band), 1608.63, 1531.48, 1500.62, 1489.05, 1444.68 (C=C and C=N stretching bands), 1394.53, 1309.67, 1261.45, 1246.02, 1188.15, 1176.58, 1093.64, 1045.42 (C-H bending, C-N, C-O stretching and aromatic C-H in plane bending bands), 939.33, 927.76, 844.82, 796.60, 742.59, 711.73, 665.44 (Aromatic C-H out of plane bending bands and C-S stretching band) (Figure 12).

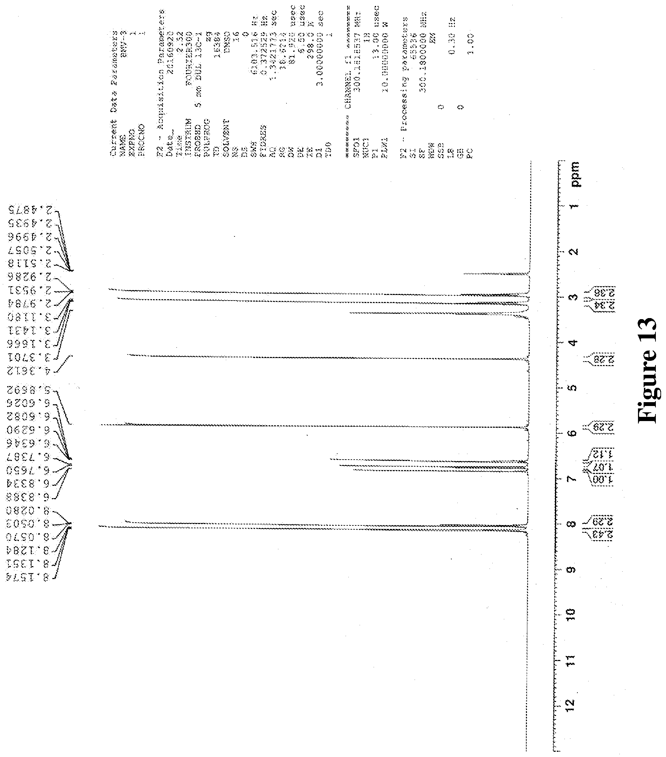

¹H NMR (300 MHz, DMSO-*d₆*): 2.95 (t, *J*= 7.35 Hz, *J*= 7.59 Hz, 2H), 3.14 (t, *J*= 7.05 Hz, 7.53 Hz, 2H), 4.36 (s, 2H), 5.87 (s, 2H), 6.62 (d, *J*= 7.92 Hz, 1H), 6.75 (d, *J*= 7.89 Hz, 1H), 6.83 (s, 1H), 8.04 (d, J= 8.70 Hz, 2H), 8.14 (d, *J*= 8.70 Hz, 2H) (Figure 13).

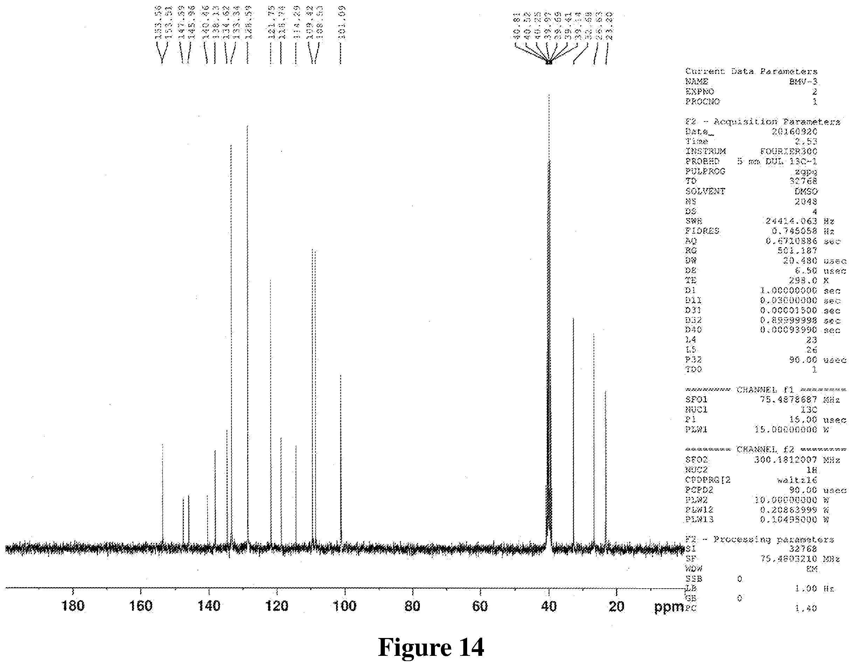

¹³C NMR (75 MHz, DMSO-*d₆*): 23.20 (CH₂), 26.63 (CH₂), 32.68 (CH₂), 101.09 (CH₂), 108.53 (CH), 109.42 (CH), 114.29 (C), 118.74 (C), 121.75 (CH), 128.59 (2CH), 133.34 (2CH), 134.62 (C), 138.13 (C), 140.46 (C), 145.96 (C), 147.59 (C), 153.51 (C), 153.56 (C) (Figure 14).

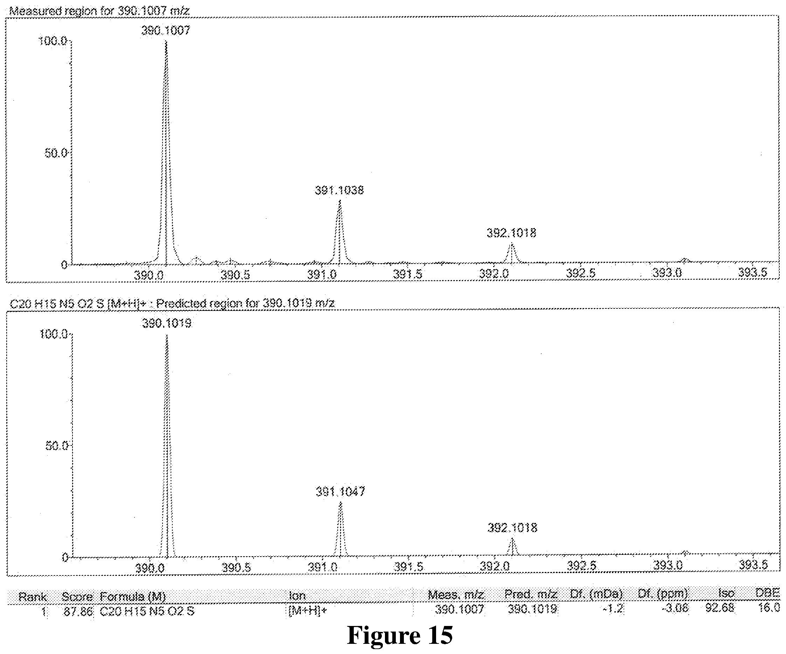

HRMS (ESI) (*m*/*z*): [M+H]⁺ calculated for C₂₀H₁₅N₅O₂S: 390.1019, found: 390.1007 (Figure 15).

### 3-[2-(1,3-Benzodioxole-5-yl)ethyl]-6-(4-fluorophenyl)-7H-[1,2,4]triazolo[3,4-b] [1,3,4]thiadiazine (Compound 4)

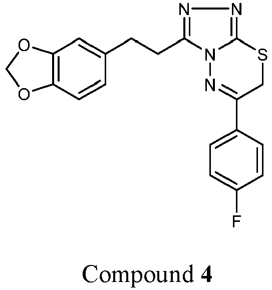

It was synthesized according to Method B using 4-amino-5-[2-(1,3-benzodioxole-5-yl)ethyl]-2,4-dihydro-3*H*-1,2,4-triazole-3-thione (0.001 mol; 0.3000 g) and 2-bromo-4'-fluoroacetophenone (0.001 mol; 0.2464 g).

### Yield: 75%, M.p.: 191.2 °C

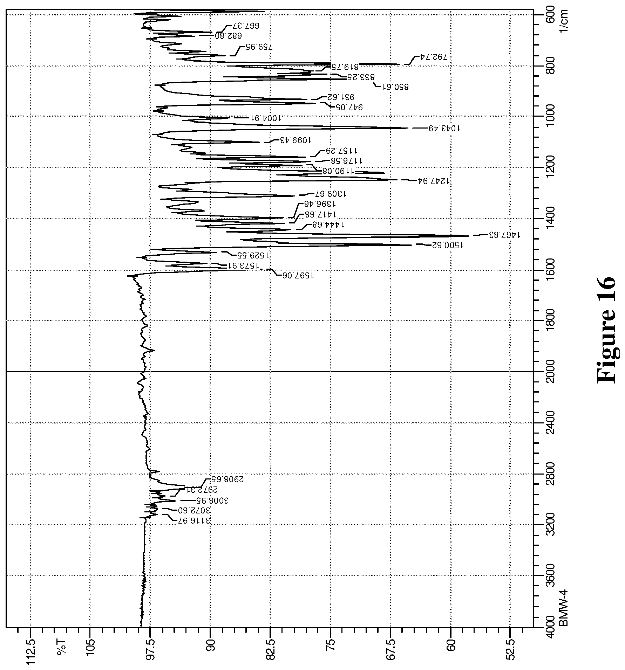

IR νₘₐₓ (cm⁻¹): 3116.97, 3072.60, 3008.95 (Aromatic C-H stretching bands), 2972.31, 2908.65 (Aliphatic C-H stretching bands), 1597.06, 1573.91, 1529.55, 1500.62, 1467.83, 1444.68 (C=C and C=N stretching bands), 1417.68, 1396.46, 1309.67, 1247.94, 1190.08, 1176.58, 1157.29, 1099.43, 1043.49, 1004.91 (C-H bending, C-N, C-O stretching and aromatic C-H in plane bending bands), 947.05, 931.62, 850.61, 833.25, 819.75, 792.74, 759.95, 682.80, 667.37 (Aromatic C-H out of plane bending bands and C-S stretching band) (Figure 16).

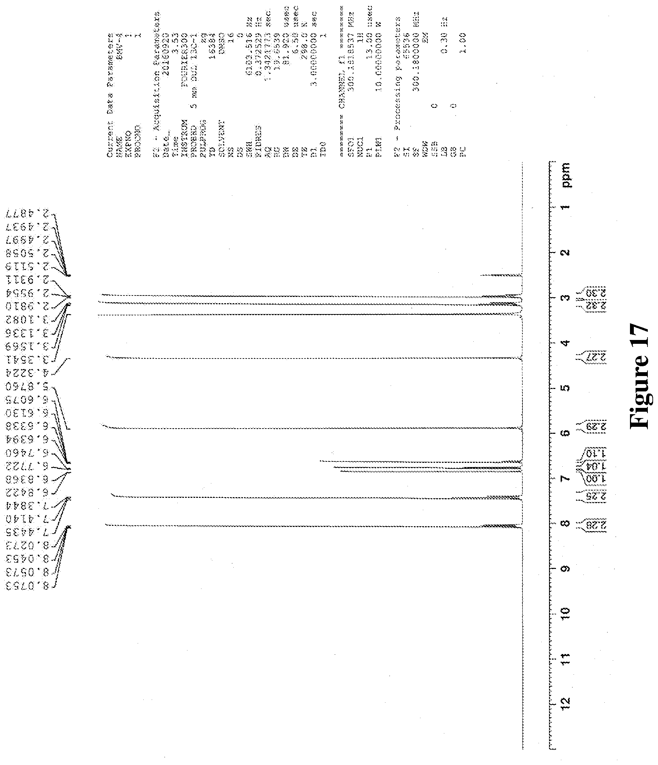

¹H NMR (300 MHz, DMSO-*d₆*): 2.96 (t, *J*= 7.29 Hz, *J*= 7.68 Hz, 2H), 3.13 (t, *J*= 6.99 Hz, 7.62 Hz, 2H), 4.32 (s, 2H), 5.88 (s, 2H), 6.62 (d, *J*= 7.89 Hz, 1H), 6.76 (d, *J*= 7.86 Hz, 1H), 6.84 (s, 1H), 7.38-7.44 (m, 2H), 8.03-8.08 (m, 2H) (Figure 17).

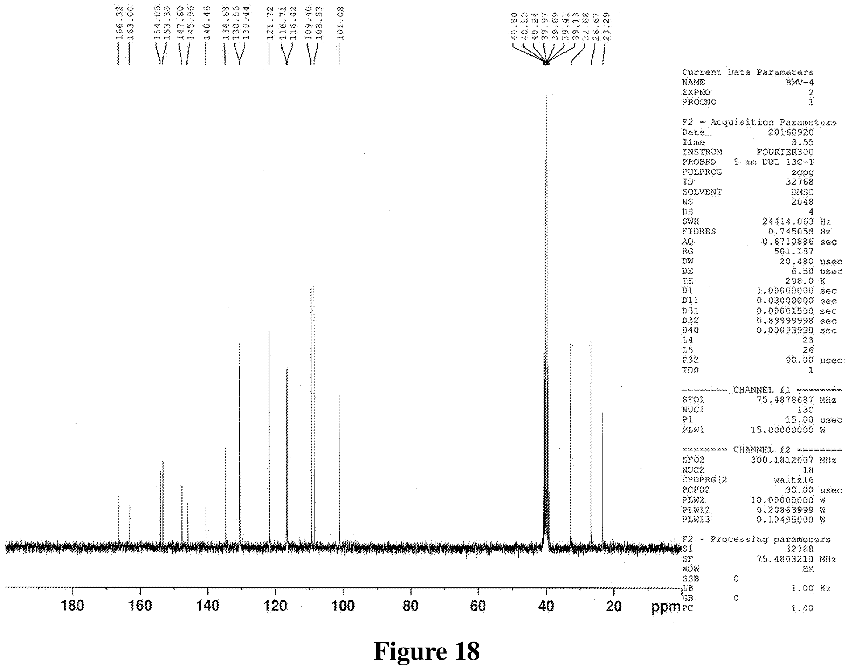

¹³C NMR (75 MHz, DMSO-*d₆*): 23.29 (CH₂), 26.67 (CH₂), 32.68 (CH₂), 101.08 (CH₂), 108.53 (CH), 109.40 (CH), 116.57 (d, *J*= 21.75 Hz, 2CH), 121.72 (CH), 130.50 (d, *J*= 9.00 Hz, 2CH), 134.68 (C), 140.46 (C), 145.96 (C), 147.60 (C), 153.30 (C), 154.06 (C), 163.00 (C), 166.32 (C) (Figure 18).

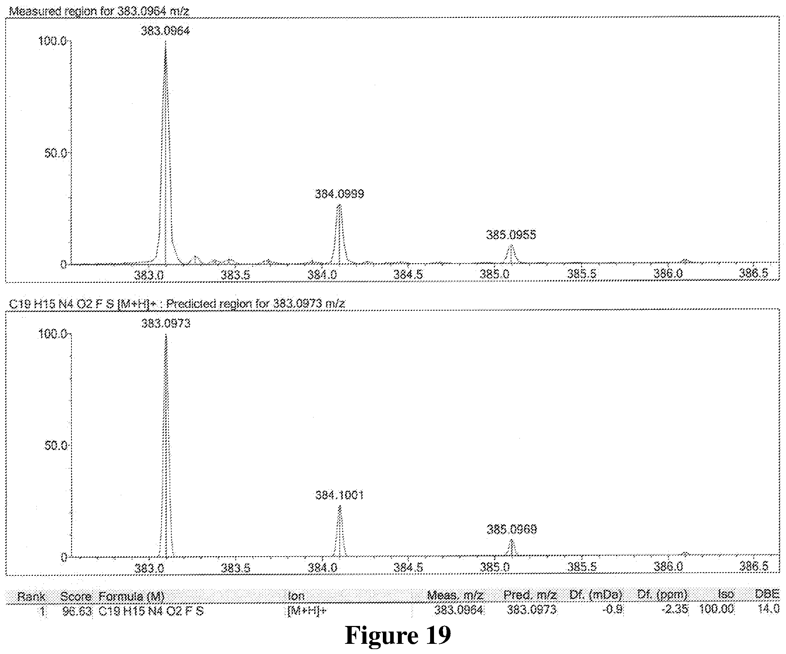

HRMS (ESI) (*m*/*z*): [M+H]⁺ calculated for C₁₉H₁₅N₄O₂FS: 383.0973, found: 383.0964 (Figure 19).

### 3-[2-(1,3-Benzodioxole-5-yl)ethyl]-6-(4-chlorophenyl)-7H-[1,2,4]triazolo[3,4-b] [1,3,4]thiadiazine (Compound 5)

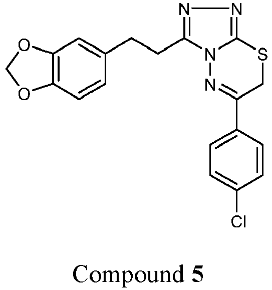

It was synthesized according to Method B using 4-amino-5-[2-(1,3-benzodioxole-5-yl)ethyl]-2,4-dihydro-3*H*-1,2,4-triazole-3-thione (0.001 mol; 0.3000 g) and 2-bromo-4'-chloroacetophenone (0.001 mol; 0.2650 g).

### Yield: 89%, M.p.: 200.2 °C

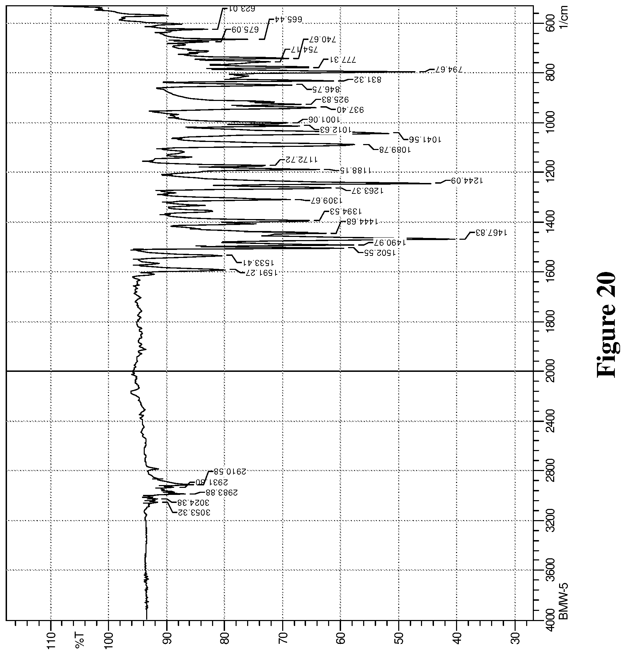

IR νₘₐₓ (cm⁻¹): 3053.32, 3024.38 (Aromatic C-H stretching bands), 2983.88, 2931.30, 2910.58 (Aliphatic C-H stretching bands), 1591.27, 1533.41, 1502.55, 1490.97, 1467.83, 1444.68 (C=C and C=N stretching bands), 1394.53, 1309.67, 1263.37, 1244.09, 1188.15, 1172.72, 1089.78, 1041.56, 1012.63, 1001.06 (C-H bending, C-N, C-O stretching and aromatic C-H in plane bending bands), 937.40, 925.83, 846.75, 831.32, 794.67, 777.31, 754.17, 740.67, 675.09, 665.44, 623.01 (Aromatic C-H out of plane bending bands and C-S stretching band) (Figure 20).

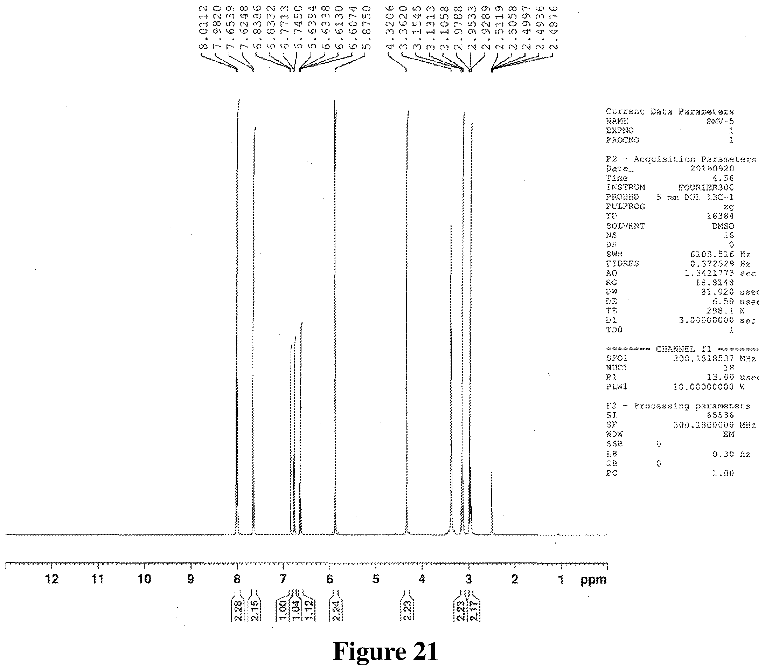

¹H NMR (300 MHz, DMSO-*d₆*): 2.95 (t, *J*= 7.32 Hz, 7.65 Hz, 2H), 3.13 (t, *J*= 6.96 Hz, 7.65 Hz, 2H), 4.32 (s, 2H), 5.88 (s, 2H), 6.62 (d, *J*= 7.92 Hz, 1H), 6.76 (d, *J*= 7.89 Hz, 1H), 6.83 (s, 1H), 7.64 (d, *J*= 8.73 Hz, 2H), 8.00 (d, *J*= 8.76 Hz, 2H) (Figure 21).

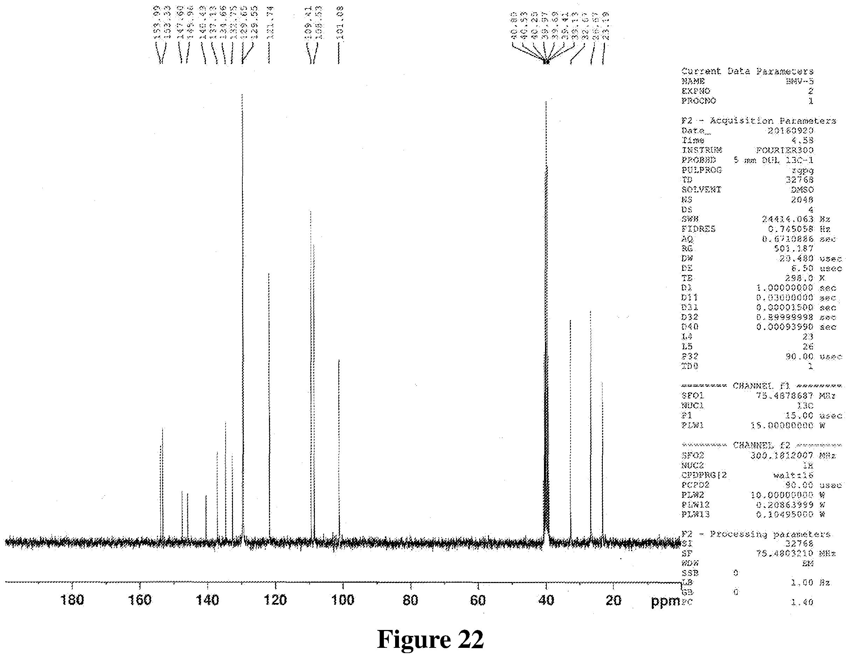

¹³C NMR (75 MHz, DMSO-*d₆*): 23.19 (CH₂), 26.67 (CH₂), 32.67 (CH₂), 101.08 (CH₂), 108.53 (CH), 109.41 (CH), 121.74 (CH), 129.55 (2CH), 129.65 (2CH), 132.75 (C), 134.66 (C), 137.13 (C), 140.49 (C), 145.96 (C), 147.60 (C), 153.33 (C), 153.99 (C) (Figure 22).

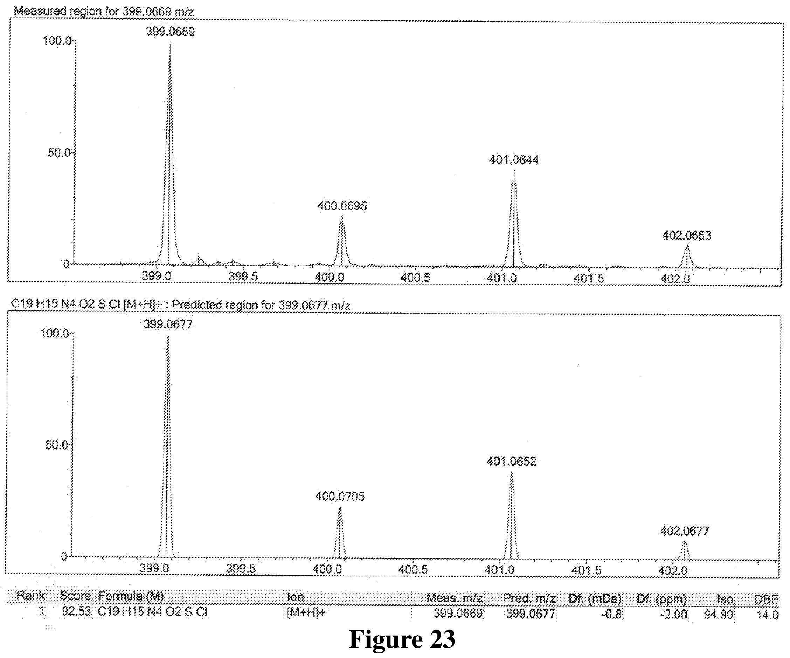

HRMS (ESI) (*m*/*z*): [M+H]⁺ calculated for C₁₉H₁₅N₄O₂SCl: 399.0677, found: 399.0669 (Figure 23).

### 3-[2-(1,3-Benzodioxole-5-yl)ethyl]-6-(4-bromophenyl)-7H-[1,2,4]triazolo[3,4-b] [1,3,4]thiadiazine (Compound 6)

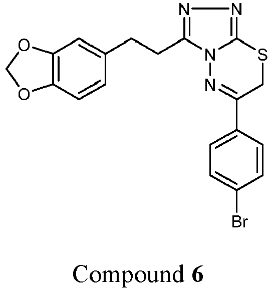

It was synthesized according to Method B using 4-amino-5-[2-(1,3-benzodioxole-5-yl)ethyl]-2,4-dihydro-3*H*-1,2,4-triazole-3-thione (0.001 mol; 0.3000 g) and 2,4'-dibromoacetophenone (0.001 mol; 0.3155 g).

### Yield: 85%, M.p.: 209.5 °C

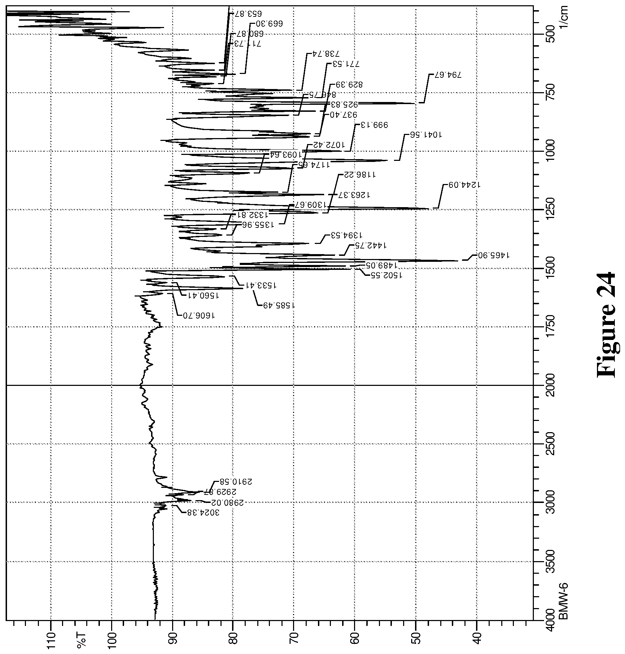

IR νₘₐₓ (cm⁻¹): 3024.38 (Aromatic C-H stretching band), 2980.02, 2929.87, 2910.58 (Aliphatic C-H stretching bands), 1606.70, 1585.49, 1560.41, 1533.41, 1502.55, 1489.05, 1465.90, 1442.75 (C=C and C=N stretching bands), 1394.53, 1355.96, 1332.81, 1309.67, 1263.37, 1244.09, 1186.22, 1174.65, 1093.64, 1072.42, 1041.56 (C-H bending, C-N, C-O stretching and aromatic C-H in plane bending bands), 999.13, 937.40, 925.83, 846.75, 829.39, 794.67, 771.53, 738.74, 711.73, 680.87, 669.30, 653.87 (Aromatic C-H out of plane bending bands and C-S stretching band) (Figure 24).

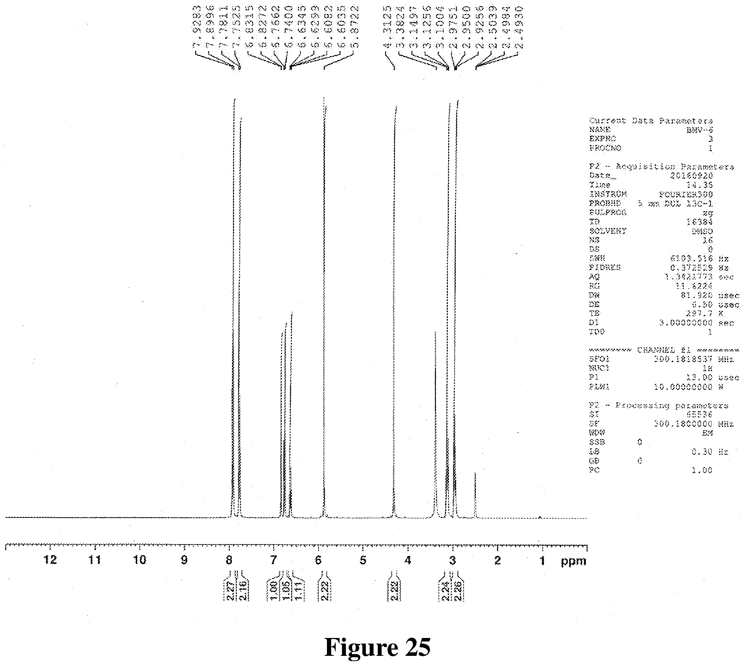

¹H NMR (300 MHz, DMSO-*d₆*): 2.95 (t, *J*= 7.32 Hz, 7.53 Hz, 2H), 3.13 (t, *J*= 7.23 Hz, 7.56 Hz, 2H), 4.31 (s, 2H), 5.87 (s, 2H), 6.62 (d, *J*= 7.92 Hz, 1H), 6.75 (d, *J*= 7.86 Hz, 1H), 6.83 (s, 1H), 7.77 (d, *J*= 8.58 Hz, 2H), 7.91 (d, *J*= 8.61 Hz, 2H) (Figure 25).

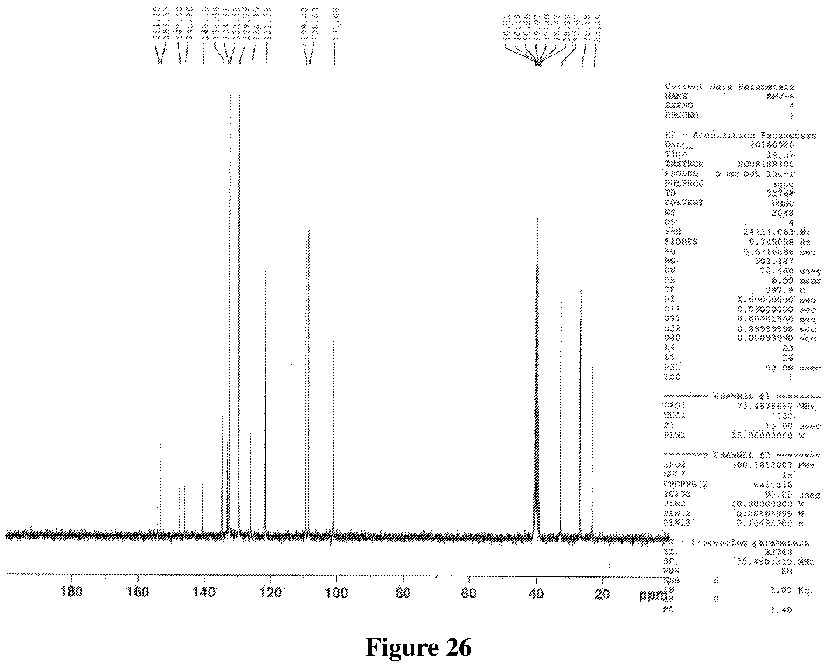

¹³C NMR (75 MHz, DMSO-*d₆*): 23.16 (CH₂), 26.68 (CH₂), 32.67 (CH₂), 101.08 (CH₂), 108.53 (CH), 109.40 (CH), 121.73 (CH), 126.10 (C), 129.79 (2CH), 132.48 (2CH), 133.11 (C), 134.66 (C), 140.49 (C), 145.96 (C), 147.60 (C), 153.33 (C), 154.10 (C) (Figure 26).

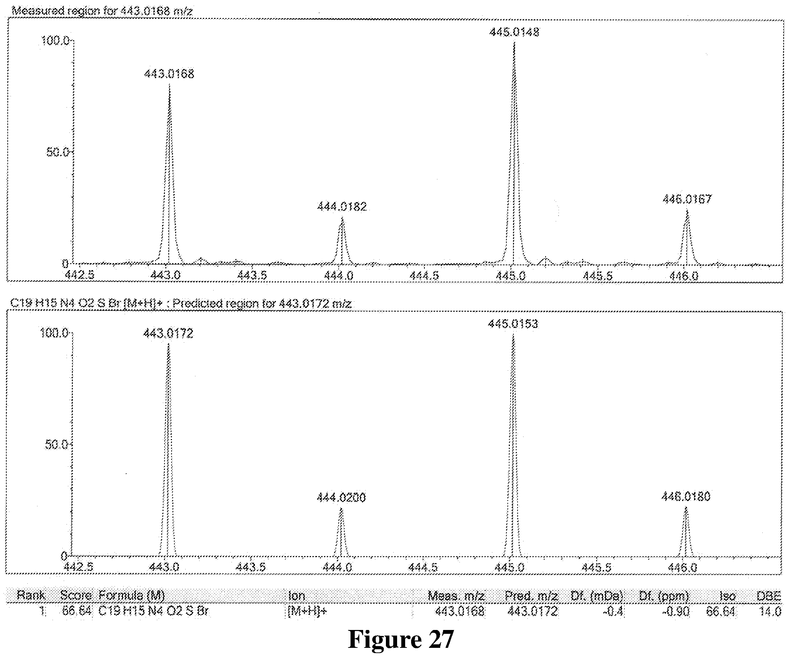

HRMS (ESI) (*m*/*z*): [M+H]⁺ calculated for C₁₉H₁₅N₄O₂SBr: 443.0172, found: 443.0168 (Figure 27).

### 3-[2-(1,3-Benzodioxole-5-yl)ethyl]-6-(4-methoxyphenyl)-7H-[1,2,4]triazolo[3,4-b] [1,3,4]thiadiazine (Compound 7)

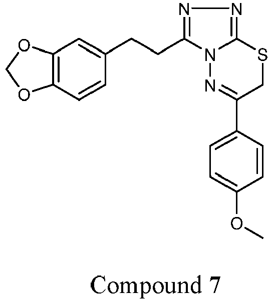

It was synthesized according to Method B using 4-amino-5-[2-(1,3-benzodioxole-5-yl)ethyl]-2,4-dihydro-3*H*-1,2,4-triazole-3-thione (0.001 mol; 0.3000 g) and 2-bromo-4'-methoxyacetophenone (0.001 mol; 0.2600 g).

### Yield: 81%, M.p.: 175.2 °C

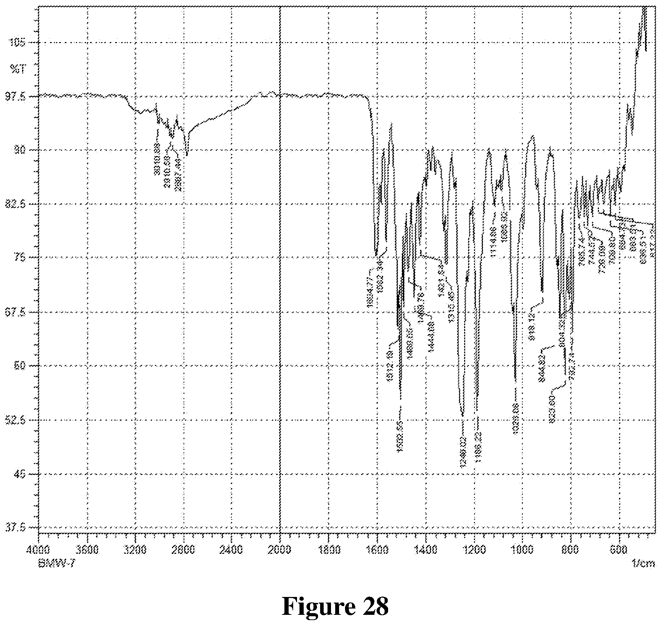

IR νₘₐₓ (cm⁻¹): 3010.88 (Aromatic C-H stretching band), 2910.58, 2887.44 (Aliphatic C-H stretching bands), 1604.77, 1562.34, 1512.19, 1502.55, 1489.05, 1469.76, 1444.68 (C=C and C=N stretching bands), 1421.54, 1315.45, 1246.02, 1186.22, 1114.86, 1085.92, 1028.06 (C-H bending, C-N, C-O stretching and aromatic C-H in plane bending bands), 918.12, 844.82, 823.60, 804.32, 792.74, 765.74, 744.52, 729.09, 709.80, 684.73, 663.51, 636.51, 617.22 (Aromatic C-H out of plane bending bands and C-S stretching band) (Figure 28).

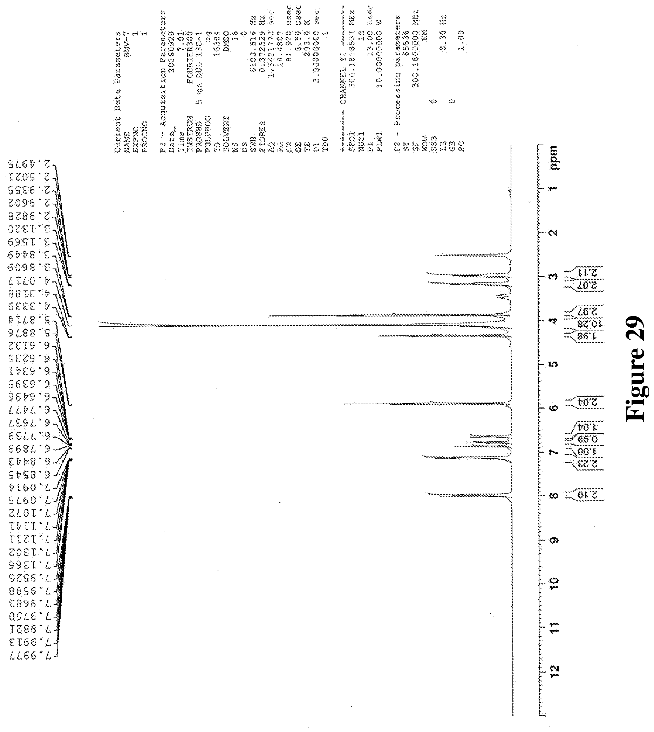

¹H NMR (300 MHz, DMSO-*d₆*): 2.94-2.98 (m, 2H), 3.13-3.16 (m, 2H), 3.84 (s, 3H), 4.32 (s, 2H), 5.87 (s, 2H), 6.61-6.65 (m, 1H), 6.75-6.79 (m, 1H), 6.84 (s, 1H), 7.09-7.14 (m, 2H), 7.95-8.00 (m, 2H) (Figure 29).

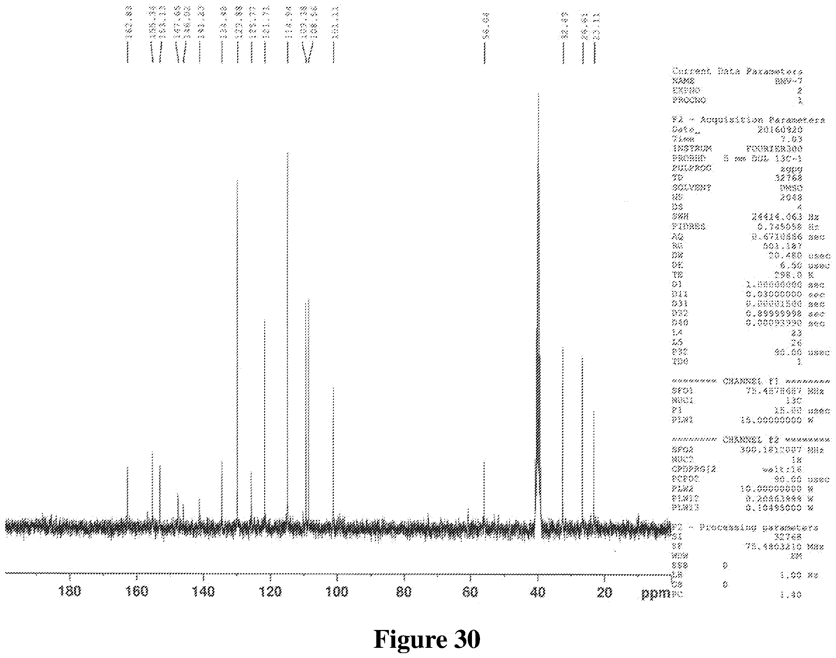

¹³C NMR (75 MHz, DMSO-*d₆*): 23.11 (CH₂), 26.61 (CH₂), 32.49 (CH₂), 56.04 (CH₃), 101.11 (CH₂), 108.56 (CH), 109.38 (CH), 114.94 (2CH), 121.71 (CH), 125.77 (C), 129.88 (2CH), 134.48 (C), 141.23 (C), 146.02 (C), 147.65 (C), 153.13 (C), 155.34 (C), 162.83 (C) (Figure 30).

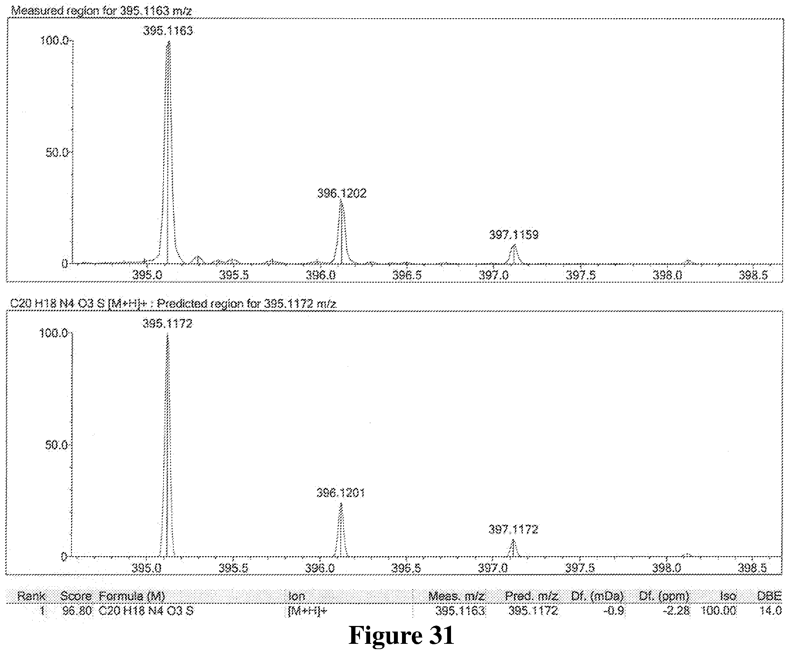

HRMS (ESI) (*m*/*z*): [M+H]⁺ calculated for C₂₀H₁₈N₄O₃S: 395.1172, found: 395.1163 (Figure 31).

### 3-[2-(1,3-Benzodioxole-5-yl)ethyl]-6-(4-methylphenyl)-7H- [1,2,4] triazolo [3,4-b] [1,3,4]thiadiazine (Compound 8)

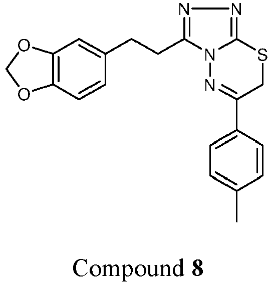

It was synthesized according to Method B using 4-amino-5-[2-(1,3-benzodioxole-5-yl)ethyl]-2,4-dihydro-3*H*-1,2,4-triazole-3-thione (0.001 mol; 0.3000 g) and 2-bromo-4'-methylacetophenone (0.001 mol; 0.2419 g).

### Yield: 79%, M.p.: 198.7 °C

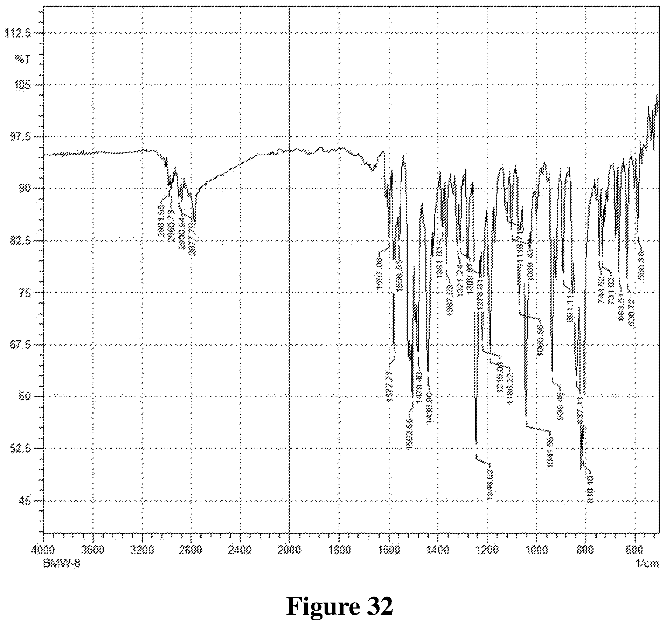

IR νₘₐₓ (cm⁻¹): 2981.95, 2960.73, 2900.94, 2877.79 (Aliphatic C-H stretching bands), 1597.06, 1577.77, 1556.55, 1502.55, 1479.40 (C=C and C=N stretching bands), 1438.90, 1381.03, 1367.53, 1321.24, 1309.67, 1278.81, 1246.02, 1219.01, 1186.22, 1118.71, 1099.43, 1068.56, 1041.56 (C-H bending, C-N, C-O stretching and aromatic C-H in plane bending bands), 935.48, 891.11, 837.11, 810.10, 744.52, 731.02, 663.51, 630.72, 586.36 (Aromatic C-H out of plane bending bands and C-S stretching band) (Figure 32).

¹H NMR (300 MHz, DMSO-*d₆*): 2.39 (s, 3H), 2.97 (t, *J*= 7.35 Hz, 7.53 Hz, 2H), 3.20 (t, *J*= 7.38 Hz, 7.47 Hz, 2H), 4.38 (s, 2H), 5.88 (s, 2H), 6.63 (d, *J*= 7.92 Hz, 1H), 6.77 (d, *J*= 7.86 Hz, 1H), 6.86 (s, 1H), 7.39 (d, *J*= 8.22 Hz, 2H), 7.91 (d, *J*= 8.16 Hz, 2H) (Figure 33).

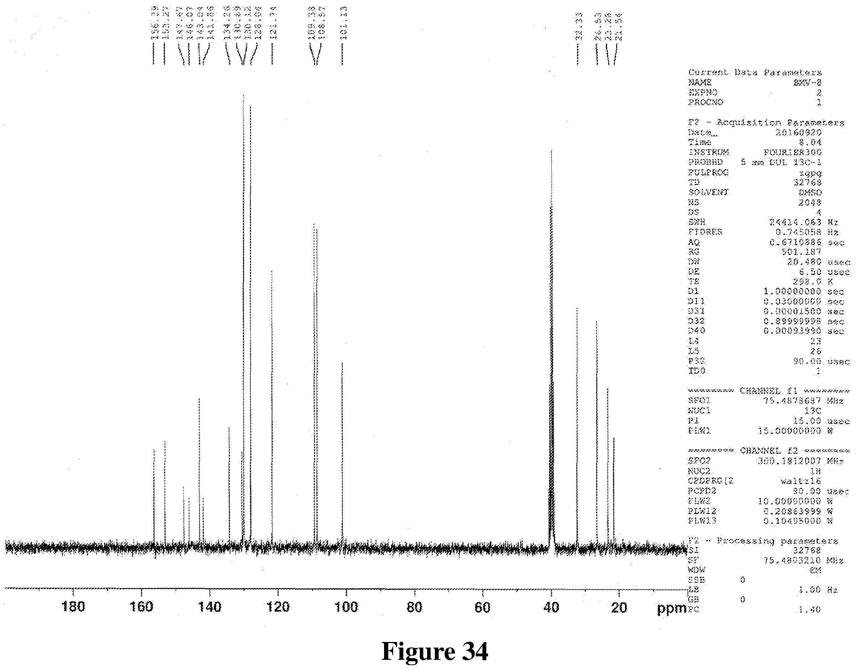

¹³C NMR (75 MHz, DMSO-*d₆*): 21.54 (CH₃), 23.28 (CH₂), 26.53 (CH₂), 32.33 (CH₂), 101.13 (CH₂), 108.57 (CH), 109.38 (CH), 121.74 (CH), 128.04 (2CH), 130.12 (2CH), 130.69 (C), 134.26 (C), 141.86 (C), 143.04 (C), 146.07 (C), 147.67 (C), 153.27 (C), 156.39 (C) (Figure 34).

HRMS (ESI) (*m*/*z*): [M+H]⁺ calculated for C₂₀H₁₈N₄O₂S: 379.1223, found: 379.1214 (Figure 35).

### 3-[2-(1,3-Benzodioxole-5-yl)ethyl]-6-[4-(methylsulfonyl)phenyl)]-7H-[1,2,4]triazolo[3,4-b] [1,3,4]thiadiazine (Compound 9)

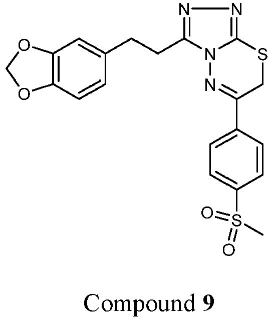

It was synthesized according to Method B using 4-amino-5-[2-(1,3-benzodioxole-5-yl)ethyl]-2,4-dihydro-3*H*-1,2,4-triazole-3-thione (0.001 mol; 0.3000 g) and 2-bromo-1-[4-(methylsulfonyl)phenyl]-1-ethanone (0.001 mol; 0.3146 g).

### Yield: 89%, M.p.: 201.3 °C

IR νₘₐₓ (cm⁻¹): 3007.02 (Aromatic C-H stretching band), 2927.94, 2885.51 (Aliphatic C-H stretching bands), 1606.70, 1531.48, 1504.48, 1489.05, 1465.90, 1442.75 (C=C and C=N stretching bands), 1392.61, 1307.74, 1288.45, 1265.30, 1244.09, 1192.01, 1149.57, 1091.71, 1037.70 (C-H bending, C-N, C-O stretching and aromatic C-H in plane bending bands), 960.55, 925.83, 869.90, 854.47, 819.75, 792.74, 773.46, 750.31, 713.66, 663.51 (Aromatic C-H out of plane bending bands and C-S stretching band) (Figure 36).

¹H NMR (300 MHz, DMSO-*d₆*): 2.96 (t, *J*= 7.32 Hz, 7.53 Hz, 2H), 3.15 (t, *J*= 7.38 Hz, 7.44 Hz, 2H), 3.43 (s, 3H), 4.39 (s, 2H), 5.86 (s, 2H), 6.62 (d, *J*= 7.92 Hz, 1H), 6.76 (d, *J*= 7.86 Hz, 1H), 6.84 (s, 1H), 8.10 (d, *J*= 8.58 Hz, 2H), 8.21 (d, J= 8.61 Hz, 2H) (Figure 37).

¹³C NMR (75 MHz, DMSO-*d₆*): 23.45 (CH₂), 26.67 (CH₂), 32.72 (CH₂), 43.75 (CH₃), 101.09 (CH₂), 108.54 (CH), 109.43 (CH), 121.76 (CH), 127.99 (2CH), 128.79 (2CH), 134.62 (C), 138.61 (C), 140.47 (C), 143.56 (C), 145.96 (C), 147.60 (C), 153.50 (C), 153.75 (C) (Figure 38).

HRMS (ESI) (*m*/*z*): [M+H]⁺ calculated for C₂₀H₁₈N₄O₄S₂: 443.0842, found: 443.0825 (Figure 39).

### 3- [2-(1,3-Benzodioxole-5-yl)ethyl]-6-(naphthalene-2-yl)-7H- [1,2,4]triazolo [3,4-b][1,3,4]thiadiazine (Compound 10)

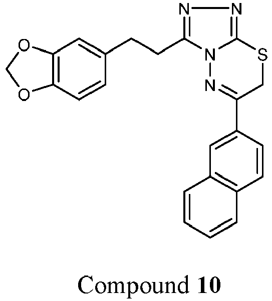

It was synthesized according to Method B using 4-amino-5-[2-(1,3-benzodioxole-5-yl)ethyl]-2,4-dihydro-3*H*-1,2,4-triazole-3-thione (0.001 mol; 0.3000 g) and 2-bromo-2'-acetonaphthone (0.001 mol; 0.2827 g).

### Yield: 83%, M.p.: 170.6 °C

IR νₘₐₓ (cm⁻¹): 3045.60 (Aromatic C-H stretching band), 2991.59, 2910.58, 2883.58 (Aliphatic C-H stretching bands), 1629.85, 1597.06, 1573.91,1529.55, 1492.90, 1462.04, 1444.68 (C=C and C=N stretching bands), 1411.89, 1390.68, 1367.53, 1307.74, 1244.09, 1192.01, 1176.78, 1095.57, 1041.56 (C-H bending, C-N, C-O stretching and aromatic C-H in plane bending bands), 927.76, 908.47, 866.04, 854.47, 812.03, 796.60, 758.02, 742.59, 717.52, 667.37 (Aromatic C-H out of plane bending bands and C-S stretching band) (Figure 40).

¹H NMR (300 MHz, DMSO-*d₆*): 2.99 (t, *J*= 7.35 Hz, 7.59 Hz, 2H), 3.19 (t, *J*= 7.17 Hz, 7.59 Hz, 2H), 4.48 (s, 2H), 5.83 (s, 2H), 6.65 (d, *J*= 7.92 Hz, 1H), 6.76 (d, *J*= 7.86 Hz, 1H), 6.87 (s, 1H), 7.63-7.69 (m, 2H), 8.00-8.14 (m, 4H), 8.59 (s, 1H) (Figure 41).

¹³C NMR (75 MHz, DMSO-*d₆*): 23.18 (CH₂), 26.73 (CH₂), 32.72 (CH₂), 101.05 (CH₂), 108.54 (CH), 109.43 (CH), 121.75 (CH), 123.63 (2CH), 127.63 (CH), 128.21 (CH), 128.75 (C), 129.16 (CH), 129.47 (2CH), 131.13 (C), 132.85 (C), 134.66 (C), 140.80 (C), 145.99 (C), 147.63 (C), 153.39 (C), 154.97 (C) (Figure 42).

HRMS (ESI) (*m*/*z*): [M+H]⁺ calculated for C₂₃H₁₈N₄O₂S: 415.1223, found: 415.1219 (Figure 43).

### 3- [2-(1,3-Benzodioxole-5-yl)ethyl]-6-(4-trifluoromethylphenyl)-7H- [1,2,4]triazolo [3,4-b][1,3,4]thiadiazine (Compound 11)

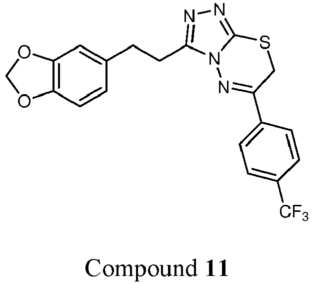

It was synthesized according to Method B using 4-amino-5-[2-(1,3-benzodioxole-5-yl)ethyl]-2,4-dihydro-3*H*-1,2,4-triazole-3-thione (0.001 mol; 0.3000 g) and 2-bromo-4'-trifluoromethylacetophenone (0.001 mol; 0.3031 g).

### Yield: 76%, M.p.: 214 °C

IR νₘₐₓ (cm⁻¹): 3049.46 (Aromatic C-H stretching band), 2966.52, 2908.65 (Aliphatic C-H stretching bands), 1608.63, 1533.41, 1504.48, 1490.97,1465.90, 1444.68 (C=C and C=N stretching bands), 1413.82, 1396.46, 1325.10, 1307.74, 1263.37, 1244.09, 1188.15, 1178.51, 1161.15, 1114.86, 1095.57, 1070.49, 1041.56, 1014.56 (C-H bending, C-N, C-O stretching and aromatic C-H in plane bending bands), 925.83, 848.68, 837.11, 796.60, 748.38, 732.95, 680.87 (Aromatic C-H out of plane bending bands and C-S stretching band) (Figure 44).

¹H NMR (300 MHz, DMSO-*d₆*): 2.96 (t, *J*= 7.29 Hz, 7.35 Hz, 2H), 3.15 (t, *J*= 7.17 Hz, 7.59 Hz, 2H), 4.39 (s, 2H), 5.87 (s, 2H), 6.63 (d, *J*= 7.92 Hz, 1H), 6.77 (d, *J*= 7.86 Hz, 1H), 6.85 (s, 1H), 7.95 (d, *J*= 8.37 Hz, 2H), 8.18 (d, *J*= 8.22 Hz, 2H) (Figure 45).

¹³C NMR (75 MHz, DMSO-*d₆*): 23.36 (CH₂), 26.69 (CH₂), 32.68 (CH₂), 101.07 (CH₂), 108.55 (CH), 109.44 (CH), 121.77 (CH), 126.35 (C), 128.71 (4CH), 134.64 (C), 137.87 (C), 140.49 (C), 145.96 (C), 147.59 (C), 153.46 (2C), 153.83 (C) (Figure 46).

HRMS (ESI) (*m*/*z*): [M+H]⁺ calculated for C₂₀H₁₅N₄O₂F₃S: 443.0941, found: 443.0926 (Figure 47).

### 3-[2-(1,3-Benzodioxole-5-yl)ethyl]-6-(3-fluorophenyl)-7H-[1,2,4]triazolo [3,4-b] [1,3,4]thiadiazine (Compound 12)

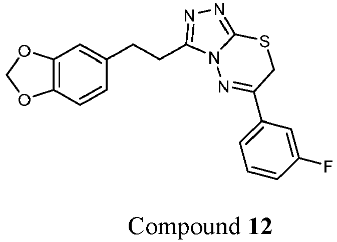

It was synthesized according to Method B using 4-amino-5-[2-(1,3-benzodioxole-5-yl)ethyl]-2,4-dihydro-3*H*-1,2,4-triazole-3-thione (0.001 mol; 0.3000 g) and 2-bromo-3'-fluoroacetophenone (0.001 mol; 0.2464 g).

### Yield: 74%, M.p.: 164 °C

IR νₘₐₓ (cm⁻¹): 3068.75, 3035.96, 3008.95 (Aromatic C-H stretching bands), 2933.73, 2889.37 (Aliphatic C-H stretching bands), 1608.63, 1575.84, 1531.48, 1502.55, 1487.12, 1460.11, 1442.75 (C=C and C=N stretching bands), 1390.68, 1344.38, 1327.03, 1307.74, 1269.16, 1253.73, 1240.23, 1184.29, 1151.50, 1122.57, 1095.57, 1033.85 (C-H bending, C-N, C-O stretching and aromatic C-H in plane bending bands), 931.62, 906.54, 889.18, 862.18, 819.75, 802.39, 758.02, 738.74, 725.23, 690.52 (Aromatic C-H out of plane bending bands and C-S stretching band) (Figure 48).

¹H NMR (300 MHz, DMSO-*d₆*): 2.95 (t, *J*= 7.35 Hz, *J*= 7.53 Hz, 2H), 3.15 (t, *J*= 7.17 Hz, 7.50 Hz, 2H), 4.33 (s, 2H), 5.86 (s, 2H), 6.61 (d, *J*= 7.91 Hz, 1H), 6.75 (d, *J*= 7.89 Hz, 1H), 6.85 (s, 1H), 7.44-7.51 (m, 1H), 7.58-7.66 (m, 1H), 7.79-7.84 (m, 2H) (Figure 49).

¹³C NMR (75 MHz, DMSO-*d₆*): 23.27 (CH₂), 26.62 (CH₂), 32.76 (CH₂), 101.08 (CH₂), 108.51 (CH), 109.43 (CH), 114.58 (d, *J*= 23.47 Hz, CH), 119.16 (d, *J*= 21.14 Hz, CH), 121.73 (CH), 124.10 (CH), 131.61 (d, *J*= 8.18 Hz, CH), 134.66 (C), 136.24 (d, *J*= 7.70 Hz, C), 140.52 (C), 145.95 (C), 147.60 (C), 153.42 (C), 161.13 (C), 164.37 (C) (Figure 50).

HRMS (ESI) (*m*/*z*): [M+H]⁺ calculated for C₁₉H₁₅N₄O₂FS: 383.0973, found: 383.0955 (Figure 51).

### 3-[2-(1,3-Benzodioxole-5-yl)ethyl]-6-(3-chlorophenyl)-7H-[1,2,4]triazolo[3,4-b][1,3,4]thiadiazine (Compound 13)

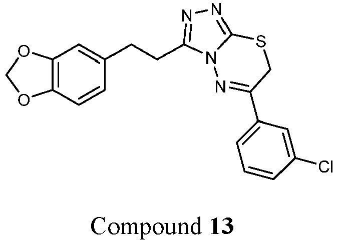

It was synthesized according to Method B using 4-amino-5-[2-(1,3-benzodioxole-5-yl)ethyl]-2,4-dihydro-3*H*-1,2,4-triazole-3-thione (0.001 mol; 0.3000 g) and 2-bromo-3'-chloroacetophenone (0.001 mol; 0.2650 g).

### Yield: 84%, M.p.: 190.3 °C

IR νₘₐₓ (cm⁻¹): 3078.39, 3022.45 (Aromatic C-H stretching bands), 2995.45, 2966.52, 2912.51, 2785.21 (Aliphatic C-H stretching bands), 1562.34, 1531.48, 1500.62, 1490.97, 1463.97, 1444.68 (C=C and C=N stretching bands), 1427.32, 1417.68, 1394.53, 1357.89, 1330.88, 1305.81, 1265.30, 1244.09, 1188.15, 1165.00, 1139.93, 1095.57, 1082.07, 1041.56, 1014.56 (C-H bending, C-N, C-O stretching and aromatic C-H in plane bending bands), 995.27, 941.26, 923.90, 881.47, 844.82, 802.39, 786.96, 740.67, 682.80, 624.94 (Aromatic C-H out of plane bending bands and C-S stretching band) (Figure 52).

¹H NMR (300 MHz, DMSO-*d₆*): 2.95 (t, *J*= 7.50 Hz, *J*= 7.38 Hz, 2H), 3.14 (t, *J*= 7.17 Hz, 7.47 Hz, 2H), 4.33 (s, 2H), 5.86 (s, 2H), 6.62 (d, *J*= 7.91 Hz, 1H), 6.76 (d, *J*= 7.89 Hz, 1H), 6.85 (s, 1H), 7.60 (t, *J*= 7.86 Hz, *J*= 7.95 Hz, 1H), 7.67-7.71 (m, 1H), 7.94 (d, *J*= 7.89 Hz, 1H), 8.01-8.02 (m, 1H) (Figure 53).

¹³C NMR (75 MHz, DMSO-*d₆*): 23.27 (CH₂), 26.67 (CH₂), 32.75 (CH₂), 101.08 (CH₂), 108.52 (CH), 109.42 (CH), 121.72 (CH), 126.55 (CH), 127.53 (CH), 131.37 (CH), 132.00 (CH), 134.32 (C), 134.68 (C), 136.00 (C), 140.53 (C), 145.95 (C), 147.61 (C), 153.42 (C), 153.80 (C) (Figure 54).

HRMS (ESI) (*m*/*z*): [M+H]⁺ calculated for C₁₉H₁₅N₄O₂SCl: 399.0677, found: 399.0687 (Figure 55).

### 3-[2-(1,3-Benzodioxole-5-yl)ethyl]-6-(biphenyl-4-yl)-7H-[1,2,4]triazolo[3,4-b] [1,3,4]thiadiazine (Compound 14)

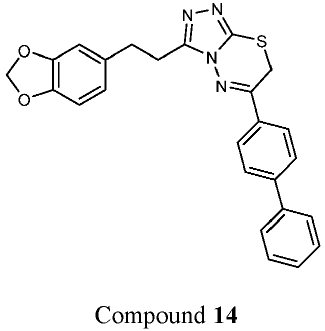

It was synthesized according to Method B using 4-amino-5-[2-(1,3-benzodioxole-5-yl)ethyl]-2,4-dihydro-3*H*-1,2,4-triazole-3-thione (0.001 mol; 0.3000 g) and 2-bromo-4'-phenylacetophenone (0.001 mol; 0.3123 g).

### Yield: 78%, M.p.: 246 °C

IR νₘₐₓ (cm⁻¹): 3055.24, 3032.10, 3003.17 (Aromatic C-H stretching bands), 2966.52, 2897.08, 2872.01, 2812.21 (Aliphatic C-H stretching bands), 1593.20, 1581.63, 1519, 91, 1498.69, 1487.12, 1444.68 (C=C and C=N stretching bands), 1408.04, 1359.82, 1323.17, 1305.81, 1284.59, 1267.23, 1244.09, 1209.37, 1188.15, 1095.57, 1082.07, 1039.63, 1006.84 (C-H bending, C-N, C-O stretching and aromatic C-H in plane bending bands), 925.83, 848.68, 825.53, 792.74, 769.60, 748.38, 738.74, 715.59, 700.16, 657.73 (Aromatic C-H out of plane bending bands and C-S stretching band) (Figure 56).

¹H NMR (300 MHz, DMSO-*d₆*): 2.99 (t, *J*= 7.59 Hz, *J*= 7.32 Hz, 2H), 3.19 (t, *J*= 7.29 Hz, 7.56 Hz, 2H), 4.42 (s, 2H), 5.88 (s, 2H), 6.65 (d, *J*= 7.92 Hz, 1H), 6.78 (d, *J*= 7.89 Hz, 1H), 6.88 (s, 1H), 7.45 (d, *J=* 7.17 Hz, 1H), 7.52 (t, *J*= 7.74 Hz, *J*= 6.93 Hz, 2H), 7.78 (d, *J*= 7.14 Hz, 2H), 7.89 (d, *J*= 8.52 Hz, 2H), 8.09 (d, *J*= 8.55 Hz, 2H) (Figure 57).

¹³C NMR (75 MHz, DMSO-*d₆*): 23.26 (CH₂), 26.65 (CH₂), 32.53 (CH₂), 101.11 (CH₂), 108.58 (CH), 109.42 (CH), 121.75 (CH), 127.37 (2CH), 127.62 (2CH), 128.63 (2CH), 128.86 (CH), 129.60 (2CH), 132.58 (C), 134.48 (C), 139.21 (C), 141.23 (C), 143.87 (C), 146.03 (C), 147.65 (C), 153.32 (C), 155.37 (C) (Figure 58).

HRMS (ESI) (*m*/*z*): [M+H]⁺ calculated for: C₂₅H₂₀N₄O₂S 441.1380, found: 441.1362 (Figure 59).

### 3- [2-(1,3-Benzodioxole-5-yl)ethyl] -6-(4-(thiophen-2-yl)phenyl)-7H- [1,2,4] triazolo [3,4-b] [1,3,4]thiadiazine (Compound 15)

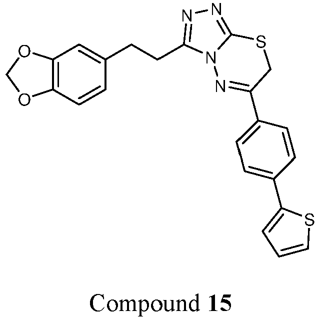

It was synthesized according to Method B using 4-amino-5-[2-(1,3-benzodioxole-5-yl)ethyl]-2,4-dihydro-3*H*-1,2,4-triazole-3-thione (0.001 mol; 0.3000 g) and 2-bromo-1-[4-(thiophen-2-yl)phenyl]-1-ethanone (0.001 mol; 0.3191 g).

### Yield: 76%, M.p.: 186 °C

IR νₘₐₓ (cm⁻¹): 3078.39 (Aromatic C-H stretching band), 2916.37, 2848.86 (Aliphatic C-H stretching bands), 1606.70, 1589.34, 1556.55, 1531.48, 1498.69, 1485.19, 1462.04, 1442.75 (C=C and C=N stretching bands), 1423.47, 1390.68, 1359.82, 1336.67, 1311.59, 1240.23, 1220.94, 1186.22, 1168.86, 1120.64, 1095.57, 1039.63, 1001.06 (C-H bending, C-N, C-O stretching and aromatic C-H in plane bending bands), 958.62, 937.40, 927.76, 860.25, 844.82, 831.32, 810.10, 800.46, 781.17, 680.87, 663.51 (Aromatic C-H out of plane bending bands and C-S stretching band) (Figure 60).

¹H NMR (300 MHz, DMSO-*d₆*): 2.97 (t, *J*= 7.29 Hz, *J*= 7.71 Hz, 2H), 3.15 (t, *J*= 7.14 Hz, 7.59 Hz, 2H), 4.35 (s, 2H), 5.88 (s, 2H), 6.64 (d, *J*= 7.91 Hz, 1H), 6.78 (d, *J*= 7.89 Hz, 1H), 6.86 (s, 1H), 7.19-7.22 (m, 1H), 7.66-7.73 (m, 2H), 7.86 (d, *J*= 8.58 Hz, 2H), 8.03 (d, *J*= 8.58 Hz, 2H) ( 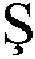 ekil 61).

¹³C NMR (75 MHz, DMSO-*d₆*): 23.11 (CH₂), 26.74 (CH₂), 32.73 (CH₂), 101.08 (CH₂), 108.55 (CH), 109.42 (CH), 121.73 (CH), 125.82 (CH), 126.07 (2CH), 127.75 (CH), 128.70 (2CH), 129.34 (CH), 132.53 (C), 134.70 (C), 137.27 (C), 140.60 (C), 142.46 (C), 145.97 (C), 147.62 (C), 153.28 (C), 154.39 (C) (Figure 62).

HRMS (ESI) (*m*/*z*): [M+H]⁺ calculated for C₂₃H₁₈N₄O₂S₂ : 447.0944, found: 447.0920 (Figure 63).

### B. Analysis of The Obtained Compounds and The Evaluation of Their Results

### Thin Layer Chromatography (TLC) Studies

The study was used to monitor the reactions. In TLC studies, 20x20 cm ready silica gel plates were used as adsorbents and petroleum ether-ethyl acetate (3:1, 1:1) solvent system was used as the solvent system. The samples obtained from the reaction medium and the starting compounds were dissolved in ethyl alcohol and applied onto the plate and then dragged in a TLC tank. After the dragging was over, spots were detected under UV light at a wavelength of 254 and 366 nm.

### Melting point (M.p.) Determination

The melting points of the obtained compounds were determined with a melting point apparatus (Mettler Toledo, Ohio, USA) using one end closed capillary tubes.

### Taking Infrared (IR) Spectra and Evaluation of The Results

The IR spectra of the obtained compounds were recorded with an IRPrestige-21 Fourier Transform (FT) IR spectrophotometry (Shimadzu, Japan).

In the IR spectra of compounds **1-15,** the absence of N-H stretching bands shows that the triazolothiadiazine ring is closed. In the IR spectra of these compounds, aromatic and aliphatic C-H stretching bands were observed at 3116.97-3003.17 cm⁻¹ and 2995.45-2771.71 cm⁻¹, respectively. C=C and C=N stretching bands appeared at 1629.85-1440.83 cm⁻¹, C-H bending, C-N stretching, C-O stretching and aromatic C-H in-plane bending bands were observed at 1438.90-1001.06 cm⁻¹, aromatic C-H out of plane bending bands and C-S stretching bands occurred at 999.13-586.36 cm⁻¹. In the IR spectrum of compound **3** carrying the cyano substituent, the C=N stretching band was observed at 2233.57 cm⁻¹.

### Obtaining Nuclear Magnetic Resonance (NMR) Spectra and Evaluation of The Results

The ¹H and ¹³C NMR spectra of the obtained compounds were taken in NMR spectrometer (Bruker, Billerica, MA, USA) after they were dissolved in DMSO-*d*₆.

In the ¹H NMR spectra of compounds **1-15,** the absence of protons belonging to the amino group shows that the triazolothiadiazine ring is closed. The methylene protons bound to the 7th position of the triazolothiadiazine ring were observed as a singlet at 4.31-4.48 ppm. The ethyl protons attached to the third position of the triazolothiadiazine ring were observed as triplets at 2.94-2.99 ppm and 3.13-3.20 ppm, respectively. The methylene protons of the benzodioxole ring were observed as a singlet at 5.83-5.88 ppm. The protons belonging to aromatic and heteroaromatic rings were observed at 6.61-8.59 ppm.

The protons belonging to the methoxy group of compound 7 were observed as a singlet at 3.85 ppm, the protons belonging to the methyl substituent of compound **8** were observed as a singlet at 2.39 ppm. The protons belonging to the methylsulfonyl group of compound **9** appeared as a singlet at 3.43 ppm.

In the ¹³C NMR spectra of compounds **1-15,** the C₃, C₆, C₇ and C₈ₐ carbons, the common carbons of the triazolothiadiazine ring, were observed in the region 147.59-154.06 ppm, 153.56-163.00 ppm, 32.33-32.76 ppm, and 153.28-154.06 ppm, respectively. The ethyl carbons attached to the third position of the triazolothiadiazine ring were observed at 23.11-23.48 ppm and 26.53-26.74, respectively. The methylene carbons of the benzodioxole ring were observed at 101.07-101.13 ppm. The aromatic carbons appeared at 108.51-164.37 ppm. The cyano carbon of compound **3** was observed at 118.74 ppm. The methoxy carbon of compound **7** was determined at 56.04 ppm, the methyl carbon of compound **8** was observed at 21.54 ppm, the methylsulfonyl carbon of compound **9** appeared at 43.75 ppm. The trifluoromethyl carbon of compound **11** was observed at 126.35 ppm.

### Obtaining Mass Spectra and Evaluation of The Results

The High Resolution Mass Spectrometry (HRMS) analyses of the compounds were carried out at a LCMS-IT-TOF Mass spectrometry (Shimadzu, Kyoto, Japan) using the electrospray ionization (ESI) technique after they were solved in methanol and the obtained charged atom or groups were determined according to the mass/charge (m/z) ratio. In the mass spectra of these compounds, the [M+H]⁺ peaks were observed.

### C. In silico Studies

### C.1 In Silico Molecular Docking Studies

Molecular docking studies were carried out at the substrate binding sites of COX-2, Akt and ROCK enzymes for the novel triazolothiadiazine derivatives (**1-15**), which were synthesized within the scope of the invention. The compounds were prepared with energy minimization at the physiological pH (pH= 7.4) *via* Optimized Potentials for Liquid Simulations (OPLS_2005) in the "ligand preparation" module of Schrödinger's Maestro molecular docking program. The complex X-ray crystal structure of COX-2 together with indometacin was obtained from the PDB server (PDB code: 4COX). Moreover, the X-ray crystal structures of the Akt (PDB code: 3OW4) and ROCK (PDB code: 5HVU) enzymes were also obtained from PDB and all of these enzymes were optimized in the "protein preparation" module of the Schrodinger software. For the prediction of the topologies of the ligands in molecular docking studies, Glide/XP docking protocols were applied at the substrate binding sites of these enzymes.

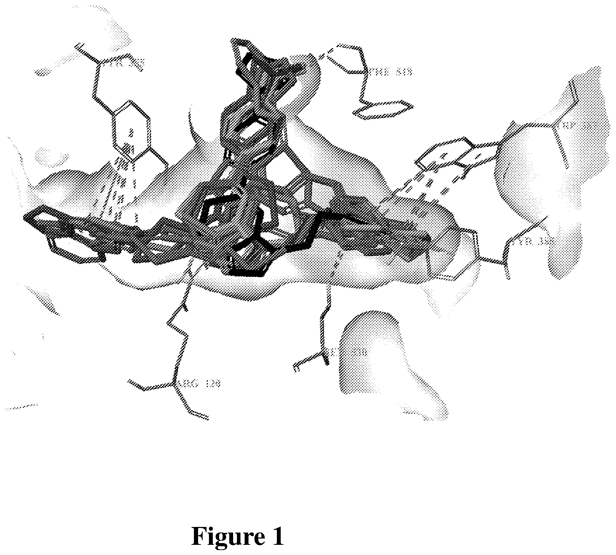

According to the results of the molecular docking studies carried out at the active site of COX-2 enzyme, in particular the 1,3-benzodioxole ring plays a significant role in high affinity to the active site by forming π-π interactions generally with Arg120, Ser530, Trp387, Tyr355 and Tyr385 amino acids. Besides, in several compounds the triazolothiadiazine ring system also formed a π-cation bond with Arg120 amino acid. It was observed that the nitro and methylsulfonyl groups linked to the 4th position of the phenyl ring at the 6th position of the triazolothiadiazine ring formed a hydrogen bond with Phe518 amino acid and the chlorophenyl, bromophenyl, methylphenyl groups and the naphthyl moieties formed π-π interactions with Tyr355 and 385 amino acids (Figure 1).

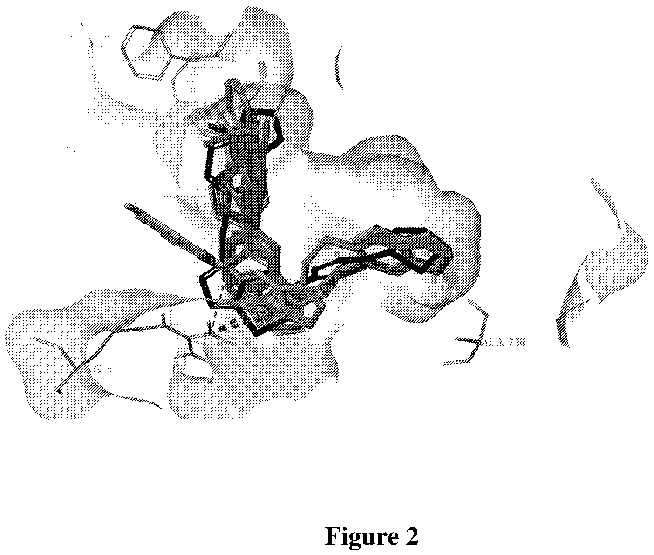

According to the results of the molecular docking studies carried out at the active site of Akt enzyme, the 1,3-benzodioxole ring formed a hydrogen bond with Ala230 amino acid in all compounds, however the triazolothiadiazine ring formed a π-cation bond with Arg4 amino acid. The nitro, the cyano, the methylsulfonyl groups linked to the phenyl at the 6th position of the triazolothiadiazine ring, formed hydrogen bonds with Phe161 and Lys179 amino acids and the biphenyl group formed a π-π interaction with Phe161 (Figure 2).

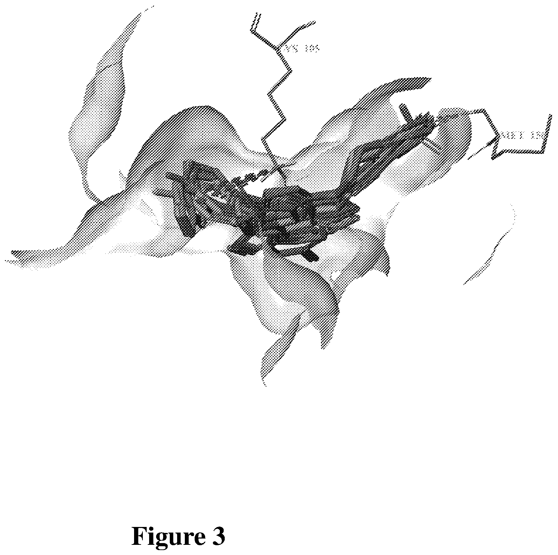

According to the results of the molecular docking studies carried out at the active site of ROCK enzyme, the 1,3-benzodioxole ring of all compounds formed a hydrogen bond with Met156 amino acid and a π-cation bond with Lys105 amino acid. The nitro and methylsulfonyl groups linked to the 4th position of the phenyl moiety at the 6th position of the triazolothiadiazine ring formed hydrogen bonds with Met156 and the fluoro, the chloro and the methoxy groups formed π-cation bonds with Lys105 amino acid (Figure 3).

The glide emodel scores of the compounds are generally used to compare the docking results of different conformations of the same compound, while the docking scores of the compounds are used to compare the docking results of different compounds. The docking score, glide gscore and the glide emodel results obtained as a result of the docking studies carried out for three different enzymes of all compounds are illustrated in Table 1.

**Table 1. The results of the docking score (kcal/mol), glide gscore (kcal/mol) and glide emodel (kcal/mol) results of the compounds for COX-2 (PDB code: 4COX), Akt (PDB code: 3OW4) and ROCK (PDB code: 5HVU) enzymes**

| **Compound** | **4COX** | | | **3OW4** | | | **5HVU** | | |
|---|---|---|---|---|---|---|---|---|---|
| | **Docking score** | **Glide gscore** | **Glide emodel** | **Docking score** | **Glide gscore** | **Glide emodel** | **Docking score** | **Glide gscore** | **Glide emodel** |
| **1** | -8.44 | -8.44 | -47.75 | -7.04 | -7.04 | -69.06 | -7.43 | 7.43 | -62.84 |
| **2** | -8.51 | -8.51 | -54.57 | -7.59 | -7.59 | -80.25 | -7.73 | -7.73 | -75.66 |
| **3** | -7.85 | -7.85 | -48.11 | -7.41 | -7.41 | -72.53 | -6.37 | -6.37 | -66.66 |
| **4** | -8.54 | -8.54 | -53.65 | -7.04 | -7.04 | -71.05 | -7.86 | -7.86 | -65.34 |
| **5** | -8.86 | -8.86 | -50.39 | -7.06 | -7.06 | -74.00 | -7.88 | -7.88 | -68.20 |
| **6** | -8.71 | -8.71 | -61.56 | -6.94 | -6.94 | -73.77 | -7.20 | -7.20 | -68.93 |
| **7** | -8.32 | -8.32 | -42.74 | -7.09 | -7.09 | -70.86 | -7.98 | -7.98 | -71.81 |
| **8** | -8.87 | -8.87 | -56.20 | -7.21 | -7.21 | -68.47 | -6.84 | -6.84 | -75.19 |
| **9** | -9.60 | -9.60 | -71.91 | -7.59 | -7.59 | -78.27 | -7.77 | -7.77 | -68.68 |
| **10** | -8.75 | -8.75 | -56.20 | -7.28 | -7.28 | -78.74 | -6.79 | -6.79 | -69.28 |
| **11** | -8.11 | -8.11 | -35.49 | -7.21 | -7.21 | -81.87 | -7.64 | -7.64 | -68.03 |
| **12** | -8.13 | -8.13 | -39.92 | -6.53 | -6.53 | -77.49 | -7.58 | -7.58 | -64.63 |
| **13** | -8.28 | -8.28 | -45.75 | -6.79 | -6.79 | -73.24 | -7.37 | -7.37 | -70.12 |
| **14** | -6.90 | -6.90 | -29.59 | -7.32 | -7.32 | -70.19 | -6.91 | -6.91 | -71.62 |
| **15** | -8.40 | -8.40 | -47.01 | -6.72 | -6.72 | -73.65 | -7.57 | -7.57 | -65.40 |

### C.2 In silico ADME Studies

Some pharmacokinetic features of the compounds of the invention (compounds **1-15**) and their compliance to Lipinski's rule of five and Jorgensen's rule of three were *in silico* predicted. According to the results, all of the compounds complied with the recommended values / ranges. This outcome shows that the compounds can be highly orally bioavailable potential drug candidates (Table 2).

**Table 2. In silico ADME results of the compounds (1-15)**

| **Compound** | **QPlogBB* (-3 - 1.2)** | **QPlogKhsa* (-1.5 - 1.5)** | **PSA* (7.0 - 200.0)** | **Oral absorption% (>%80 high, <%25 low)** | **Lipinski's rule of five**** | **Jorgensen's rule of three***** |
|---|---|---|---|---|---|---|
| **1** | -0.125 | 0.453 | 62.153 | 100.000 | 1 | 0 |
| **2** | -1.163 | 0.261 | 105.430 | 91.525 | 0 | 0 |
| **3** | -0.930 | 0.177 | 87.784 | 95.302 | 0 | 1 |
| **4** | 0.042 | 0.393 | 59.918 | 100.000 | 0 | 0 |
| **5** | 0.025 | 0.587 | 62.619 | 100.000 | 0 | 1 |
| **6** | 0.036 | 0.612 | 62.590 | 100.000 | 0 | 1 |
| **7** | -0.226 | 0.332 | 68.408 | 100.000 | 0 | 0 |
| **8** | -0.157 | 0.542 | 60.785 | 100.000 | 0 | 0 |
| **9** | -0.998 | -0.307 | 98.175 | 90.261 | 0 | 0 |
| **10** | 0.005 | 0.812 | 60.276 | 100.000 | 1 | 1 |
| **11** | 0.144 | 0.865 | 63.575 | 100.000 | 1 | 1 |
| **12** | -0.004 | 0.714 | 63.565 | 100.000 | 0 | 0 |
| **13** | 0.048 | 0.559 | 63.533 | 100.000 | 0 | 1 |
| **14** | -0.238 | 1.070 | 63.569 | 100.000 | 1 | 1 |
| **15** | -0.070 | 1.023 | 63.570 | 100.000 | 1 | 1 |

| | | | | | | |
|---|---|---|---|---|---|---|
| * QPlogBB: Predicted brain/blood partition coefficient, QPlogKhsa: Prediction of binding to human serum albumin, PSA: Van der Waals surface area of polar nitrogen and oxygen atoms. ** Lipinski's rule of five: Number of violations of Lipinski's rule of five. These rules: The molecular weight of the molecule < 500, QPlogPo/w (Predicted octanol/water partition coefficient) < 5, Hydrogen bond donor atoms ≤ 5, Hydrogen bond acceptor atoms ≤ 10. Compounds that do not violate these rules more than once can be considered as drug candidates. *** Jorgensen's rule of three: Number of violations of Jorgensen's rule of three. These rules: QPlogS (Predicted aqueous solubility) > -5.7, QPPCaco (Predicted apparent Caco-2 cell permeability in nm/sec) > 22 nm/s, # Primary Metabolites < 7. Compounds with fewer (and preferably no) violations of these rules are considered as more suitable agents to be used orally. | | | | | | |

### D. Anticancer Activity Studies

### D.1. Cell Culture and In Vitro Cytotoxic Activity Studies

The *in vitro* cytotoxic effects of the compounds were determined with the MTT assay. For this purpose, MTT test was performed using A549 and CCD-19Lu cell lines. After the cell lines used in the invention were centrifuged following taking them out from liquid nitrogen (-196 °C), the cells were cultured in a CO₂ incubator comprising 5% CO₂ at 37 °C in flasks containing 10% fetal bovine serum (FBS), where CCD-19Lu cells were cultured with Dulbecco's Modified Eagle's Medium (DMEM) and A549 cells were cultured with Roswell Park Memorial Institute (RPMI). The cells that reach the sufficient number for the assay, were seeded to 96 well plates such that 2×10³ cells were placed in each well and at the same time, components at concentrations of 1-500 µg/mL were added to cells and they were incubated at 37 °C. At the end of the 24 hour incubation period, the MTT powder (5 mg/mL in 1 × phosphate buffered saline (PBS)) was added to each well (20 µL). After a 2-4 h period under the same conditions, the medium was removed from the plate and 100 µL of DMSO was added to each well to dissolve the dye and kept for 10 minutes. Then the plates were read at a wavelength of 540 nm using ELx808-IU Bio-Tek plate reader. The control cell viability which was not treated with the components was accepted to be 100% and the viability rates of the cells were expressed as %. Cisplatin was used as a positive control and the results were compared according to this positive control. The dosage and time ranges, were determined according to the cytotoxic concentrations exhibited by similar derivatives in literature against various cancer types. According to the results obtained, the dose range was extended until the IC₅₀ value was determined.

### D.2. Apoptotic Activity Studies

### Annexin V FITC: Determination of Early Apoptosis

After the active compounds were determined with the MTT assay, their apoptotic effects on A549 cells depending on IC₅₀ concentrations were determined in flow cytometry with BD, Pharmingen flow cytometry kit according to the manufacturer's recommendations. Briefly, after the cells were centrifuged for 4 minutes at 800 rpm, they were washed twice with 1 × PBS and were suspended in the 1 × Annexin-V binding solution at a concentration of 2-3 × 10⁶ cell/mL and then 100 µL of the cell solution was transferred into tubes. Then, 5 µL of Annexin-V Fluorescein isothiocyanate (FITC) and 10 µL of propidium iodide (PI) solution were added to each tube, after incubation for 15 minutes at room temperature, 400 µL × Annexin-V binding solution was added to each tube and the cells were analyzed by flow cytometry (BD FACSCalibur^{™}) within 1 hour. The early and late apoptotic cell percentages were calculated according to positive control.

### Determination of Caspase-3 Activity with Flow Cytometry

After the active compounds were determined with the MTT assay, their caspase-3 activities on A549 cells depending on IC₅₀ concentrations were determined in flow cytometry with BD, Pharmingen flow cytometry kit according to the manufacturer's recommendations. Briefly, the cells were incubated at the determined doses and times with the compounds. The cells were washed twice with cold 1 X PBS. These cells were suspended with BD Cytofix/Cytoperm solution (1×10⁶ cell / 0.5 mL). Then they were left to wait on ice for 20 minutes. After centrifugation, the cells were removed with the BD Cytofix/Cytoperm solution. The cells were washed twice with BD Perm/Wash^{™} buffer (1×10⁶ cell/0.5 mL). Afterwards the cells were re-suspended with BD Perm/Wash^{™} buffer and antibodies and they were kept at room temperature for 30 minutes. For each test, the cells were washed in 1 mL BD Perm/Wash^{TM} buffer and were suspended in 0.5 mL BD Perm/Wash^{TM} buffer and they were analyzed with flow cytometry. Cisplatin was used as a positive control and the results were compared according to this positive control.

### Staining Cells with JC-1 (Determination of Mitochondrial Membrane Integrity)

After the active compounds were determined with the MTT assay, the determination of mitochondrial membrane integrity in A549 cells depending on IC₅₀ concentrations were carried out in flow cytometry with BD, Pharmingen flow cytometry kit according to the manufacturer's recommendations. Briefly, the cells were seeded with optimal density (must not exceed 1×10⁶ cell/mL). Following this, the cells were incubated with concentrations and time suitable for the compounds to be tested. After treatment, each cell suspension was transferred into a 15 mL polystyrene centrifuge tube and the cells were centrifuged at 400 × g for 5 minutes at room temperature, and supernatant was removed. Later, 0.5 mL freshly prepared working solution was added to each pellet and was vortexed. The cells were incubated in JC-1 working solution at 37 °C for 10-15 minutes and they were washed twice. In the first wash, 2 mL 1X assay buffer was added and the cells were precisely suspended. Following this, the cells were centrifuged at 400 × g for 5 minutes and the supernatant was removed carefully. In the next wash, 1 mL 1X assay buffer was added and was vortexed. After the cells were centrifuged at 400 × g for 5 minutes, each cell pellet was suspended in a 0.5 mL 1X assay buffer and was vortexed. Finally, the cell is analyzed with flow cytometry. Cisplatin was used as a positive control and the results were compared according to this positive control.

### Determination of Cell Cycle Arrest

After the active compounds were determined with the MTT assay, their effects on the cell cycle in A549 cells depending on IC₅₀ concentrations were carried out according to the manufacturer's recommendations. Briefly, the cells were suspended in a citrate buffer solution. The cells were centrifuged at 400 × g for 5 minutes at room temperature. The supernatant was removed and 250 µL solution A was added on the pellet. Following this, 200 µL solution B was added and it was kept at room temperature 10 minutes. Afterwards 200 µL solution C was added and it was kept at +4 °C for 10 minutes. The cells were analyzed with flow cytometry using BD Biosciences ModFit software.

### D.3 Investigation of In Vitro COX, Akt and ROCK Inhibitory Effects

The intracellular COX, Akt and ROCK inhibitory effects of the active compounds determined with the MTT assay, were measured with the colorimetric assay according to the manufacturer's recommendations.

### Investigation of In vitro COX Inhibitory Effects

The lysates of A549 cells incubated for 24 hours with IC₅₀ doses of the active compounds determined with MTT assay were obtained and used in the test. COX activity was carried out using Cayman COX activity assay kit in compliance with the manufacturer's recommendations. Briefly, 150 µL of each sample was kept in boiling water for 5 minutes and centrifuged at 8000 × g for 1 minute and it was used to obtain background values. 120 µL assay solution, 10 µL hemin and 40 µL sample and inactive samples were applied to the sample and background wells in triplicate. 110 µL assay solution, 10 µL hemin and 40 µL sample, 10 µL DuP-697 or SC-560 was added to the inhibitor wells (DuP-697 inhibits COX-2 activity; while SC-560 inhibits COX-1 activity). The plate was shaked for a few seconds and it was left at 25 °C for 5 minutes. 20 µL colorimetric substrate was added to all wells. Moreover, a reaction was started by adding 20 µL arachidonic acid solution and the plate was shaked for a few seconds and it was left at 25 °C for 5 minutes. The absorbance values were read at 540 nm.

### Investigation of In Vitro Akt Inhibitory Effects

After 10.000 cells were seeded in the wells, they were kept in the incubator for 24 hours. After the cells were incubated with IC₅₀, IC₅₀/2 and IC₅₀/4 doses of compounds **9, 11, 12** and **14,** cisplatin and Akt inhibitor GSK690693 for 24 hours, the Akt colorimetric in cell ELISA kit protocol was applied in accordance with the manufacturer's recommendations. Briefly, the medium was removed from the wells and 100 µL of 4% formaldehyde was added. The plate was incubated in a fume hood for 15 minutes. Formaldehyde was removed and the plate was washed twice with 1X tris-borate solution (TBS) such as to be 100 µL/well. 1X TBS was removed and 100 µL 1X permeabilization solution was added to each well and it was kept at room temperature for 15 minutes. After the 1X permeabilization solution was removed, the wells were washed with 100 µL 1X TBS once. 1X TBS was removed and 100 µL quenching solution was added to each well and it was kept at room temperature for 20 minutes. Afterwards, this solution was removed. The plate was washed 1 time with 1X TBS and 100 µL blocking solution was added to the wells and it was kept at room temperature for 30 minutes. At the end of this period, the blocking solution was removed and 50 µL primary antibody was added to the wells. The plate was sealed with a cover and it was incubated overnight at 4 °C. The primary antibody was removed and the plate wells were washed 3 times with 100 µL 1X washing solution. After the washing solution was removed, 100 µL diluted horseradish peroxidase (HRP) conjugate was added and it was incubated at room temperature for 30 minutes. Later on this conjugate was removed and 100 µL 3,3',5,5'-tetramethylbenzidine (TMB) stop solution was added to each well and the absorbance values were read at 450 nm for 30 minutes. The experiment was carried out in triplicate. The values of the blank wells were subtracted from the values of the untreated control and treated cells. The Akt activity% was determined as the relative absorbance of treated versus untreated control cells.

### Investigation of In vitro ROCK Inhibitory Effects

The lysates of A549 cells incubated for 24 hours with IC₅₀ doses of the active compounds determined with MTT assay were obtained and used. Two wells were used for each sample. 90 µL active ROCK-II positive control and samples were added to the wells of the substrate plate. 10 X kinase solution comprising DTT and ATP was added to 10 µL wells and the kinase reaction was started and the plate was incubated at 30 °C for 30-60 minutes. 50 µL 0.5 M EDTA was added to each well at pH=8.0 and the reaction was stopped. The wells were emptied and they were washed 3 times with 250 µL 1X washing solution. After the final washing, 100 µL anti-phospho-MYPT1 (Thr696) antibody was added. The plate was closed and it was incubated at room temperature for 1 hour on an orbital shaker. After the wells were washed three times again, 100 µL HRP-conjugated secondary antibody was added and it was incubated again at room temperature for 1 hour on an orbital shaker. After the wells were emptied, they were washed 3 times. Then 100 µL of substrate solution was added. It was incubated at room temperature for 5-20 minutes on an orbital shaker. 100 µL of stop solution was added. The absorbance of each well was read at a wavelength of 450 nm in a spectrophotometric microplate reader.

### E. Evaluation of In Vitro Activity Results

### E.1 Evaluation of MTT Assay Results

MTT assay is a frequently used method which measures the cytotoxic effects of the compounds on the cells by converting the yellow colored MTT stain into purple colored formazan crystals *via* the mitochondrial succinate dehydrogenase activity. In the cytotoxic activity tests of the compounds, A549 lung adenocarcinoma and CCDLu19 normal lung fibroblast cell lines were used.

The anticancer effects of the final compounds on A549 human lung adenocarcinoma cell line were evaluated using the MTT assay and the IC₅₀ values were determined (Table 3). Compounds **7, 9, 10, 11, 12, 14** and **15** exhibited cytotoxic activity against A549 cell line at lower doses (IC₅₀= 45.67±5.51 µg/mL, 5.75±2.47 µg/mL, 4.10±0.14 µg/mL, 0.73±0.16 µg/mL, 0.80±0.05 µg/mL, 2.63±0.72 µg/mL and 4.00±0.20 µg/mL, respectively). Cisplatin (IC₅₀= 23.00±1.41 µg/mL) and GSK690693 (IC₅₀= 6.75±1.06 µg/mL) were used as positive controls. However, compounds **7, 10** and **15** exhibited cytotoxic activity against CCDLu19 cells (IC₅₀= 44.33±1.15 µg/mL, <3.90 µg/mL and <3.90 µg/mL, respectively).

**Table 3. The cytotoxic effects of the compounds on A549 and CCDLu19 cell lines.**

| **Compound** | **IC₅₀ values (µg/mL)** | | **SI values*** |
|---|---|---|---|
| | **A549 cell line** | **CCDLul9 cell line** | |
| **1** | 103.33±11.50 | <3.90 | - |
| **2** | 380.00±70.71 | 3.93±0.06 | - |
| **3** | >500 | >500 | - |
| **4** | 325.00±35.36 | >500 | - |
| **5** | >500 | <3.90 | - |
| **6** | 306.67±58.59 | <3.90 | - |
| **7** | 45.67±5.51 | 44.33±1.15 | - |
| **8** | >500 | 14.67±6.43 | - |
| **9** | 5.75±2.47 | 101.67±10.41 | 17.68 |
| **10** | 4.10±0.14 | <3.90 | - |
| **11** | 0.73±0.16 | 113.33±5.77 | 155.25 |
| **12** | 0.80±0.05 | 34.33±2.08 | 42.91 |
| **13** | >500 | >500 | - |
| **14** | 2.63±0.72 | 65.00±5.00 | 24.71 |
| **15** | 4.00±0.20 | <3.90 | - |
| **Cisplatin** | 23.00±1.41 | - | - |
| **GSK690693** | 6.75±1.06 | - | - |

| | | | |
|---|---|---|---|
| * SI= IC₅₀ for CCDLul9 cell line / IC₅₀ for A549 cell line. | | | |

Compounds **9, 11, 12** and **14,** which are non-cytotoxic against CCDLu19 cells, have higher cytotoxic activity against A549 cells than cisplatin (Table 3). According to these results, it was determined that 4-methylsulfonylphenyl, 4-trifluoromethylphenyl, 3-fluorophenyl and biphenyl substitutents at the sixth position of the triazolothiadiazine ring significantly increased anticancer activity against A549 cell line. The fact that compound **12** carrying 3-fluorophenyl moiety has high anticancer efficacy and compound **4** carrying 4-fluorophenyl moiety does not show any anticancer efficacy, indicates the importance of the position of the fluorine substituent for anticancer efficacy.

### E.2 Evaluation of Effects on Apoptosis

One of the general aims of the chemotherapeutic agents used in cancer treatment is to force cancerous tissue cells to undergo programmed cell death (apoptosis). In recent years, apoptosis has been the target of the studies related to the search for more effective therapeutic agents for cancer treatment. Apoptosis is a morphologically different type of cell death that plays an important role in early growth and development of normal mature tissues. In this study, Annexin V FITC, caspase-3, JC-1 and cell cycle assays were conducted to determine apoptosis.

Phosphatidylserine is carried from the inner surface of the cell membrane towards the external surface of the cell membrane in apoptotic cells. This change was determined in flow cytometry with the Annexin V FITC method. This apoptotic pathway enables the activation of proteolytic enzymes known as caspases that mediate the rapid disintegration of cellular organelles and skeleton. Among them, caspase-3 is the most important and the best defined one. Caspase-3 activates death proteases that catalyze the specific cleavage of most cellular key proteins. For this reason, in this study caspase-3 activation was determined with flow cytometry. JC-1 staining assay is a method that measures mitochondrial membrane potential in cells. In apoptotic cells, the mitochondria membrane is depolarized.

### Annexin V FITC: Determination of Early Apoptosis

At the end of the incubation of compounds **9, 11, 12** and **14** and cisplatin in A549 cells for 24 hours, the Annexin V-PI binding capacities were analyzed in flow cytometry (Table 4 and Figure 64).

**Table 4. The Annexin V-FITC / PI flow cytometry quandrant analysis percentages of A549 cells treated with compounds 9, 11, 12 and 14 and cisplatin**

| **Compound** | **Early apoptotic cells%** | **Late apoptotic cells%** | **Viable cells%** | **Necrosis%** |
|---|---|---|---|---|
| **Control** | 2.70 | 3.60 | 91.40 | 2.30 |
| **9** | 5.00 | 16.20 | 72.50 | 6.30 |
| **11** | 2.90 | 9.90 | 72.30 | 14.80 |
| **12** | 4.80 | 29.70 | 24.70 | 40.80 |
| **14** | 4.20 | 21.70 | 39.50 | 34.60 |
| **Cisplatin** | 14.70 | 30.10 | 26.60 | 28.70 |

At the end of the flow cytometric analysis of A549 cells, the early and late apoptotic effects of compounds **9, 11, 12** and **14** and cisplatin (5.75, 0.73, 0.80, 2.63 and 23.00 µg/mL, respectively at IC₅₀ concentrations) were determined as 21.20%, 12.80%, 34.50%, 25.90% and 44.80%, respectively, whereas the necrotic cell percentages were determined as 6.30%, 14.80%, 40.80%, 34.60% and 28.70%, respectively. For each sample, at least 10.000 cell quadrant analysis was performed. According to these findings, compounds **12** and **14** caused higher early and late apoptotic effects on A549 cell line in comparison to compounds **9** and **11.** It was determined that 3-fluorophenyl and biphenyl substituents at the 6th position of the triazolothiadiazine ring system increased apoptotic activity.

### Caspase-3 Antibody Apoptosis: Determination of Early Apoptosis

In order to investigate caspase-3 activation, A549 cells were incubated with compounds **9, 11, 12, 14** and cisplatin (5.75, 0.73, 0.80, 2.63 and 23 µg/mL at IC₅₀ concentrations) for 24 hours (Table 5 and Figure 65).

**Table 5.** The flow cytometry quadrant analysis percentages of active caspase-3 activity in A549 cells treated with compounds **9, 11, 12,** and **14** and cisplatin (5.75, 0.73, 0.80, 2.63 and 23 µg/mL for A549 cells).

| **Compound** | **Caspase-3 negative (-) cells%** | **Caspase-3 positive (+) cells%** |
|---|---|---|
| **Control** | 97.70 | 2.40 |
| **9** | 96.70 | 3.40 |
| **11** | 97.00 | 3.10 |
| **12** | 97.80 | 2.40 |
| **14** | 96.60 | 3.70 |
| **Cisplatin** | 89.70 | 10.30 |

Following flow cytometry analysis, the caspase-3 positive cell percentages of compounds **9, 11, 12** and **14** and cisplatin on A549 cells were determined as 3.40%, 3.10%, 2.40%, 3.70% and 10.30%, respectively, whereas the caspase-3 negative cell percentages of these compounds were determined as 96.70%, 97.00%, 97.80%, 96.60% and 89.70%, respectively. According to these results, the compounds (except compound **12)** caused caspase-3 activation on A549 cells in comparison to control.

### Determination of Mitochondrial Membrane Polarization / Depolarization

In order to determine the mitochondrial membrane polarization/depolarization, A549 cells were incubated with compounds **9, 11, 12** and **14** and cisplatin (5.75, 4.10, 0.73, 0.80, 2.63 and 23 µg/mL at IC₅₀ concentrations) for 24 hours (Table 6 and Figure 66).

**Table 6. The mitochondrial membrane polarized/depolarized percentages of A549 cells treated with compounds 9, 11, 12, 14 and cisplatin (5.75, 0.73, 0.80, 2.63 and 23 µg/mL for A549 cells)**

| **Groups** | **Mitochondrial membrane polarized (P1) cells%** | **Mitochondrial membrane depolarized (P2) cells%** |
|---|---|---|
| Control | 94.30 | 5.60 |
| Cells treated with compound **9** | 91.10 | 8.80 |
| Cells treated with compound **11** | 41.30 | 58.80 |
| Cells treated with compound **12** | 53.00 | 47.00 |
| Cells treated with compound **14** | 53.20 | 47.20 |
| Cells treated with **cisplatin** | 52.40 | 48.00 |

Following flow cytometry analysis, mitochondrial membrane depolarized cell percentages of compounds **9, 11, 12, 14** and cisplatin on A549 cells were determined as 8.80%, 58.80%, 47.00%, 47.20% and 48.00%, respectively, whereas the mitochondrial membrane polarized cell percentages of these compounds were determined as 91.10%, 41.30%, 53.00%, 53.20% and 52.40%, respectively. According to these results, compounds **11, 12** and **14** caused higher mitochondrial membrane depolarization than compound **9** on A549 cells. Moroever, compound **11** also caused higher mitochondrial membrane depolarization than positive control cisplatin. It was determined that the trifluoromethyl substituent at the 4th position of the phenyl ring linked to the 6th position of the triazolothiadiazine ring system increased mitochondrial membrane depolarization.

### Determination of Cell Cycle Arrest

The development of cell proliferation comprises three major control points, namely G₁/S, S and G₂/M phases. The molecules playing a role in the late G₁ phase are necessary for apoptosis induction, as apoptosis mostly occurs in proliferating cells. The correlation between the cell cycle and apoptosis was reported. For this reason, compounds **9, 11, 12** and **14** caused an increase in the G₁ phase (53.29%, 57.29%, 54.20%, 54.92%) and this increase is higher than the increase caused by cisplatin (45.92%) (Table 7, Figure 67).

**Table 7. The effects of compounds 9, 11, 12, 14 and cisplatin on the cell cycle in A549 cells**

| **Groups** | **A549 cells** | | |
|---|---|---|---|
| | **G₀/G₁%** | **S%** | **G₂/M%** |
| Control | 36.31 | 57.77 | 5.92 |
| Cells treated with compound **9** | 53.29 | 37.04 | 9.66 |
| Cells treated with compound **11** | 57.29 | 34.57 | 8.15 |
| Cells treated with compound **12** | 54.20 | 39.28 | 6.52 |
| Cells treated with compound **14** | 54.92 | 37.17 | 7.91 |
| Cells treated with **cisplatin** | 45.92 | 50.07 | 4.00 |

The obtained results show that the inhibitory effects of these compounds on A549 cells are related with the cell cycle regulation effects and the compounds induce apoptosis in A549 cells at the G₀/G₁ phase of the cell cycle.

### E.3 Investigation of In vitro COX, Akt and ROCK Inhibitory Effects

### Investigation of In vitro COX Inhibitory Effects

The activity test results performed to investigate the COX inhibitory effects of the compounds, showed that after 24 hours incubation at IC₅₀ concentrations, compounds **11, 12** and cisplatin caused higher COX-2 inhibition on A549 cells (Table 8). However, compound **9** caused similar effects on both COX-1 (35.21%) and COX-2 (32.66%). Compound **11** showed the highest effects on both COX-1 (54.18%) and COX-2 (65.33%). Compound **12** exhibited COX-2 inhibition selectively (for COX-1, 38.58% and for COX-2, 53.52%). Compound **14** did not exhibit significant COX-1 and COX-2 inhibition (for COX-1, 2.19% and for COX-2, 15.17%). Both the COX-1 and the COX-2 inhibitory effects of compounds **11** and **12** were higher than those of cisplatin (for COX-1, 24.65% and for COX-2, 43.46%) (Table 8). It was determined that the fluorine substituent at the 3rd position and the trifluoromethyl substituent at the 4th position of the phenyl moiety linked to the 6th position of the triazolothiadiazine ring increased COX-2 inhibition.

**Table 8. COX-1% and COX-2% inhibitions (U/mL) after 24 hours incubation of A549 cells with IC₅₀ concentrations of compounds 9, 11, 12, 14 and cisplatin.**

| | **COX-1 activity (U/mL)** | **COX-2 activity (U/mL)** | **COX-1 Inhibition%** | **COX-2 Inhibition%** |
|---|---|---|---|---|
| Control | 4.09 | 4.22 | | |
| **9** | 3.41 | 3.60 | 35.21 | 32.66 |
| **11** | 1.88 | 1.46 | 54.18 | 65.33 |
| **12** | 2.51 | 1.95 | 38.58 | 53.52 |
| **14** | 4.15 | 3.56 | 2.19 | 15.70 |
| **Cisplatin** | 3.08 | 2.38 | 24.65 | 43.46 |
| Standard | 10.99 | | | |

### Investigation of In vitro Akt Inhibitory Effects

The activity test results performed to investigate the Akt inhibitory effects of the compounds showed that after 24 hours incubation at IC₅₀ concentrations, compounds **9, 12** and **14** (45.47%, 46.87% and 44.30%, respectively) caused moderate Akt inhibition as compared to the standard agent cisplatin (59.55%) and GSK690693 (61.67%). Compound **11** did not exhibit significant Akt inhibition. The concentrations used are IC₅₀, IC₅₀/2 and IC₅₀/4 values. The effects of compounds **9, 12** and **14** do not seem to be dose-dependent. It was determined that the methylsulfonyl and phenyl substituents at the 4th position, and the fluorine substituent at 3rd position of the phenyl group bound to the 6th position of the triazolothiadiazine ring system increased Akt inhibitory effects (Table 9).

**Table 9. Akt inhibition% results after 24 hours incubation of A549 cells treated with IC₅₀, IC₅₀/2 and IC₅₀/4 concentrations of compounds 9, 11, 12, 14 and cisplatin**

| **Compound** | **Concentration** | **Inhibition%** |
|---|---|---|
| **9** | **1.44 µg/mL** | 15.99±1.06 |
| | **2.88 µg/mL** | 7.31±1.48 |
| | **5.75 µg/mL** | 45.47±3.76 |
| **11** | **0.18 µg/mL** | 0.91±1.29 |
| | **0.37 µg/mL** | 6.66±3.51 |
| | **0.73 µg/mL** | 8.18±3.74 |
| **12** | **0.20 µg/mL** | 19.24±2.43 |
| | **0.40 µg/mL** | 16.67±0.05 |
| | **0.80 µg/mL** | 46.87±4.60 |
| **14** | **0.66 µg/mL** | 23.51±3.00 |
| | **1.32 µg/mL** | 15.99±3.66 |
| | **2.63 µg/mL** | 44.30±5.68 |
| **GSK690693** | **1.69 µg/mL** | 59.23±9.77 |
| | **3.34 µg/mL** | 60.56±3.04 |
| | **6.75 µg/mL** | 61.67±1.21 |
| **Cisplatin** | **5.75 µg/mL** | 50.46±6.89 |
| | **11.50 µg/mL** | 56.23±1.45 |
| | **23.00 µg/mL** | 59.55±8.44 |

### Investigation of In vitro ROCK Inhibitory Effects

The activity test results performed to investigate the ROCK inhibitory effects of the compounds showed that after 24 hours incubation at IC₅₀ concentrations, only compound **14** (53.85%; IC₅₀= 2.55±0.21 µg/mL) caused high ROCK inhibition as compared to the standard agent cisplatin (29.84%). Moreover, the ROCK inhibitory effect of this compound is dose-dependent. Compounds **9, 11** and **12** did not exhibit significant ROCK inhibition. The concentrations used are IC₅₀ and IC₅₀/2 values. It was determined that the biphenyl substituent at the 6th position of the triazolothiadiazine ring system increased ROCK inhibitory activity (Table 10).

**Table 10. ROCK inhibition% results after 24 hours incubation of A549 cells treated with IC₅₀ and IC₅₀/2 concentrations of compounds 9, 11, 12, 14 and cisplatin**

| **Compound** | **Concentration** | **Inhibition%** |
|---|---|---|
| **9** | **2.88 µg/mL** | - |
| | **5.75 µg/mL** | 6.89±0.44 |
| **11** | **0.37 µg/mL** | - |
| | **0.73 µg/mL** | - |
| **12** | **0.40 µg/mL** | - |
| | **0.80 µg/mL** | - |
| **14** | **1.32 µg/mL** | - |
| | **2.63 µg/mL** | 53.85±8.85 |
| **Cisplatin** | **11.50 µg/mL** | - |
| | **23.00 µg/mL** | 29.84±0.16 |

## Claims

1. A compound having formula (I), or pharmaceutically acceptable salt and/or solvate thereof, **characterized by**; 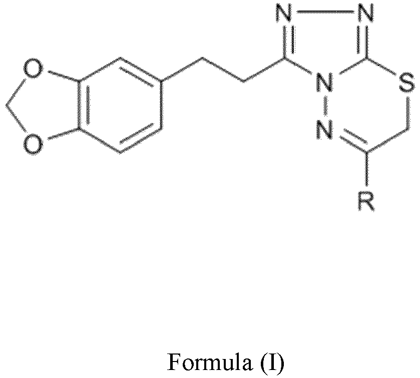 **R:** Phenyl, 4-Nitrophenyl, 4-Cyanophenyl, 4-Fluorophenyl, 4-Chlorophenyl, 4-Bromophenyl, 4-Methoxyphenyl, 4-Methylphenyl, 4-Methylsulfonylphenyl, Naphthalene-2-yl, 4-Trifluoromethylphenyl, 3-Fluorophenyl, 3-Chlorophenyl, Biphenyl-4-yl or 4-(Thiophene-2-yl)phenyl.

2. A compound according to Claim 1, wherein said compound is selected from the group below.
| | |
|---|---|
| 3-[2-(1,3-Benzodioxole-5-yl)ethyl]-6-phenyl-7*H*-[1,2,4]triazolo[3,4-*b*][1,3,4]thiadiazine | 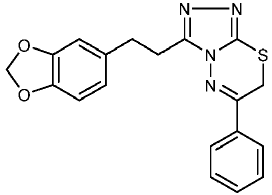 |
| 3-[2-(1,3-Benzodioxole-5-yl)ethyl]-6-(4-nitrophenyl)-7*H*-[1,2,4]triazolo[3,4-*b*][1,3,4]thiadiazine | 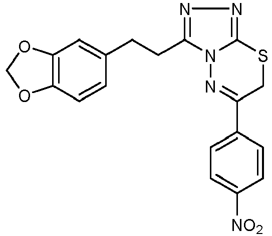 |
| 3-[2-(1,3-Benzodioxole-5-yl)ethyl]-6-(4-cyanophenyl)-7*H*-[1,2,4]triazolo[3,4-*b*][1,3,4]thiadiazine | 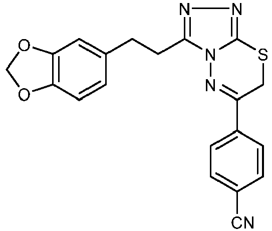 |
| 3-[2-(1,3-Benzodioxole-5-yl)ethyl]-6-(4-fluorophenyl)-7*H*-[1,2,4]triazolo[3,4-*b*][1,3,4]thiadiazine | 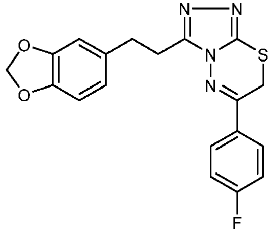 |
| 3-[2-(1,3-Benzodioxole-5-yl)ethyl]-6-(4-chlorophenyl)-7*H*-[1,2,4]triazolo[3,4-*b*][1,3,4]thiadiazine | 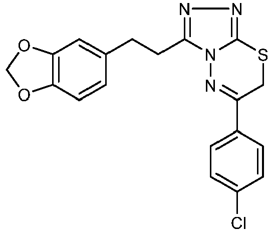 |
| 3-[2-(1,3-Benzodioxole-5-yl)ethyl]-6-(4-bromophenyl)-7*H*-[1,2,4]triazolo[3,4-*b*][1,3,4]thiadiazine | 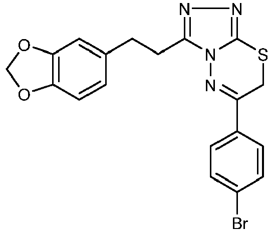 |
| 3-[2-(1,3-Benzodioxole-5-yl)ethyl]-6-(4-methoxyphenyl)-7*H*-[1,2,4]triazolo[3,4-*b*][1,3,4]thiadiazine | 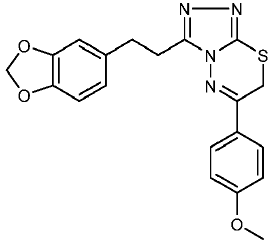 |
| 3-[2-(1,3-Benzodioxole-5-yl)ethyl]-6-(4-methylphenyl)-7*H*-[1,2,4]triazolo[3,4-*b*][1,3,4]thiadiazine | 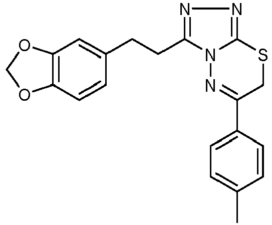 |
| 3-[2-(1,3-Benzodioxole-5-yl)ethyl]-6-[4-(methylsulfonyl)phenyl)]-7*H-*[1,2,4]triazolo[3,4-*b*][1,3,4]thiadiazine | 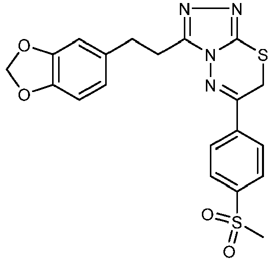 |
| 3-[2-(1,3-Benzodioxole-5-yl)ethyl]-6-(4-trifluoromethylphenyl)-7*H*-[1,2,4]triazolo[3,4-*b*][1,3,4]thiadiazine | 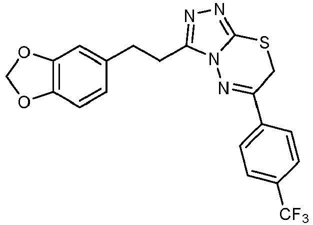 |
| 3-[2-(1,3-Benzodioxole-5-yl)ethyl]-6-(3-fluorophenyl)-7*H*-[1,2,4]triazolo[3,4-*b*][1,3,4]thiadiazine | 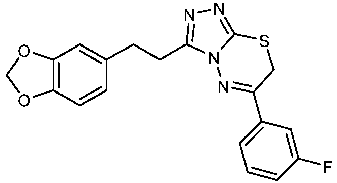 |
| 3-[2-(1,3-Benzodioxole-5-yl)ethyl]-6-(3-chlorophenyl)-7*H*-[1,2,4]triazolo[3,4-*b*][1,3,4]thiadiazine | 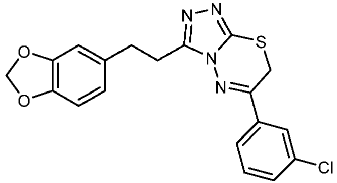 |
| 3-[2-(1,3-Benzodioxole-5-yl)ethyl]-6-(biphenyl-4-yl)-7*H*-[1,2,4]triazolo[3,4-*b*][1,3,4]thiadiazine | 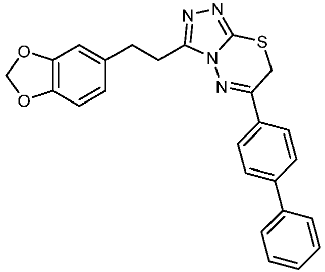 |
| 3-[2-(1,3-Benzodioxole-5-yl)ethyl]-6-(4-(thiophene-2-yl)phenyl)-7*H-*[1,2,4]triazolo[3,4-*b*][1,3,4]thiadiazine | 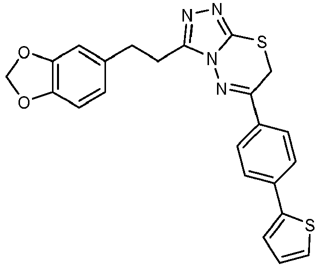 |

3. A compound of Claim 2, which is 3-[2-(1,3-Benzodioxole-5-yl)ethyl]-6-(3-fluorophenyl)-7*H*-[1,2,4]triazolo[3,4-*b*][1,3,4]thiadiazine for use as a selective COX-2 inhibitor in A549 human lung adenocarcinoma cells.

4. A compound of Claim 2, which is 3-[2-(1,3-Benzodioxole-5-yl)ethyl]-6-(4-trifluoromethylphenyl)-7*H*-[1,2,4]triazolo[3,4-*b*][1,3,4]thiadiazine for use as a nonselective COX inhibitor in A549 human lung adenocarcinoma cells.

5. A compound according to Claim 2, wherein the compound is selected between the group comprising of 3-[2-(1,3-Benzodioxole-5-yl)ethyl]-6-[4-(methylsulfonyl)phenyl)]-7*H-*[1,2,4]triazolo[3,4-*b*][1,3,4]thiadiazine, 3-[2-(1,3-Benzodioxole-5-yl)ethyl]-6-(3-fluorophenyl)-7*H*-[1,2,4]triazolo[3,4-*b*][1,3,4]thiadiazine and 3-[2-(1,3-Benzodioxole-5-yl)ethyl]-6-(biphenyl-4-yl)-7*H*-[1,2,4]triazolo[3,4-*b*][1,3,4]thiadiazine for use as an Akt inhibitor in A549 human lung adenocarcinoma cells.

6. A compound of Claim 2, which is 3-[2-(1,3-Benzodioxole-5-yl)ethyl]-6-(biphenyl-4-yl)-*7H*-[1,2,4]triazolo[3,4-*b*][1,3,4]thiadiazine for use as a ROCK inhibitor in A549 human lung adenocarcinoma cells.

7. A compound according to Claim 2, wherein the compound is selected between the group comprising of 3-[2-(1,3-Benzodioxole-5-yl)ethyl]-6-[4-(methylsulfonyl)phenyl)]-7*H-*[1,2,4]triazolo[3,4-*b*][1,3,4]thiadiazine, 3-[2-(1,3-Benzodioxole-5-yl)ethyl]-6-(4-trifluoromethylphenyl)-7*H*-[1,2,4]triazolo[3,4-*b*][1,3,4]thiadiazine, 3-[2-(1,3-Benzodioxole-5-yl)ethyl]-6-(3-fluorophenyl)-7*H*-[1,2,4]triazolo[3,4-*b*][1,3,4]thiadiazine and 3-[2-(1,3-Benzodioxole-5-yl)ethyl]-6-(biphenyl-4-yl)-7*H*-[1,2,4]triazolo[3,4-*b*][1,3,4]thiadiazine for use as an apoptosis inducer in A549 human lung adenocarcinoma cells.

8. A compound according to Claim 2, wherein the compound is selected between the group comprising of 3-[2-(1,3-Benzodioxole-5-yl)ethyl]-6-[4-(methylsulfonyl)phenyl)]-7*H-*[1,2,4]triazolo[3,4-*b*][1,3,4]thiadiazine, 3-[2-(1,3-Benzodioxole-5-yl)ethyl]-6-(4-trifluoromethylphenyl)-7*H*-[1,2,4]triazolo[3,4-*b*][1,3,4]thiadiazine and 3-[2-(1,3-Benzodioxole-5-yl)ethyl]-6-(biphenyl-4-yl)-7*H*-[1,2,4]triazolo[3,4-*b*][1,3,4]thiadiazine for use as a caspase-3 enzyme activator in A549 human lung adenocarcinoma cells.

9. A compound according to Claim 2, wherein the compound is selected between the group comprising of 3-[2-(1,3-Benzodioxole-5-yl)ethyl]-6-(4-trifluoromethylphenyl)-7*H-*[1,2,4]triazolo[3,4-*b*][1,3,4]thiadiazine, 3-[2-(1,3-Benzodioxole-5-yl)ethyl]-6-(3-fluorophenyl)-7*H*-[1,2,4]triazolo[3,4-*b*][1,3,4]thiadiazine and 3-[2-(1,3-Benzodioxole-5-yl)ethyl]-6-(biphenyl-4-yl)-7*H*-[1,2,4]triazolo[3,4-*b*][1,3,4]thiadiazine for use as a mitochondrial membrane depolarizer in A549 human lung adenocarcinoma cells.

10. A compound according to Claim 2, wherein the compound is selected between the group comprising of 3-[2-(1,3-Benzodioxole-5-yl)ethyl]-6-[4-(methylsulfonyl)phenyl)]-7*H-*[1,2,4]triazolo[3,4-*b*][1,3,4]thiadiazine, 3-[2-(1,3-Benzodioxole-5-yl)ethyl]-6-(4-trifluoromethylphenyl)-7*H*-[1,2,4]triazolo[3,4-*b*][1,3,4]thiadiazine and 3-[2-(1,3-Benzodioxole-5-yl)ethyl]-6-(3-fluorophenyl)-7*H*-[1,2,4]triazolo[3,4-*b*][1,3,4]thiadiazine and 3-[2-(1,3-Benzodioxole-5-yl)ethyl]-6-(biphenyl-4-yl)-7*H*-[1,2,4]triazolo[3,4-*b*][1,3,4]thiadiazine for use as an inducer of the cell cycle arrest at G₀/G₁ phase in A549 human lung adenocarcinoma cells.

11. A pharmaceutical composition comprising a compound according to any of the preceding claims.

12. The use of a pharmaceutical composition according to Claim 11 in the preparation of a drug to be used in the treatment of lung cancer.

13. A synthesis method of the compound according to Claim 2, **characterized in that** it comprises the steps of;
a) heating 4-amino-5-[2-(1,3-benzodioxole-5-yl)ethyl]-2,4-dihydro-3H-1,2,4-triazole-3-thione with 2-bromoacetophenone, 2-bromo-4'-nitroacetophenone, 2-bromo-4'-cyanoacetophenone, 2-bromo-4'-fluoroacetophenone, 2-bromo-4'-chloroacetophenone, 2,4'-dibromoacetophenone, 2-bromo-4'-methoxyacetophenone, 2-bromo-4'-methylacetophenone, 2-bromo-1-[4-(methylsulfonyl)phenyl]-1-ethanone, 2-bromo-2'-acetonaphthone, 2-bromo-4'-phenylacetophenone, 2-bromo-4'-trifluoromethylacetophenone, 2-bromo-3'-fluoroacetophenone, 2-bromo-3'-chloroacetophenone or 2-bromo-1-[4-(thiophene-2-yl)phenyl]-1-ethanone which is a derivative of 2-bromo-1-arylethanone at 1:1 mol ratio under reflux in ethanol for 6 hours,
b) obtaining the precipitate by filtering.

14. A method according to Claim 13, **characterized in that** after the precipitate is obtained by filtering, it is dried and said precipitate is then subjected to crystallization processes with ethanol for purification.

## Patentansprüche

1. Verbindung der Formel (I) oder ein pharmazeutisch annehmbares Salz und/oder Solvat davon, **gekennzeichnet durch**; 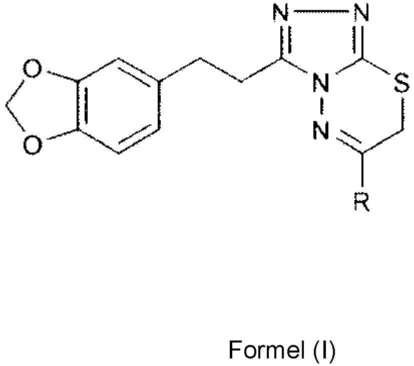 R: Phenyl, 4-Nitrophenyl, 4-Cyanophenyl, 4-Fluorphenyl, 4-Chlorphenyl, 4-Bromophenyl, 4-Methoxyphenyl, 4-Methylphenyl, 4-Methylsulfonylphenyl, Naphthalin-2-yl, 4-Trifluormethylphenyl, 3-Fluorphenyl, 3-Chlorphenyl, Biphenyl-4-yl oder 4-(Thiophen-2-yl)phenyl.

2. Verbindung nach Anspruch 1, wobei die Verbindung aus der nachstehenden Gruppe ausgewählt ist.
3-[2-(I,3-Benzodioxole-5-yl)ethyl]-6-phenyl-7H-[1,2,4]Triazolo[3,4-b] [ 1,3,4]thiadiazine 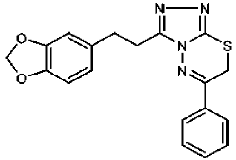
3-[2-(I,3-Benzodioxole-5-yl)ethyl]-6-(4- nitrophenyl) -7H-[1,2,4]triazolo[3,4- b][I,3,4]thiadiazine 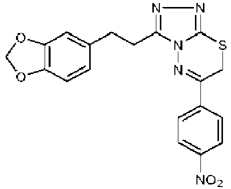
3-[2-(I,3-Benzodioxole-5-yl)ethyl]-6-(4- cyanophenyl)-7H-[1,2,4]triazolo[3,4- b][I,3,4]thiadiazine 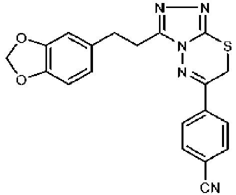
3-[2-(I,3-Benzodioxole-5-yl)ethyl]-6-(4- fluorophenyl)-7H-[1,2,4]triazolo[3,4- b][I,3,4]thiadiazine 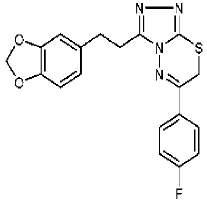
3-[2-(I,3-Benzodioxole-5-yl)ethyl]-6-(4- chlorophenyl)-7H-[1,2,4]triazolo[3,4- b][I,3,4]thiadiazine 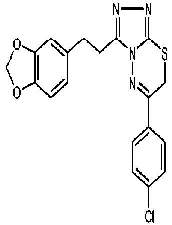
3-[2-(I,3-Benzodioxole-5-yl)ethyl]-6-(4- bromopheny1)-7H-[ 1,2,4]triazolo[3,4- b][I,3,4]thiadiazine 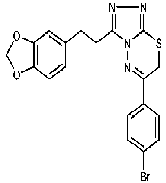
3-[2-(I,3-Benzodioxole-5-yl)ethyl]-6-(4- methoxyphenyl)-7H-[1,2,4]triazolo[3,4- b][1,3,4]thiadiazine 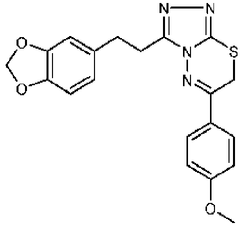
3-[2-(I,3-Benzodioxole-5-yl)ethyl]-6-(4-methylphenyl)-7H-[1,2,4]triazolo[3,4-b][1,3,4]thiadiazine 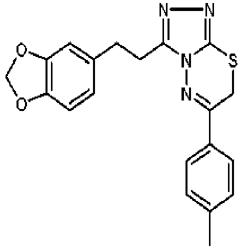
3-[2-(I,3-Benzodioxole-5-yl)ethyl]-6-[4-(methylsulfony l)phenyl)]-7H-[ 1,2,4]triazolo[3,4-b] [ 1,3,4]thiadiazine 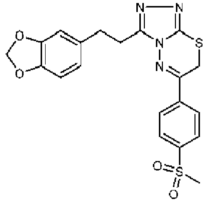
3-[2-(I,3-Benzodioxole-5-yl)ethyl]-6-(4-trifluoromethylphenyl)-7H-[I,2,4]triazolo[3,4-b][1, 3,4]thiadiazine 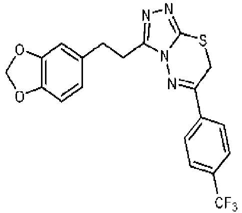
3-[2-(I,3-Benzodioxole-5-yl)ethyl]-6-(3-fluorophenyl)-7H-[1,2,4]triazolo[3,4- b][1, 3,4]thiadiazine 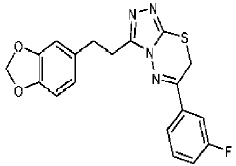
3-[2-(I,3-Benzodioxole-5-yl)ethyl]-6-(3-chlorophenyl)-7H-[I,2,4]triazolo[3,4-b][I,3,4]thiadiazine 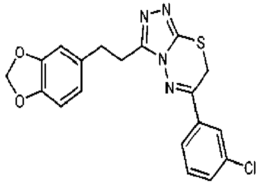
3-[2-(I,3-Benzodioxole-5-yl)ethyl]-6-(biphenyl-4-yl)-7H-[I,2,4]triazolo[3,4-b][I,3,4]thiadiazine 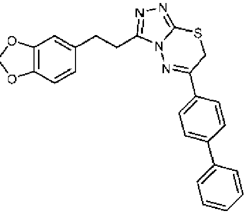
3-[2-(I,3-Benzodioxole-5-yl)ethyl]-6-(4-(thiophene-2-yl)phenyl)-7H-[ 1,2,4]triazolo[3,4-b] [ 1,3,4]thiadiazine

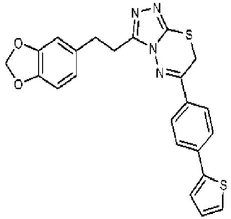

3. Verbindung nach Anspruch 2, wobei es sich um 3-[2-(I,3-Benzodioxol-5-yl)ethyl]-6-(3-Fluorphenyl)-7H-[1,2,4]triazolo[3,4-b][1,3,4]thiadiazin handelt, zur Verwendung als selektiver COX-2-Inhibitor in A549 menschlichen Lungenadenokarzinomzellen.

4. Verbindung nach Anspruch 2, wobei es sich um 3-[2-(I,3-Benzodioxol-5-yl)ethyl]-6-(4-trifluormethylphenyl)-7H-[I,2,4]triazolo[3,4-b][I,3,4]thiadiazin handelt, zur Verwendung als nicht-selektiver COX-Inhibitor in A549 menschlichen Lungenadenokarzinomzellen.

5. Verbindung nach Anspruch 2, wobei die Verbindung ausgewählt ist aus der Gruppe bestehend aus 3-[2-(I,3-Benzodioxol-5-yl)ethyl]-6-[4-(methylsulfonyl)phenyl)]-7H-[I,2,4]triazolo[3,4-b][I,3,4]thiadiazin, 3-[2-(I, 3-Benzodioxole-5-yl)ethyl]-6-(3- fluorophenyl)-7H-[I,2,4]triazolo[3,4-b][I,3,4]thiadiazine and 3-[2-(I,3-Benzodioxole-5- yl)ethyl]-6-(biphenyl-4-yl)-7H-[I, 2,4]Triazolo[3,4-b][I,3,4]thiadiazine zur Verwendung als Akt-Inhibitor in A549 menschlichen Lungenadenokarzinomzellen.

6. Verbindung nach Anspruch 2, wobei es sich um 3-[2-(I,3-Benzodioxol-5-yl)ethyl]-6-(biphenyl-4-yl)-7H-[I,2,4]triazolo[3,4-b][I,3,4]thiadiazin zur Verwendung als ROCK-Inhibitor in A549 menschlichen Lungenadenokarzinomzellen handelt.

7. Verbindung nach Anspruch 2, wobei die Verbindung ausgewählt ist aus der Gruppe bestehend aus 3-[2-(I,3-Benzodioxol-5-yl)ethyl]-6-[4-(methylsulfonyl)phenyl)]-7H-[I, 2,4]triazolo[3,4-b][1,3,4]thiadiazine, 3-[2-(I,3-Benzodioxole-5-yl)ethyl]-6-(4-trifluoromethylphenyl)-7H-[I,2,4]triazolo[3, 4-b][I,3,4]thiadiazin, 3-[2-(I ,3- Benzodioxol-5-yl)ethyl]-6-(3-fluorphenyl)-7H-[I,2,4]triazolo[3,4-b][I,3,4]thiadiazin und 3-[2-(I, 3-Benzodioxol-5-yl)ethyl]-6-(biphenyl-4-yl)-7H-[1,2,4]triazolo[3,4- b][I,3,4]thiadiazine zur Verwendung als Apoptoseinduktor in A549 menschlichen Lungenadenokarzinomzellen.

8. Verbindung nach Anspruch 2, wobei die Verbindung ausgewählt ist aus der Gruppe bestehend aus 3-[2-(I,3-Benzodioxol-5-yl)ethyl]-6-[4-(methylsulfonyl)phenyl)]-7H-[I,2,4]triazolo[3,4-b][I,3,4]thiadiazin, 3-[2-(I,3-Benzodioxol-5-yl)ethyl]-6-(4-trifluormethylphenyl)-7H-[I, 2,4]triazolo[3,4-b][I,3,4]thiadiazine und 3-[2-(I,3-Benzodioxol-5-yl)ethyl]-6-(biphenyl-4-yl)-7H-[I,2,4]triazolo[3,4-b][I,3,4]thiadiazin zur Verwendung als Caspase-3-Enzymaktivator in A549 menschlichen Lungenadenokarzinomzellen.

9. Verbindung nach Anspruch 2, wobei die Verbindung ausgewählt ist aus der Gruppe bestehend aus 3-[2-(I,3-Benzodioxol-5-yl)ethyl]-6-(4-trifluormethylphenyl)-7H-[I,2,4]triazolo[3,4-b][I,3,4]thiadiazin, 3-[2-(I,3-Benzodioxol-5-yl)ethyl]-6-(3-fluorphenyl)-7H-[I, 2,4]triazolo[3,4-b][I,3,4]thiadiazin und 3-[2-(I,3-Benzodioxol-5- yl)ethyl]-6-(biphenyl-4-yl)-7H-[I,2,4]triazolo[3,4-b][I,3,4]thiadiazin zur Verwendung als mitochondrialer Membrandepolarisator in A549 menschlichen Lungenadenokarzinomzellen.

10. Verbindung nach Anspruch 2, wobei die Verbindung ausgewählt ist aus der Gruppe bestehend aus 3-[2-(I,3-Benzodioxol-5-yl)ethyl]-6-[4-(methylsulfonyl)phenyl)]-7H-[I, 2,4]triazolo[3,4-b][1,3,4]thiadiazine, 3-[2-(I,3-Benzodioxole-5-yl)ethyl]-6-(4- trifluoromethylphenyl)-7H-[I,2,4]triazolo[3,4-b][I,3, 4]thiadiazine and 3-[2-(I,3- Benzodioxole-5-yl)ethyl]-6-(3-fluorophenyl)-7H-[1,2,4]triazolo[3,4-b][1,3,4]thiadiazine and 3-[2-(I,3-Benzodioxole-5-yl)ethyl]-6-(biphenyl-4-yl)-7H-[I, 2,4]Triazolo[3,4- b][I,3,4]thiadiazin zur Verwendung als Auslöser des Zellzyklusarrests in der G0/G1-Phase in A549 menschlichen Lungen-Adenokarzinomzellen.

11. Pharmazeutische Zusammensetzung, die eine Verbindung nach einem der vorangehenden Ansprüche enthält.

12. Verwendung einer pharmazeutischen Zusammensetzung nach Anspruch 11 bei der Herstellung eines Arzneimittels zur Verwendung bei der Behandlung von Lungenkrebs.

13. Syntheseverfahren für die Verbindung nach Anspruch 2, **dadurch gekennzeichnet, dass** es die folgenden Schritte umfasst;
a) Erhitzung von 4-Amino-5-[2-(I,3-benzodioxol-5-yl)ethyl]-2,4-dihydro-3H-I,2,4-triazol-3- thion mit 2-Bromacetophenon, 2-Brom-4'-nitroacetophenon, 2-Brom-4'-cyanoacetophenon, 2-Brom-4'-fluoracetophenon, 2-Brom-4'-chloracetophenon, 2,4'-Dibromacetophenon, 2-Brom-4'-methoxyacetophenon, 2-Brom-4'-methylacetophenon, 2-Brom-[4-(methylsulfonyl)phenyl]-I-ethanon, 2-Brom-2'-acetonaphthon, 2-Brom-4'-phenylacetophenon, 2-Brom-4'-trifluormethylacetophenon, 2-Brom-3'-fluoracetophenon, 2-Brom-3-chloracetophenon oder 2-Brom-I-[4-(thiophen-2-yl)phenyl]-I-ethanon, das ein Derivat von 2-Brom-1 -arylethanon ist, im Verhältnis 1: 1 Molverhältnis unter Rückfluss in Ethanol für 6 Stunden,
b) Gewinnung des Niederschlags durch Filtrieren.

14. Verfahren nach Anspruch 13, **dadurch gekennzeichnet, dass** das durch Filtration erhaltene Präzipitat getrocknet und anschließend zur Reinigung Kristallisationsprozessen mit Ethanol unterzogen wird.

## Revendications

1. Composé de formule (I), ou sel et/ou solvate pharmaceutiquement acceptable de celui-ci, **caractérisé par**: 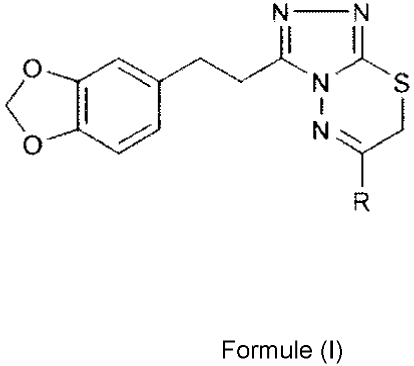 R: Phényle, 4-Nitrophényle, 4-Cyanophényle, 4-Fluorophényle, 4-Chlorophényle, 4-Bromophényle, 4-Méthoxyphényle, 4-Méthylphényle, 4-Méthylsulfonylphényle, Naphtalène-2-Yle, 4- Trifluorométhylphényle, 3-Fluorophényle, 3-Chlorophényle, Biphényl-4-Yle ou 4-(Thiophène-2-yl)Phényle.

2. Composé selon la revendication 1, dans lequel ledit composé est choisi du groupe suivant.
3-[2-(I,3-Benzodioxole-5-yl)éthyl]-6-phényl-7H-[1,2,4]triazolo[3,4-b] [ 1,3,4]thiadiazine 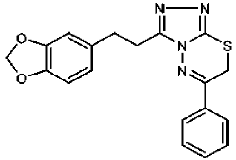
3-[2-(I,3-Benzodioxole-5-yl)éthyl]-6-(4-nitrophényl) -7H-[1,2,4]triazolo[3,4- b][I,3,4]thiadiazine 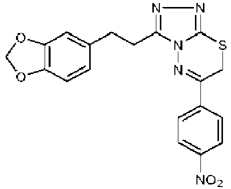
3-[2-(I,3-Benzodioxole-5-yl)éthyl]-6-(4- cyanophényl)-7H-[1,2,4]triazolo[3,4- b][I,3,4 ]thiadiazine 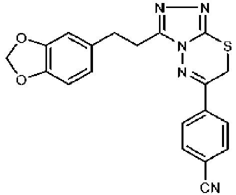
3-[2-(I,3-Benzodioxole-5-yl)éthyl]-6-(4- fluorophényl)-7H-[1,2,4]triazolo[3,4- b][I,3,4 ]thiadiazine 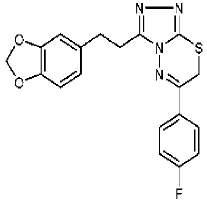
3-[2-(I,3-Benzodioxole-5-yl)éthyl]-6-(4- chlorophényl)-7H-[1,2,4]triazolo[3,4- b][I,3,4]thiadiazine 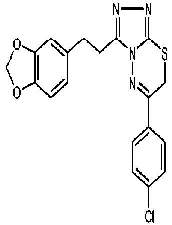
3-[2-(I,3-Benzodioxole-5-yl)éthyl]-6-(4- bromophényl)-7H-[ 1,2,4]triazolo[3,4- b][I,3,4]thiadiazine 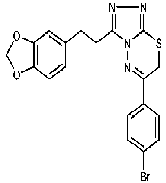
3-[2-(I,3-Benzodioxole-5-yl)éthyl]-6-(4- méthoxyphényl)-7H-[1,2,4]triazolo[3,4- b][1,3,4]thiadiazine 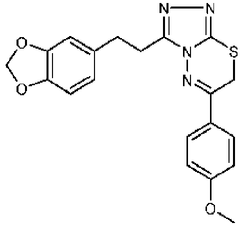
3-[2-(I,3-Benzodioxole-5-yl)éthyl]-6-(4-méthylphényl)-7H-[1,2,4]triazolo[3,4-b][1,3,4]thiadiazine 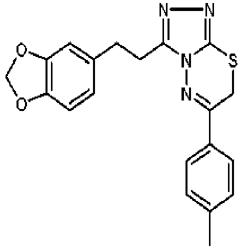
3-[2-(I,3-Benzodioxole-5-yl)éthyl]-6-[4-(méthylsulfonyl)phényl)]-7H-[ 1,2,4]triazolo[3,4-b] [ 1,3,4]thiadiazine 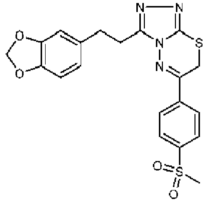
3-[2-(I,3-Benzodioxole-5-yl)éthyl]-6-(4-trifluorométhylphényl)-7H-[I,2,4]triazolo[3,4-b][1, 3,4]thiadiazine 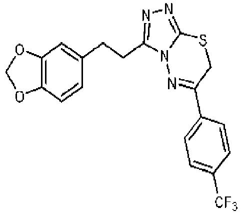
3-[2-(I,3-Benzodioxole-5-yl)éthyl]-6-(3-fluorophényl)-7H-[1,2,4]triazolo[3,4- b][1, 3,4]thiadiazine 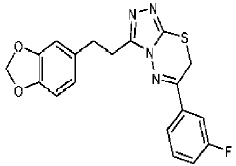
3-[2-(I,3-Benzodioxole-5-yl)éthyl]-6-(3-chlorophényl)-7H-[I,2,4]triazolo[3,4-b][I,3,4]thiadiazine 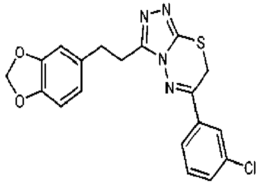
3-[2-(I,3-Benzodioxole-5-yl)éthyl]-6-(biphényl-4-yl)-7H-[I,2,4]triazolo[3,4-b][I,3,4]thiadiazine 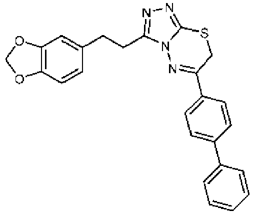
3-[2-(I,3-Benzodioxole-5-yl)éthyl]-6-(4-(thiophène-2-yl)phényl)-7H-[ 1,2,4]triazolo[3,4-b] [ 1,3,4]thiadiazine

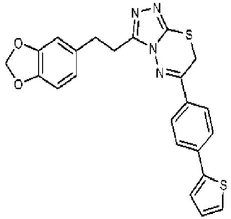

3. Composé de la revendication 2, qui est le 3-[2-(I,3-benzodioxole-5-yl)éthyl]-6-(3-fluorophényl)-7H-[I,2,4]triazolo[3,4-b][I,3,4]thiadiazine pour utilisation comme inhibiteur sélectif de la COX-2 dans les cellules d'adénocarcinome du poumon humain A549.

4. Composé de la revendication 2, qui est le 3-[2-(I,3-benzodioxole-5-yl)éthyl]-6-(4-trifluorométhylphényl)-7H-[I,2,4]triazolo[3,4-b][I,3,4]thiadiazine pour utilisation comme inhibiteur non sélectif de la COX dans les cellules d'adénocarcinome du poumon humain A549.

5. Composé selon la revendication 2, dans lequel le composé est choisi parmi le groupe comprenant 3-[2-(I,3-benzodioxole-5-yl)éthyl]-6-[4-(méthylsulfonyl)phényl)]-7H-[I,2,4]triazolo[3,4-b][I,3,4]thiadiazine, 3-[2-(I,3-benzodioxole-5-yl)éthyl]-6-(3-fluorophényl)-7H-[I,2,4]triazolo[3,4b][I,3,4] thiadiazine et 3-[2-(I,3-Benzodioxole-5-yl)éthyl]-6-(biphényle-4-yl)-7H-[I,2,4]triazolo[3,4-b][I,3,4]thiadiazine pour utilisation comme inhibiteur Akt dans les cellules d'adénocarcinome du poumon humain A549.

6. Composé de la revendication 2, qui est le 3-[2-(I,3-benzodioxole-5-yl)éthyl]-6-(biphényl-4-yl)-7H-[I,2,4]triazolo[3,4-b][I,3,4]thiadiazine pour utilisation comme inhibiteur ROCK dans les cellules d'adénocarcinome du poumon humain A549.

7. Composé selon la revendication 2, dans lequel le composé est choisi parmi le groupe comprenant 3-[2-(I,3-benzodioxole-5-yl)éthyl]-6-[4-(méthylsulfonyl)phényl)]-7H-[I,2,4]triazolo[3,4-b][I,3,4]thiadiazine, 3-[2-(I,3-benzodioxole-5-yl)éthyl]-6-(4-trifluorométhylphényl)-7H-[1,2,4]triazolo[3,4-b][I,3,4]thiadiazine, 3-[2-(I,3- Benzodioxole-5-yl)éthyl]-6-(3 -fluorophényl)-7H-[I,2,4]triazolo[3,4-b][I,3,4]thiadiazine et 3-[2-(I,3-Benzodioxole-5-yl)éthyl]-6-(biphényl-4-yl)-7H-[I,2,4]triazolo[3,4-b][I,3,4]thiadiazine pour utilisation comme inducteur d'apoptose dans les cellules d'adénocarcinome du poumon humain A549.

8. Composé selon la revendication 2, dans lequel le composé est choisi parmi le groupe comprenant 3-[2-(I,3-benzodioxole-5-yl)éthyl]-6-[4-(méthylsulfonyl)phényl)]-7H-[I,2,4]triazolo[3,4-b][I,3,4]thiadiazine, 3-[2-(I,3-benzodioxole-5-yl)éthyl]-6-(4-trifluorométhylphényl)-7H-[1,2,4]triazolo[3,4-b][I,3,4]thiadiazine et 3-[2-(I,3-Benzodioxole-5-yl)éthyl]-6-(biphényle-4-yl)-7H-[I,2,4]triazolo[3,4-b][I,3,4]thiadiazine pour utilisation comme activateur de l'enzyme caspase-3 dans les cellules d'adénocarcinome du poumon humain A549.

9. Composé selon la revendication 2, dans lequel le composé est choisi parmi le groupe comprenant 3-[2-(I,3-benzodioxole-5-yl)éthyl]-6-(4-trifluorométhylphényl)-7H-[I,2,4]triazolo[3,4-b][I,3,4]thiadiazine,3-[2-(I,3-Benzodioxole-5-yl)éthyl]-6-(3-fluorophényl)-7H-[1,2,4]triazolo[3,4-b][I,3,4]thiadiazine et 3-[2-(I,3-Benzodioxole-5- yl)éthyl]-6-(biphényl-4-yl)-7H-[I,2,4]triazolo[3,4-b][I,3,4]thiadiazine pour utilisation comme dépolariseur de membrane mitochondriale dans les cellules d'adénocarcinome du poumon humain A549.

10. Composé selon la revendication 2, dans lequel le composé est choisi parmi le groupe comprenant 3-[2-(I,3-benzodioxole-5-yl)éthyl]-6-[4-(méthylsulfonyl)phényl)]-7H-[I,2,4]triazolo[3,4-b][I,3,4]thiadiazine, 3-[2-(I,3-benzodioxole-5-yl)éthyl]-6-(4-trifluorométhylphényl)-7H-[1,2,4]triazolo[3,4-b][I,3,4]thiadiazine et 3-[2-(I,3-Benzodioxole-5-yl)éthyl]-6-(3-fluorophényl)-7H-[I,2,4]triazolo[3,4-b][I,3,4]thiadiazine et 3-[2-(I,3-Benzodioxole-5-yl)éthyl]-6-(biphényl-4-yl)-7H-[I,2,4]triazolo[3,4-b][I,3,4]thiadiazine pour utilisation comme inducteur de l'arrêt du cycle cellulaire à la phase G0/G1 dans les cellules d'adénocarcinome du poumon humain A549.

11. Composition pharmaceutique comprenant un composé selon l'une quelconque des revendications précédentes.

12. Utilisation d'une composition pharmaceutique selon la revendication 11 dans la préparation d'un médicament destiné à être utilisé dans le traitement du cancer du poumon.

13. Procédé de synthèse du composé selon la revendication 2, **caractérisé en ce qu'**il comprend les étapes suivantes:
a) échauffement de la 4-amino-5-[2-(I,3-benzodioxole-5-yl)éthyl]-2,4-dihydro-3H-I,2,4-triazole-3-thione avec de la 2-bromoacétophénone, 2- bromo-4'-nitroacétophénone, 2-bromo-4'-cyanoacétophénone, 2-bromo-4'-fluoroacétophénone, 2-bromo-4'-chloroacétophénone, 2,4'-dibromoacétophénone, 2-bromo-4'-méthoxyacétophénone, 2-bromo-4'-méthylacétophénone, 2-bromo-I-[4-(méthylsulfonyl)phényl]-I-éthanone, 2-bromo-2'-acétonaphtone, 2-bromo-4'-phénylacétophénone, 2-bromo- 4'-trifluorométhylacétophénone, 2-bromo-3'-fluoroacétophénone, 2-bromo-3 chloroacétophénone ou 2-bromo-I-[4-(thiophène-2-yl)phényl]-I-éthanone qui est un dérivé de la 2- bromo-1-aryléthanone dans un rapport molaire 1:1 sous reflux dans l'éthanol pendant 6 heures,
b) obtention du précipité par filtration.

14. Procédé selon la revendication 13, **caractérisé en ce qu'**après que le précipité a été obtenu par filtration, il est séché et ledit précipité est ensuite soumis à des processus de cristallisation avec l'éthanol pour purification.
